# EUROPEAN PATENT SPECIFICATION

(11) **EP 1 367 058 B1**
(45) Date of publication and mention of the grant of the patent: **09.11.2005**
(21) Application number: 03253394.5
(22) Date of filing: 30.05.2003
(51) Int. Cl.: C07D 471/04, C07D 407/10, A61P 9/10, A61K 31/44

(54) **Tetrahydropyran derivatives**
Tetrahydropyran Derivate
Dérivés de tetrahydropyrane

(30) Priority: 31.05.2002 JP 2002158555
(43) Date of publication of application: 03.12.2003
(73) Proprietor: Astellas Pharma Inc., Chuo-ku, Tokyo (JP)
(72) Inventor: Suga, Akira, Yamanouchi Pharmaceutical Co. Ltd., Tsukuba-shi, Ibaraki 305-8585 (JP); Kubota, Hideki, Yamanouchi Pharmaceutical Co. Ltd., Tsukuba-shi, Ibaraki 305-8585 (JP); Miura, Masanori Yamanouchi Pharmaceutical Co. Ltd., Tsukuba-shi, Ibaraki 305-8585 (JP); Sasuga, Daisuke Yamanouchi Pharmaceutical Co. Ltd., Tsukuba-shi, Ibaraki 305-8585 (JP); Moritani, Hiroshi, Tsukuba-shi, Ibaraki 305-8585 (JP)
(74) Representative: Bates, Philip Ian

(56) References cited:
- EP-A- 0 705 831
- WO-A-01/00184
- WO-A-01/00189
- WO-A-01/97787
- DE-A- 19 951 022
- WERMUTH ET AL: "The Practise of Medicinal Chemistry" , PRACTICE OF MEDICINAL CHEMISTRY, XX, XX, PAGE(S) 203-237 XP002190259 * Table 13.1 * * Section IV B, pages 228-229 *

## Description

The present invention relates to novel tetrahydropyran derivatives and their salts that have apo B-related lipoprotein secretion-inhibiting activity, and to pharmaceutical compositions that comprise it as the active ingredient.

Hyperlipemia is one risk factor of arteriosclerotic diseases such as ischemic cardiopathy, along with diabetes, hypertension, smoking, etc., and ameliorating it is effective in treatment of the diseases [*JAMA*. 1986;256:2835-2838, *JAMA.* 1986;256:2823-2828, *JAMA.* 1993;268:3015-3023]. Hypercholesterolemia is a risk factor of arteriosclerosis and causes coronary disorders; and hypertriglyceridemia is considered as one cause of ischemic cardiopathy such as myocardial infarction. Accordingly, for treatment of hyperlipemia, it is desirable to lower the level of cholesterol and triglyceride in blood. Of the remedies that have heretofore been in clinical use for hyperlipemia, HMG-CoA reductase inhibitors, anion-exchange resin compositions, probucol and the like are essentially for lowering blood cholesterol; and fibrate and nicotinic acid compositions are essentially for lowering blood triglyceride.

Cholesterol absorbed in small intestine forms a chylomicron complex with apolipoprotein B (apo B), phospholipid and triglyceride, in the granular endoplasmic reticulia of intestinal epithelyocytes, and it is secreted into blood via lymph vessels and carried to tissues such as liver. Cholesterol synthesized in liver forms an VLDL (very-low-density lipoprotein) complex with apo B, phospholipid and triglyceride, in the granular endoplasmic reticulia in liver cells, and it is secreted into blood, converted into LDL (low-density lipoprotein), and carried to other tissues [*New England Journal of Medicine*, 1983;309:288-296].

Apo B includes two molecular species, apo B-100 and apo B-48, and these are synthesized in the granular endoplasmic reticulia of cells. In liver cells, synthesized is apo B-100, while in intestinal cells, synthesized is apo B-48 through apo B mRNA editing, and these are to be the structural apolipoproteins of VLDL and chylomicron, respectively. Cholesterol esters, triglycerides and others synthesized in agranular endoplasmic reticulia are transferred by MTP, and bound to apo B in endoplasmic reticulia to form a premature lipoprotein. The premature lipoprotein goes through the process of further lipid loading, sugar chain loading in Golgi body, and others to be a mature lipoprotein, which is then secreted outside cells *[Biochem. Biophys. Acta.,* 1999;1440:1-31; *Biochem. Biophys. Acta.,* 1997;1345:11-26].

Accordingly, when the secretion of apo B-related lipoprotein (generic term for chylomicron, VLDL and LDL) that comprises apo B as the constitutive component, from small intestine and/or liver into blood is inhibited, then it is possible to lower the level of cholesterol and triglyceride in blood, and compounds, if any, having the capability for such inhibition are useful for remedies for hyperlipemia, arteriosclerosis, obesity, pancreatitis, etc.

For apo B-related lipoprotein production and/or secretion inhibitors, amido compounds have been reported, for example, cycloalkanolindole and.cycloalkanazaindole derivatives as in JP-A 8-225526 and 10-45759. The compound described in Example 5 in JP-A 8-225526 had been in clinical stages as Implitapide (Bay-13-9952). Apart from these, other various compounds have been reported, for example, hydrazide derivatives as in WO00/71502 and WO01/74817. However, their efficacy is not satisfactory, and compounds having better and higher efficacy are desired.

On the other hand, JP-A 8-225526 and 10-45759 disclose compounds in which a nitro atom in a pyridoindole or carbazole ring is substituted with carbamoylmethyl-substituted benzyl. However, compounds in which a methyl moiety in the carbamoylmethyl is substituted with tetrahydropyran have not been reported yet.

### DISCLOSURE OF THE INVENTION

An object of the invention is to provide novel tetrahydropyran derivatives and their salts that have excellent apo B-related lipoprotein secretion-inhibiting activity and are useful for blood cholesterol and triglyceride depressors, and to provide pharmaceutical compositions comprising it.

We, the present inventors have assiduously studied for obtaining novel compounds that have apo B-related lipoprotein secretion-inhibiting activity, and, as a result, have found out novel derivatives having an tetrahydropyran which has never known before. Further, we have found that the tetrahydropyran derivatives have a strong apo B-related lipoprotein secretion-inhibiting activity and a strong cholesterol and triglyceride-lowering effect, and on the basis of these findings, we have completed the present invention.

Specifically, the invention relates to a tetrahydropyran derivative of the following general formula (I) or a salt thereof: 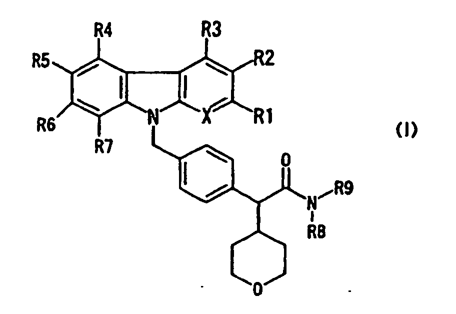 wherein R¹ and R³ are the same or different and each represents H or C₁-C₆ alkyl;
R² represents H, halogen, R^{a}- C₁-C₆ alkyl-, or R²⁰O-CO-;
R^{a} represents H, R²¹O-CO-, R²²R²³N-, R²⁴R²⁵N-CO-, R²⁶O-, cyano, or optionally-substituted hetero ring-;
R⁴, R⁵, R⁶ and R⁷ are the same or different and each represents H, halogen, haloalkyl, cyano, C₁-C₆ alkyl, C₁-C₆ alkyl-O-, R²¹O-CO- C₁-C₆ alkyl-, R²⁷-CO-, or R²⁸R²⁹N-S(O)₂-;
R⁸ and R⁹ are the same or different and each represents H, C₁-C₆ alkyl, R³⁰- C₁-C₆ alkyl-, R³¹R³²N-, optionally-substituted hetero ring, or R³³R³⁴R³⁵C-;
R⁸ and R⁹ may together form optionally-substituted hetero ring-;
X represents N or CR³⁶;
R²⁰, R²² to R²⁶, R²⁸, R²⁹, R³², R³⁵ and R³⁶ are the same or different and each represents H or C₁-C₆ alkyl;
R²¹ represents H, C₁-C₆ alkyl, or aryl- C₁-C₆ alkyl-;
R²⁷ represents HO-, C₁-C₆ alkyl-O-, or optionally-substituted hetero ring-;
R³⁰ represents optionally-substituted aryl, optionally-substituted hetero ring-, or C₁-C₆ alkyl-O-;
R³¹ represents optionally-substituted aryl, or optionally-substituted hetero ring-;
R³³ represents HO-C₁-C₆ alkyl-, or optionally-substituted hetero ring- C₁-C₆ alkyl;
R³⁴ represents optionally-substituted aryl-;
wherein aryl is a mono- to tricyclic aromatic hydrocarbon group that is 6- to 14-membered; and hetero ring represents a saturated hetero ring or an unsaturated hetero ring which are optionally-condensed having from 1 to 3 hetero atoms selected from nitrogen, oxygen and .sulfur atoms; unsaturated hetero ring means an optionally-condensed, 5- or 6-membered unsaturated heterocyclic group having from 1 to 3 hetero atoms selected from nitrogen, oxygen and sulfur atoms; and saturated hetero ring means an optionally-condensed, 3- to 10-membered saturated hetero ring having from 1 to 3 hetero atoms selected from nitrogen, oxygen and sulfur atoms;
and wherein the optionally-substituted aryl groups and the optionally-substituted hetero ring groups may have from 1 to 3 substituents selected from halogen, oxo, C₁-C₆ alkyl optionally substituted with OH, haloalkyl optionally substituted with C₁-C₆ alkyl-O-, C₂-C₆ alkenyl, C₂-C₆ alkynyl, C₃-C₈ cycloalkyl, C₃-C₈ cycloalkyl- C₁-C₆ alkyl-, aryl optionally substituted with halogen, aryl- C₁-C₆ alkyl-, hetero ring-,hetero ring- C₁-C₆ alkyl-, HO-, C₁-C₆ alkyl-O-, C₁-C₆ alkyl-O- C₁-C₆ alkyl- optionally substituted with OH, C₁-C₆ alkyl-O- C₁-C₆ alkyl-CO-, C₂-C₆ alkenyl-O-, C₂-C₆ alkynyl-O-, C₃-C₈ cycloalkyl-O-, aryl-O-,
aryl-C₁-C₆ alkyl-O-, hetero rind-O-, hetero ring- C₁-C₆ alkyl-O-, C₁-C₆ alkyl-S(O)ₘ-, C₁-C₆ alkyl-S(O)ₘ- C₁-C₆ alkyl-, C₂-C₆ alkenyl-S(O)ₘ-, C₂-C₆ alkynyl-S (O)ₘ-, C₃-C₈ cycloalkylS(O)ₘ-, aryl-S(O)ₘ-, aryl- C₁-C₆ alkyl-S(O)ₘ-, hetero ringS(O)ₘ-, hetero ring- C₁-C₆ alkyl-S (O)ₘ-, C₁-C₆ alkyl-O-CO-, C₂-C₆ alkenyl-O-CO-, C₂-C₆ alkynyl-O-CO-, C₃-C₈ cycloalkyl-O-CO-, aryl-O-CO, aryl- C₁-C₆ alkyl-O-CO-, hetero ring-O-CO-, hetero ring- C₁-C₆ alkyl-O-CO-, HO-CO-, HO-CO- C₁-C₆ alkyl-, HO-CO- C₁-C₆ alkyl- O-, C₁-C₆ alkyl-CONR^{x}-, C₁-C₆ alkyl-CONR^{x}-C₁-C₆ alkyl-, R^{x}R^{y}N-, R^{x}R^{y}N-C₁-C₆ alkyl-, R^{x}R^{y}N-CO-, R^{x}R^{y}N-CO-C₁-C₆ alkyl, HCO-, C₁-C₆ alkyl-CO-, C₂-C₆ alkenyl-CO-, C₂-C₆ alkynyl-CO-, C₃-C₈ cycloalkyl-CO-, aryl-CO-, aryl- C₁-C₆ alkyl-CO-, hetero ring-CO-, hetero ring- C₁-C₆ alkyl-CO-, HCS-, C₁-C₆ alkyl- CS-, C₂-C₆ alkenyl-CS-, C₂-C₆ alkynyl-CS-, C₃-C₈ cycloalkyl-CS-, aryl-CS-, aryl-C₁-C₆ alkyl-CS-, hetero ring-CS-, hetero ring- C₁-C₆ alkyl-CS-, C₁-C₆ alkyl-O-CO-CO-, HCO-O-, C₁-C₆ alkyl-CO-O-, C₂-C₆ alkenyl-CO-O-, C₂-C₆ alkynyl-CO-O-, C₃-C₈ cycloalkyl-CO-O-, aryl-CO-O, aryl- C₁-C₆ alkyl-CO-O-, hetero ring-CO-O-, hetero ring-C₁-C₆ alkyl-CO-O-, aryl- C₁-C₆ alkyl-CO-O-, hetero ring-O-CO-; wherein m = O, 1 or 2; and R^{x} and R^{y} are the same or different and each represents H or C₁-C₆ alkyl.

Preferably in the tetrahydropyran derivatives of formula (I) and their salts, the optionally-substituted aryl and the optionally-substituted hetero ring are optionally substituted with 1 to 3 substituents selected from the following group:
Substituents: halogen, oxo, lower alkyl optionally substituted with OH, haloalkyl optionally substituted with lower alkyl-O-, lower alkenyl, C₃₋₈ cycloalkyl, C₃₋₈ cycloalkyl-lower alkyl-, aryl optionally substituted with halogen, aryl-lower alkyl-, hetero ring-, HO-, lower alkyl-O-, lower alkyl-O-lower alkyl- optionally substituted with OH, lower alkyl-O-lower alkyl-CO-, lower alkyl-S(O)ₘ-, lower alkyl-S(O)ₘ-lower alkyl, lower alkyl-O-CO-, HO-CO-lower alkyl-, HO-CO-lbwer alkyl-O-, R^{x}R^{y}N-CO-, R^{x}R^{y}N-CO-lower alkyl-, R^{x}R^{y}N-lower alkyl-, lower alkyl-CO-, aryl-lower alkyl-O-CO-, and hetero ring-O-CO-,
wherein R^{x} and R^{y} are the same or different and each
represents H or lower alkyl,
m indicates 0, 1 or 2.

More preferably.in the tetrahydropyran derivatives of formula (I) and their salts, R² is R^{a}-lower alkyl-; R⁴, R⁵, R⁶ and R⁷ are the same or different, each representing H or halogen; R⁸ and R⁹ are the same or different, each representing H, lower alkyl, or R³⁰-lower alkyl-, or R⁸ and R⁹may together form a hetero ring; and X is N.

Even more preferably in the tetrahydropyran derivatives and their salts, R² is HO-CO-lower alkyl-.

Most preferred are compounds selected from 3-(2,4-dimethyl-9-{4-[(*S*)-2-oxo-2-(4-pyridin-2-ylpiperazin-1-yl)-1-(tetrahydro-2H-pyran-4-yl)ethyl]benzyl}-9*H*-pyrido[2,3-b]indol-3-yl)propanoic acid; 3-(2,4-dimethyl-9-{4-[(*S*)-2-oxo-2-(4-propylpiperazin-1-yl)-1-(tetrahydro-2*H*-pyran-4-yl)ethyl]benzyl}-9*H*-pyrido[2,3-b]indol-3-yl)propanoic acid; 3-(9-{4-[(*S*)-(benzylcarbamoyl)(tetrahydro-2*H*-pyran-4-yl)methyl]benzyl}-2,4-dimethyl-9*H*-pyrido[2,3-b]indol-3-yl)propanoic acid; 3-(2,4-dimethyl-9-{4-[(S)-(dimethylcarbamoyl)(tetrahydro-2*H*-pyran-4-yl)methyl]benzyl}-9H-pyrido[2,3-b]indol-3-yl)propanoic acid; 3-(2,4-dimethyl-9-{4-[(*S*)-2-morpholin-4-yl-2-oxo-1-(tetrahydro-2*H*-pyran-4-yl)ethyl]benzyl}-9*H*-pyrido[2,3-b]indol-3-yl)propanoic acid; 3-(2,4-dimethyl-9-{4-[(*S*)-2-(4-isobutylpiperazin-1-yl)-2-oxo-1-(tetrahydro-2*H*-pyran-4-yl)ethyl]benzyl}-9*H*-pyrido[2,3-b]indol-3-yl)propanoic acid; 3-(9-{4-[(*S*)-2-(4-cyclobutylpiperazin-1-yl)-2-oxo-1-(tetrahydro-2*H*-pyran-4-yl)ethyl]benzyl}-2,4-dimethyl-9*H* pyrido[2,3-b]indol-3-yl)propanoic acid; 3-(9-{4-[(*S*)-2-(4-cyclopentylpiperazin-1-yl)-2-oxo-1-(tetrahydro-2*H*-pyran-4-yl)ethyl]benzyl}-2,4-dimethyl-9*H*-pyrido[2,3-b]indol-3-y)propanoic acid; and their salts.

Another aspect of the invention provides a pharmaceutical composition that comprises a tetrahydropyran derivative of formula (I) or its pharmaceutically acceptable salt as the active ingredient, along with a pharmaceutically acceptable carrier.

More preferably, the invention provides use for producing a medicament for hyperlipemia that contains a therapeutically-effective amount of the tetrahydropyran derivative or its pharmaceutically acceptable salt.

### MODES OF CARRYING OUT THE INVENTION

The invention is described further in detail.

First described are the compounds of formula (I).

Regarding the definition of general formulae referred to herein, the term "lower" means a linear or branched carbon chain having from 1 to 6 carbon atoms, unless otherwise specifically indicated.

"Lower alkyl" is C₁₋₆ alkyl, preferably C₁₋₄ alkyl such as methyl, ethyl, propyl, isopropyl or t-butyl, more preferably C₁₋₃ alkyl.

"Aryl" is a mono- to tricyclic aromatic hydrocarbon group that is 6- to 14-membered as a whole, and is preferably phenyl, naphthyl or anthranyl.

"Halogen" includes, for example, fluorine, chlorine, bromine and iodine atoms.

"Haloalkyl" is a group of the above-mentioned lower alkyl of which any hydrogen atom is substituted with the above-mentioned halogen, preferably trifluoromethyl.

"Hetero ring" includes a "saturated hetero ring" and an "unsaturated hetero ring" which are optionally-condensed having from 1 to 3 hetero atoms selected from nitrogen, oxygen and sulfur atoms.

"Unsaturated hetero ring" means an optionally-condensed, 5- or 6-membered unsaturated heterocyclic group having from 1 to 3 hetero atoms selected from nitrogen, oxygen and sulfur atoms, and includes aromatic hetero rings. Preferably, it is a 5- or 6-membered monocyclic unsaturated hetero ring with one or two nitrogen atoms, including pyrrole, 3-pyrroline, 3,6-dihydropyrimidine, 3,6-dihydropyridine, pyridine, furan, thiazole, thiophene, imidazole, triazole, tetrazole, pyrimidine, pyridazine.

"Saturated hetero ring" means an optionally-condensed, 3- to 10-membered saturated hetero ring having from 1 to 3 hetero atoms selected from nitrogen, oxygen and sulfur atoms. The saturated hetero ring includes spiro-structured rings and bridged rings. Concretely, it includes azetidine, pyrrolidine, piperidine, piperazine, azepine, diazepine, morpholine, thiazoline, 1,4-dioxa-8-azaspiro[4,5]decane, isoindoline, tetrahydroisoquinoline, 2,5-diazabicyclo[2.2.1]heptane, tetrahydropyran. Preferably, it is a 4- to 7-membered saturated hetero ring.

"Optionally-substituted hetero ring formed by R⁸ and R⁹ together" means a hetero ring of those mentioned above, which has at least one nitrogen atom as the ring-forming hetero atom. Preferably, it is a 5- or 6-membered saturated or unsaturated hetero ring with one or two ring-forming nitrogen atoms, and the hetero ring may be substituted with any of lower alkyl, lower alkylene, aryl optionally substituted with halogen, cycloalkyl, or pyridyl, and may be condensed with benzene ring.

"Optionally-substituted" aryl and hetero ring may have from 1 to 3 substituents.

The substituents mean those that are generally used in the field of the substituted groups. They include halogen, oxo, lower alkyl optionally substituted with OH, haloalkyl optionally substituted with lower alkyl-O-, lower alkenyl, lower alkynyl, C₃₋₈ cycloalkyl, C₃₋₈ cycloalkyl-lower alkyl-, aryl optionally substituted with halogen, aryl-lower alkyl-, hetero ring-, hetero ring-lower alkyl-, HO-, lower alkyl-O-, lower alkyl-O-lower alkyl- optionally substituted with OH, lower alkyl-O-lower alkyl-CO-, lower alkenyl-O-, lower alkynyl-O-, C₃₋₈ cycloalkyl-O-, aryl-0-, aryl-lower alkyl-O-, hetero ring-O-, hetero ring-lower alkyl-O-, lower alkyl-S(O)ₘ- (where m indicates 0, 1 or 2, and the same shall apply hereunder), lower alkyl-S(O)ₘ-lower alkyl-, lower alkenyl-S(O)ₘ-, lower alkynyl-S(O)ₘ-, C₃₋₈ cycloalkyl-S(O)ₘ-, aryl-S(O)ₘ-, aryl-lower alkyl-S(O)ₘ-, hetero ring-S(O)ₘ-, hetero ring-lower alkyl-S(O)ₘ-, lower alkyl-O-CO-, lower alkenyl-O-CO-, lower alkynyl-O-CO-, C₃₋₈ cycloalkyl-O-CO-, aryl-O-CO-, aryl-lower alkyl-O-CO-, hetero ring-O-CO-, hetero ring-lower alkyl-O-CO-, HO-CO-, HO-CO-lower alkyl-, HO-CO-lower alkyl-O-, lower alkyl-CONR^{x}-(where R^{x} is H or lower alkyl - the same shall apply hereunder), lower alkyl-CONR^{x}-lower alkyl-, R^{x}R^{y}N- (where R^{x} and R^{y} are the same or different and each represents H or lower alkyl - the same shall apply hereunder), R^{x}R^{y}N-lower alkyl-, R^{x}R^{y}N-CO-, R^{x}R^{y}N-CO-lower alkyl-, HCO-, lower alkyl-CO-, lower alkenyl-CO-, lower alkynyl-CO-, C₃₋₈ cycloalkyl-CO-, aryl-CO-, aryl-lower alkyl-CO-, hetero ring-CO-, hetero ring-lower alkyl-CO-, HCS-, lower alkyl-CS-, lower alkenyl-CS-, lower alkynyl-CS-, C₃₋₈ cycloalkyl-CS-, aryl-CS-, aryl-lower alkyl-CS-, hetero ring-CS-, hetero ring-lower alkyl-CS-, lower alkyl-O-CO-CO-, HCO-O-, lower alkyl-CO-0-, lower alkenyl-CO-O-, lower alkynyl-CO-O-, C₃₋₈ cycloalkyl-CO-O-, aryl-CO-O-, aryl-lower alkyl-CO-O-, hetero ring-CO-O-, hetero ring-lower alkyl-CO-O-, aryl-lower alkyl-CO-O-, hetero ring-O-CO-. Preferably, they are halogen, oxo, lower alkyl optionally substituted with OH, haloalkyl optionally substituted with lower alkyl-O-, lower alkenyl, C₃₋₈ cycloalkyl, C₃₋₈ cycloalkyl-lower alkyl, aryl optionally substituted with halogen, aryl-lower alkyl-, hetero ring-, HO-, lower alkyl-O-, lower alkyl-O-lower alkyl- optionally substituted with OH, lower alkyl-O-lower alkyl-CO-, lower alkyl-S(O)ₘ-, lower alkyl-S(O)ₘ-lower alkyl-, lower alkyl-O-CO-, HO-CO-lower alkyl-, HO-CO-lower alkyl-O-, R^{x}R^{y}N-CO-, R^{x}R^{y}N-CO-lower alkyl-, R^{x}R^{y}N-lower alkyl-, lower alkyl-CO-, aryl-lower alkyl-O-CO-, hetero ring-O-CO-.

Concretely, "C₃₋₈ cycloalkyl" includes cyclopropyl, cyclobutyl, cyclopentyl, cyclohexyl. Preferably, it is cycloalkyl having from 3 to 6 carbon atoms, more preferably from 4 to 6 carbon atoms.

Especially preferably in the compounds of formula (I) and their salts of the invention, R² is HO-CO-lower alkyl-; R⁴, R⁵, R⁶ and R⁷ are the same or different, each representing H or halogen; R⁸ and R⁹ are the same or different, each representing H, or aryl optionally substituted with halogen-lower alkyl-, or R⁸ and R⁹ may together form a hetero ring with one or two ring-forming nitrogen atoms; and X is N.

The compounds (I) of the invention have an asymmetric carbon, and include (R)- and (S)-optical isomers and racemates based on it. Further, depending on the type of the substituents therein, the compounds may have multiple asymmetric carbons, and include diastereomers and enantiomers based on these. The invention encompasses all such isomers isolated and their mixtures.

The compounds (I) of the invention may form salts with acids or bases. The salts include acid-addition salts with mineral acids such as hydrochloric acid, hydrobromic acid, hydroiodic acid, sulfuric acid, nitric acid, phosphoric acid; or with organic acids such as formic acid, acetic acid, propionic acid, oxalic acid, malonic acid, succinic acid, fumaric acid, maleic acid, lactic acid, malic acid, citric acid, tartaric acid, carbonic acid, picric acid, methanesulfonic acid, ethanesulfonic acid, glutamic acid.

The salts with bases includes salts with inorganic bases such as sodium, potassium, magnesium, calcium, aluminium; salts with organic bases such as methylamine, ethylamine, meglumine, ethanolamine, diethanolamine, tromethamine; salts with basic amino acids such as lysine, arginine, ornithine; and ammonium salts.

The invention further encompasses hydrates, solvates with ethanol or the like, and polymorphic crystals of the compounds (I) of the invention.

The compounds of the invention further include pharmaceutically acceptable prodrugs. The groups that form such pharmaceutically acceptable prodrugs of the compounds of the invention are described, for example, in *Prog. Med.* 5:2157-2161 (1985); and *Development of Medicines* published by Hirokawa Shoten, Vol. 7, Molecular Planning, pp. 163-198. Concretely, they are groups capable of being converted into primary amines, secondary amines, OH, COOH and others of the invention through hydrolysis or solvolysis or under physiological condition. Their examples are -O-CO-optionally substituted lower alkyl-CO-OR (where R is H or lower alkyl - the same shall apply hereunder), -O-CO-optionally substituted lower alkenylene-CO-OR, -O-CO-optionally substituted aryl, -O-CO-lower alkyl-O-lower alkyl-CO-OR, -O-CO-CO-R, -O-CO-optionally substituted lower alkyl, -O-SO₂-optionally substituted lower alkyl-CO-OR, -O-phthalidyl, 5-methyl-1,3-dioxolen-2-on-4-yl-methyloxy and the like for prodrugs to give OH.

The compounds (I) and their pharmaceutically acceptable salts have blood lipid lowering effect based on their apo B-related lipoprotein secretion-inhibiting activity, and are useful for remedies for hyperlipemia, arteriosclerosis, obesity and pancreatitis. Of the compounds of the invention, those with R² of HO-CO-lower alkyl do not so much inhibit chemical metabolism enzyme cytochrome P450 (CYP), especially CYP3A4, and therefore have few side effects. Accordingly, these are especially useful as medicaments for the above-mentioned diseases. The CYP3A4-inhibiting activity of the compounds may be determined according to the method described in WO02/44179, page 16, lines 9-22.

### Production Methods

The compounds (I) of the invention may be produced according to any of the following first to sixth methods, depending on the type of the substituents therein. In case where the compounds of the invention are optically-active compounds, they may be produced under any of the reaction conditions mentioned below, with no problem of racemation during their production. For example, they may be produced from optically-active compounds (XVII*) mentioned below.

### First Method:

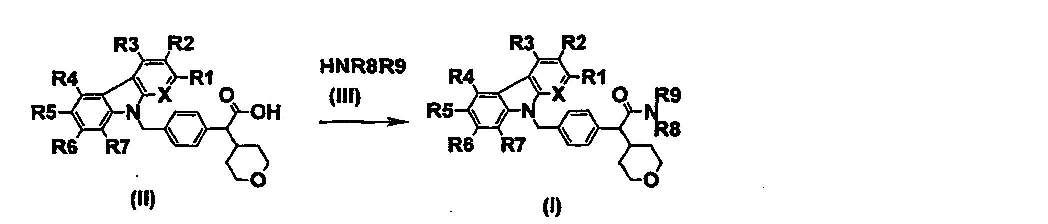

The compounds (I) of the invention may be produced through condensation of a carboxylic acid compound (II) with an amine compound (III). The reaction may be effected in an organic solvent generally not having any influence on the reaction, in the presence of a condensing agent, at room temperature or under heat. The solvent includes methylene chloride, chloroform, 1,2-dichloroethane, dimethylformamide, dimethylacetamide, 1-methylpyrrolidinone, tetrahydrofuran. The condensing agent includes, for example, dicyclohexylcarbodiimide (DCC), 1-ethyl-3-(3-dimethylaminopropyl)carbodiimide hydrochloride (WSC·HCl), diphenylphosphorylazide (DPPA), carbonyldiimidazole (CDI), diisopropylcarbodiimide (DIPCI). The reaction may be effected in the presence of a base. The base includes triethylamine, diisopropylethylamine, 4-methylmorpholine, 1,8-diazabicyclo[5,4,0]-7-undecene (DBU). The reaction may also be effected in the presence of an activator. The activator includes 1-hydroxybenzotriazole (HOBt), hydroxysuccinimide (HOSu), dimethylaminopyridine (DMAP), hydroxyphthalimide (HOPht). Preferably, the compound (II) is reacted with the compound (III) in an equimolar ratio or one of the two is excessive in some degree over the other, and the amount of the condensing agent and the base or the activator to be used is 1 to 2 equivalents to the compound (II) or (III).

Not through the reaction with the above-mentioned condensing agent, the compounds (I) may be obtained as follows: The compound (II) is reacted with thionyl chloride or oxalyl chloride to give an acid chloride, or is reacted with ethyl chloroformate or isopropyl chloroformate to give a mixed acid anhydride or the like, and the resulting reactive derivative is reacted with the compound (III) in the presence of the base mentioned above.

### Second Method:

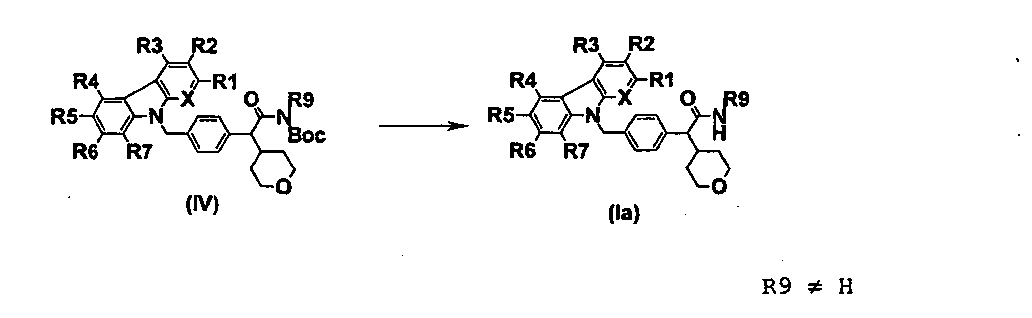

Of the compounds of the invention, secondary amide compounds (Ia) may be produced through deprotection of a compound (IV) of which the amido moiety is protected with a tert-butoxycarbonyl group (Boc group). The reaction may be effected in an organic solvent generally not having any influence on the reaction, such as dichloromethane, chloroform or dichloroethane, in the presence of an acid such as hydrochloric acid or trifluoroacetic acid, at room temperature or under heat.

Some of the compounds (I) of the invention may be produced by further converting the substituents in the compounds obtained through the above-mentioned first or second method into the intended substituents. The substituent conversion may be effected in any desired manner depending on the type of the intended substituents, for example, according to any of the following third to sixth methods.

### Third Method:

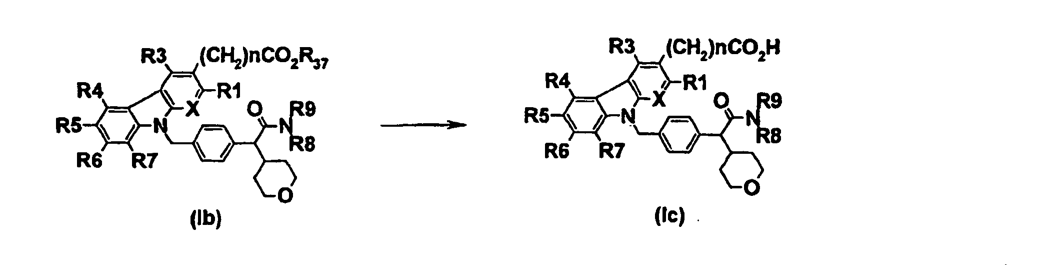

n = 0 to 6; R37 = lower alkyl or benzyl

Of the compounds of the invention, carboxylic acid compounds (Ic) may be produced through hydrolysis of the ester compounds (Ib) obtainable in the first method. In case where R37 is a lower alkyl group, the reaction may be effected in a mixed solvent of an organic solvent generally not having any influence on the reaction, such as methanol, ethanol, tetrahydrofuran or dioxane, and water, in the presence of a base or an acid, of which the amount is the same as or excessive over the ester, at room temperature or under heat. The base may be an inorganic base such as sodium hydroxide, potassium hydroxide, potassium carbonate; and the acid may be hydrochloric acid, sulfuric acid, hydrobromic acid or the like. In case where R37 is a benzyl group, the carboxylic acid compounds (Ic) may be obtained through hydrogenolysis of benzyl esters (Ib). Preferably, the reaction is effected in an organic solvent generally not having any influence on the reaction, such as ethanol, ethyl acetate, tetrahydrofuran, dimethylformamide or acetic acid, in a hydrogen gas atmosphere in the presence of a metal catalyst such as palladium-carbon, at room temperature.

### Fourth Method:

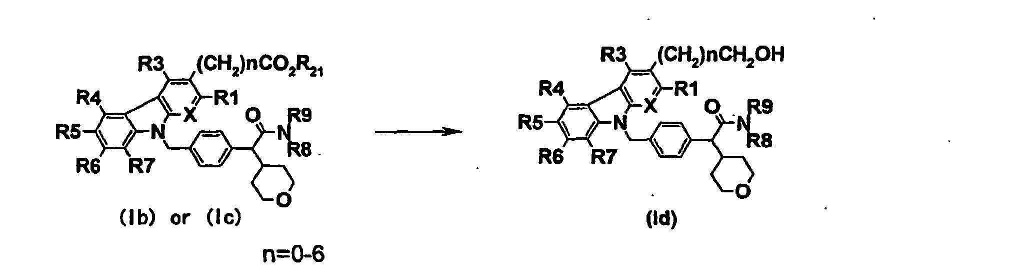

Of the compounds of the invention, alcohol compounds (Id) may be produced through reduction of the ester compounds (Ib) obtained in the first method or the carboxylic acid compounds (Ic) obtainable in the second method. The reaction may be effected in an organic solvent generally not having any influence on the reaction, in the presence of a suitable reducing agent. The solvent includes tetrahydrofuran, diethyl ether, dioxane, dimethoxyethane. The suitable reducing agent includes borane-tetrahydrofuran complex, lithiumaluminium hydride, lithiumboro hydride. Preferably, the amount of the reducing agent to be used is 1 to 2 equivalents to the compound (Ib) or (Ic).

### Fifth Method:

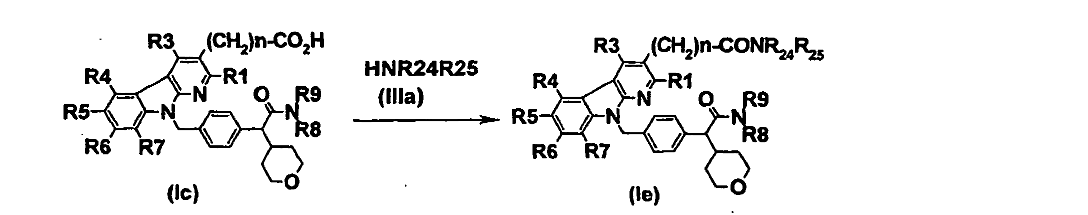

Of the compounds of the invention, amide compound (Ie) may be produced through condensation of the amine compound (Ic) obtainable in the second method with an amine compound (IIIa). The reaction may be effected in the same manner as in the first method.

### Sixth Method:

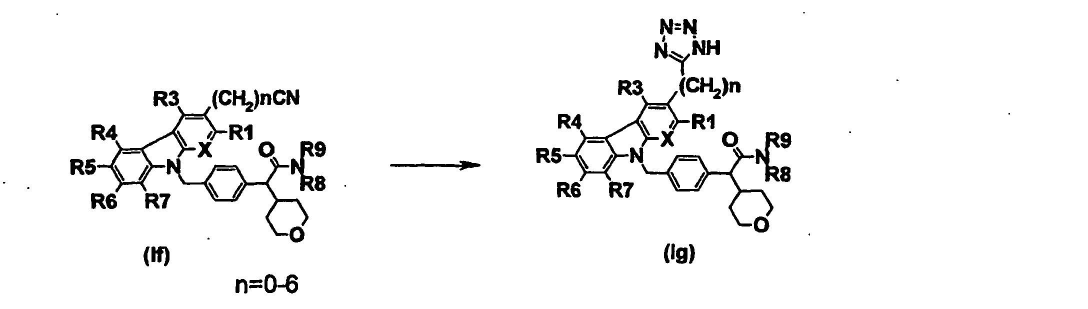

Of the compounds of the invention, tetrazole compounds (Ig) may be produced by reacting the nitrile compound (If) obtainable in the first method with an azidating agent. The reaction may be effected in an organic generally not having any influence on the reaction such as toluene, xylene, benzene, under heat. For the azidating agent, preferred are tributyltin azide and sodium azide.

A method for producing the starting compounds, carboxylic acid compounds (II) in the first method is described below. The carboxylic acid compounds (II) may be produced from a compound (V) and a bromide compound (VI) through the steps 1 and 2 mentioned below.

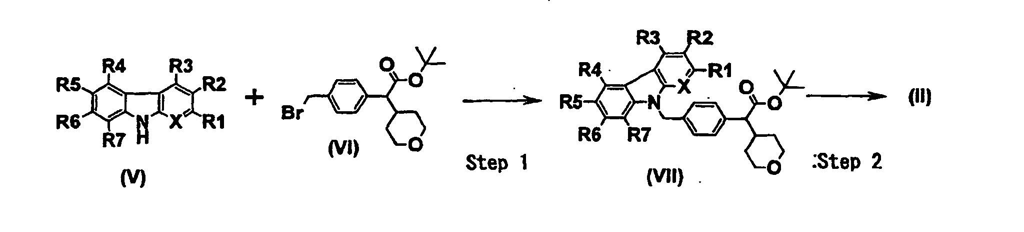

Specifically, the carboxylic acid compounds (II) may be produced through ordinary hydrolysis of tert-butyl ester compounds (VII) under an acid condition usual for those skilled in the art. The reaction may be effected with hydrochloric acid in an organic solvent not having any influence on the reaction such as dioxane or tetrahydrofuran, or with trifluoroacetic acid in an organic solvent such as dichloromethane, chloroform or 1,2-dichloroethane, at room temperature or under heat.

The *tert*-butyl ester compounds (VII) may be produced by reacting a compound (V), which is obtainable in the method mentioned below, with a bromide compound (VI) in the presence of a base. Preferably, the compound (V) is reacted with the compound (VI) in an equimolar ratio or one of the two is excessive in some degree over the other, in an organic solvent generally not having any influence on the reaction, such as dimethylformamide, dimethylacetamide, 1-methylpyrrolidinone or tetrahydrofuran, in the presence of a base that is equimolar to or excessive in some degree over the compound (V), with cooling or at room temperature. For the base, preferred are potassium tert-butoxide and sodium hydride.

Methods for producing the starting compounds (V) and the bromide compounds (VI) are described below in that order.

The compounds (V) may be produced from known 2-halogenonitrobenzene compounds (VIII) through the following steps 3 to 6. 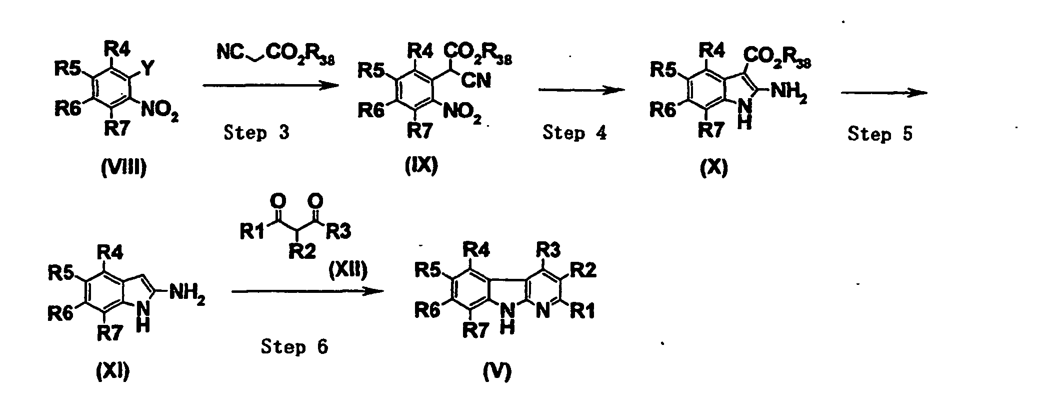 Y = halogen atom
R38 = lower alkyl

### Step 3:

Compounds (IX) may be produced by reacting a compound (VIII) with a cyanoacetate (preferably ethyl cyanoacetate or *tert*-butyl cyanoacetate) in the presence of a base, according to the method described in C.A. Grob, 0. Weissbach, *Helv. Chim. Acta.,* 44, 1748 (1961). For the base, preferred are potassium tert-butoxide and sodium hydride. The reaction may be effected in an organic solvent generally not having any influence on the reaction, such as pyridine, dimethylformamide, dimethylacetamide or 1-methylpyrrolidinone, under heat, in which the cyanoacetate and the base may be two equivalents to the compound (VIII).

### Step 4:

Compounds (X) may be produced by reacting the compound (IX) with a reducing agent, according to the method described in K.L. Munshi, H. Kohl, N.J. de Souza, J. *Heterocyclic Chem.,* 14, 1145 (1977). For the reducing agent, preferred are zinc, iron, and tin(II) chloride. Preferably, the reaction is effected in an acidic solvent such as acetic acid or hydrochloric acid, under heat at 80°C or higher.

### Step 5:

Compounds (XI) may be produced through hydrolysis followed by decarboxylation of the ester compound (X) in an acidic condition, according to the method described in R.A. Glennnon, *J. Heterocyclic Chem.,* 12, 135 (1975). Preferably, the reaction is effected in an acidic solvent such as hydrochloric acid, sulfuric acid or trifluoroacetic acid, under heat for reflux.

### Step 6:

Compounds (V) may be produced by reacting the compound (XI) with a compound (XII) in the presence of a base, according to the method described in R.S. Sagitullin, T.V. Mel'nikova, A.N. Kost, V.F. Snegirev, E.N. Frenkel, *Chemistry of Heterocyclic Compounds* (English translation), 9, 968 )1973). The reaction may be effected in an organic solvent generally not having any influence on the reaction, under heat. The base includes triethylamine and potassium hydroxide. For the solvent, preferred are ethanol and isopropyl alcohol.

Some compounds (V) may be produced by further converting the substituents in the compounds (V) obtained through the steps 3 to 6, into the intended substituents. The substituent conversion may be effected in any desired manner depending on the type of the intended substituents, for example, according to the following process.

Specifically, compounds (Vb), (Vc) and (Vd) may be produced from the compounds (Va) obtainable through the steps 3 to 6, according to the following steps 7-1, 7-2 and 7-3 in that order.

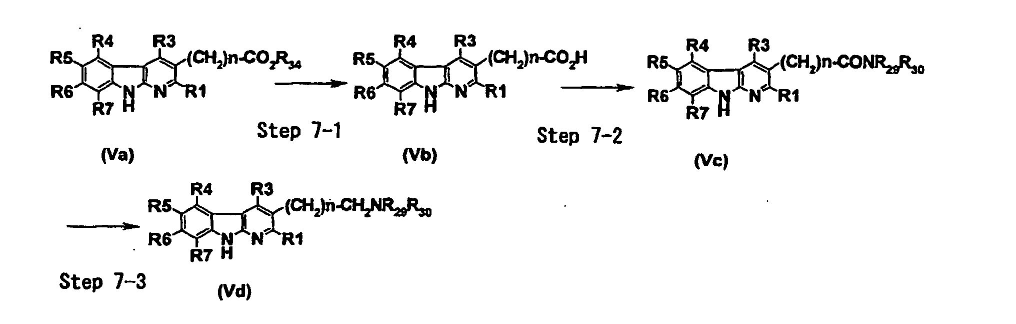

### Step 7-1:

Compounds (Vb) may be produced through hydrolysis of the compounds (Va) with an ordinary acid or base usual for those skilled in the art, and the reaction may be effected in the same manner as in the third method.

### Step 7-2:

Compounds (Vc) may be produced through amidation of the compounds (Vb) with an ordinary condensing agent usual for those skilled in the art, and the reaction may be effected in the same manner as in the first method.

### Step 7-3:

Compounds (Vd) may be produced through reduction of the compounds (Vc). The reaction may be effected in an organic solvent generally not having any influence on the reaction, in the presence of a suitable reducing agent at room temperature or under heat. The solvent includes tetrahydrofuran, diethyl ether, dioxane, dimethoxyethane. The suitable reducing agent includes lithiumaluminium hydride, lithiumboro hydride, diborane-tetrahydrofuran complex. Preferably, the amount of the reducing agent to be sued is 1 to 2 equivalents to the compound (Vc).

Compounds (Ve) and (Vf) may be produced from the above-mentioned compounds (Vb) through the following steps 7-4 and 7-5 in that order.

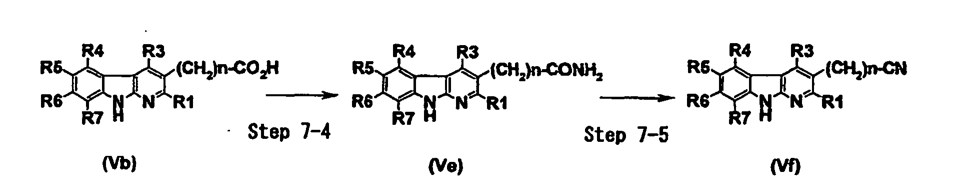

### Step 7-4:

Compounds (Ve) may be produced through amidation of the compounds (Vb) with ammonium chloride, ammonium carbonate, aqueous ammonia, ammonia gas or the like and with an ordinary condensing agent usual for those skilled in the art, and the reaction may be effected in the same manner as in the first method.

### Step 7-5:

Compounds (Vf) may be produced through dehydration of the compounds (Ve). Preferably, the reaction is effected in an organic solvent generally not having any influence on the reaction, in the presence of a dehydrating agent such as phosphorus oxychloride, with cooling.

On the other hand, the bromide compounds (VI) may be produced from known compounds (XIII) through the following steps 8 to 13.

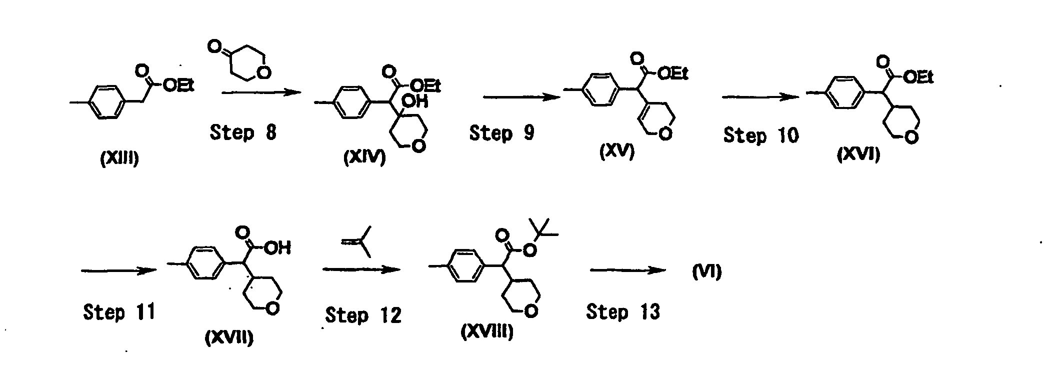

### Step 8:

Compounds (XIV) may be produced by reacting a compound (XIII) with tetrahydro-2*H*-pyran-4-one. The reaction may be effected in an organic solvent generally not having any influence on the reaction, in the presence of a base with cooling at -78°C to -20°C. For the base, preferred are organometal amides such as lithium hexamethyldisilazide and lithium diisopropylamide; and for the solvent, preferred are tetrahydrofuran and diethyl ether.

### Step 9:

Compounds (XV) may be produced through dehydration of the compounds (XIV) under an acidic condition. Preferably, the reaction is effected in an organic solvent not having any influence on the reaction such as ethanol or dioxane, or in water, in the presence of an acid such as sulfuric acid or hydrochloric acid, under heat at 40 to 80°C.

### Step 10:

Compounds (XVI) may be produced through catalytic reduction of the compounds (XV). Preferably, the reaction is effected in an organic solvent generally not having any influence on the reaction such as ethanol, ethyl acetate, tetrahydrofuran or acetic acid, in a hydrogen gas atmosphere in the presence of a metal catalyst such as palladium-carbon, at room temperature.

### Step 11:

Compounds (XVII) may be produced through hydrolysis of the compounds (XVI). The reaction may be effected in a mixed solvent of an organic solvent generally not having any influence on the reaction, such as methanol, ethanol or tetrahydrofuran, and water, in the presence of a base or an acid under heat. For the base, preferred are inorganic bases such as sodium hydroxide, potassium hydroxide; and for the acid, preferred are hydrochloric acid, sulfuric acid, hydrobromic acid.

### Step 12:

Compounds (XVIII) may be produced through *tert*-butylation of the compounds (XVII). The reaction may be effected in an organic solvent generally not having any influence on the reaction such as dichloroethane, dichloromethane or chloroform, at room temperature in the presence of isobutene gas (this is dissolved in the solvent) and by the use of a catalytic amount of sulfuric acid. Alternatively, the compound (XVII) may be reacted with isobutene gas, which is formed in the system by the use of an excessive amount of *tert*-butyl alcohol, in the presence of an excessive amount of anhydrous magnesium sulfate and an equimolar amount of sulfuric acid at room temperature to give the compound, according to the method described in S.T. Wright, D.L. Hageman, A.S. Wright, L.D. McClure, *Tetrahedron Letters,* 38 (42), 7345 (1997).

### Step 13:

The compounds (VI) may be produced through bromination of the compounds (XVIII). The reaction may be effected in an organic solvent generally not having any influence on the reaction such as carbon tetrachloride or ethyl acetate, by the use of a brominating agent such as N-bromosuccinimide, in the presence of a catalytic amount of a radical initiator such as *N,N'*-azobisisobutyronitrile or benzoyl peroxide, under heat for reflux or with exposure to light.

On the other hand, optically-pure compounds (XVII*) may be produced through optical resolution of the above-mentioned racemic compounds (XVII) according to the following steps 14 and 15.

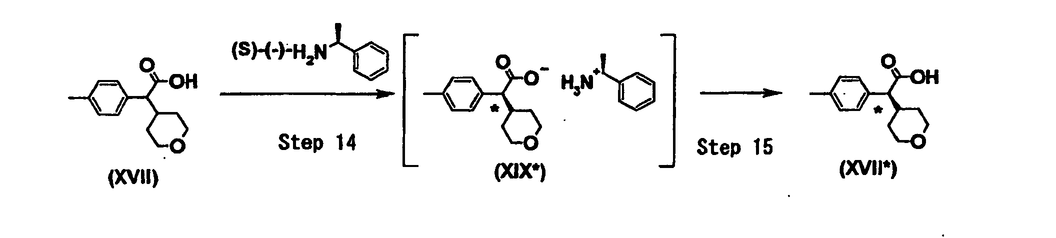

### Step 14:

Compounds (XIX*) that are pure to the level of diastereomers may be produced through differential recrystallization of salts formed by reacting the compound (XVII) with (*S*)-(-)-1-phenylethylamine. The differential recrystallization shall be effected once or multiple times. The reaction of the compound (XVII) with (*S*)-(-)-1-phenylethylamine may be effected in an organic solvent generally not having any influence on the reaction such as tetrahydrofuran or acetonitrile, at room temperature or under heat. Preferably, from 0.5 to 1 equivalent of (*S*)-(-)-1-phenylethylamine is used relative to the racemic compound (XVII). After thus recrystallized, the salt is preferably left at room temperature for a few hours and then taken out through filtration. The subsequent differential crystallization of the (*S*)-(-)-1-phenylethylammonium salt is preferably effected in a solvent of tetrahydrofuran.

### Step 15:

The optically-pure compounds (XVII*) may be obtained by desalting the compounds (XIX*) that are pure to the level of diastereomers. The desalting may be effected in a two-layer system of an organic solvent such as ethyl acetate or chloroform, and water, at room temperature by the use of an equimolar or excessive amount of an acid such as hydrochloric acid or sulfuric acid.

The optically-pure compounds (XVII*) may be led to optically-pure compounds (I*) of the invention through a process that comprises the above-mentioned steps 12, 13, 1 and 2 and the first method in that order.

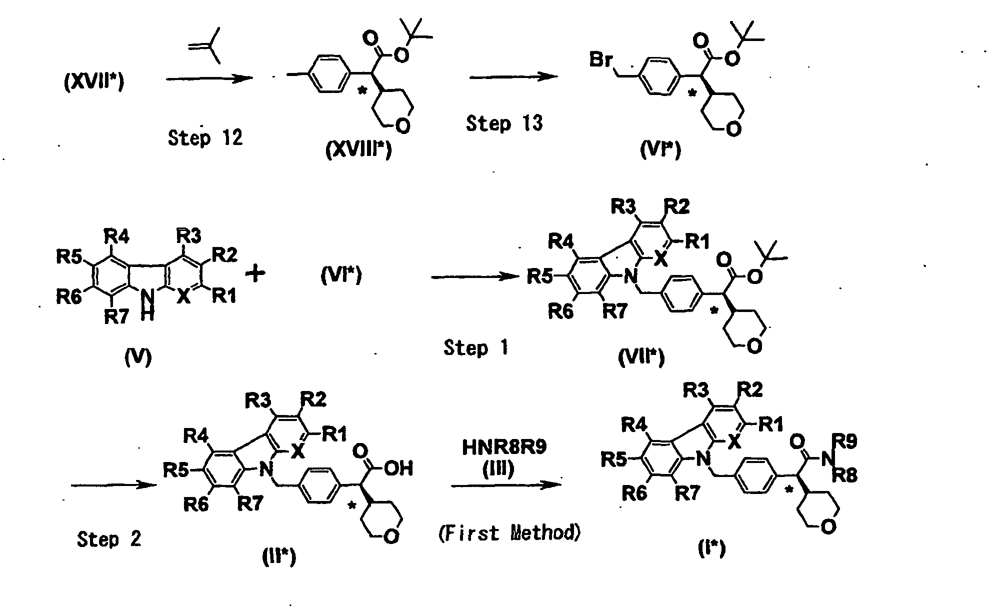

A method for producing Boc-protected amide compounds (IV), which are the starting compounds in the second method, is described below.

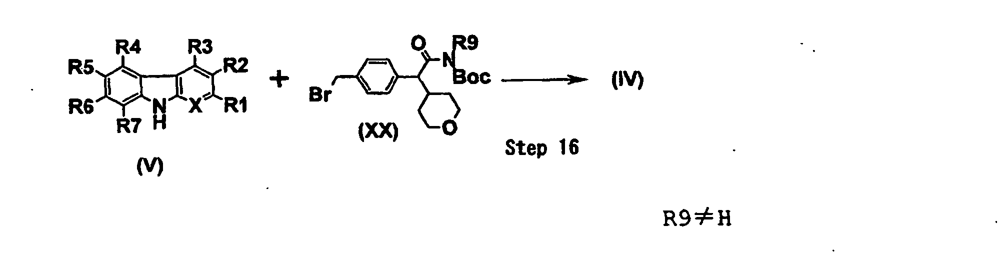

### Step 16:

Compounds (IV) may be produced from the above-mentioned compound (V) and a bromide compound (XX) that is obtainable according to the method mentioned below, in the presence of a base in the same manner as in the step 1. Preferably, the compound (V) is reacted with a compound (XX) in an equimolar ratio or one of the two is excessive in some degree in an organic solvent generally not having any influence on the reaction, such as dimethylformamide, dimethylacetamide, 1-methylpyrrolidinone or tetrahydrofuran, in the presence of a base that is equimolar to or excessive over the compound (V), with cooling or at room temperature. For the base, preferred are potassium tert-butoxide and sodium hydride.

A method for producing the bromide compounds (XX) is described below.

The compounds (XX) may be produced from the above-mentioned carboxylic acid compounds (XVII) through the steps 17 to 19 mentioned below.

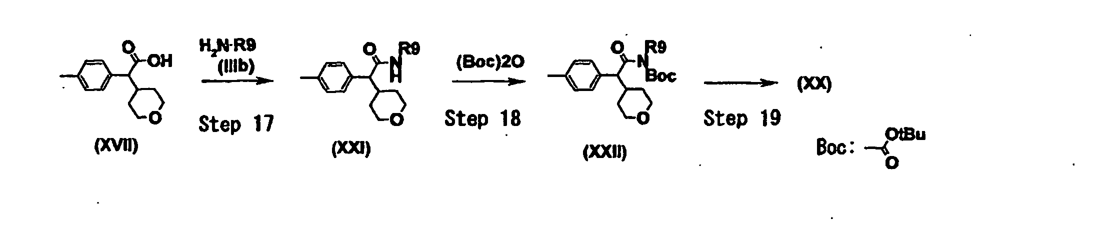

### Step 17:

Secondary amide compounds (XXI) may be produced through condensation of the carboxylic acid compound (XVII) with an amine compound (IIIb), and the reaction may be effected in the same manner as in the first method.

### Step 18:

Compounds (XXII) may be produced by reacting the compound (XXI) with di-*tert*-butyl dicarbonate (DIBOC). The compound (XXI) may be reacted with an equimolar or excessive amount of DIBOC in an organic solvent generally not having any influence on the reaction, such as acetonitrile, tetrahydrofuran or dioxane, in the presence of a catalytic amount of 4-dimethylaminopyridine (DMAP) at room temperature.

### Step 19:

The bromide compounds (XX) may be produced through bromination of the compounds (XXII), and the reaction may be effected in the same manner as in the above-mentioned step 13.

In case where an optically-pure compound (XVII*) is used in the step 17, then it may be led to optically-active compounds (Ia*) of the invention through the steps 18, 19 and 16 and the second method.

The compounds of the invention may be isolated and purified as free compounds, their salts, hydrates, solvates or polymorphic crystals. Pharmaceutically acceptable salts of the compounds (I) of the invention may be produced through ordinary salt formation.

Isolation and purification may be effected in any ordinary chemical operation such as extraction, differential crystallization, various modes of partitioning chromatography.

Isomers may be separated from each other by selecting suitable starting compounds, or by utilizing the difference in the physical properties between the isomers. For example, optical isomers may be led to stereochemically-pure isomers by selecting suitable starting materials or through racemic isolation of racemic compounds (for example, by leading a racemic compound to a diastereomer salt with an ordinary optically-active base followed by optically resolving it).

The pharmaceutical compositions that contain, as the active ingredient, one or more of the compounds and their salts of the invention may be prepared by the use of a carrier, a vehicle and other additives generally used in formulating pharmaceutical compositions.

They may be orally administered in any form of tablets, pills, capsules, granules, powders or liquids, or may be parenterally administered in any form of injections such as intravenous injections or intramuscular injections, or suppositories or subcutaneous preparation. Their dose may be suitably determined, depending on the condition, the age and the sex of the patients to which they are administered, but is, in general, from 0.01 to 500 mg/adult/day for oral administration, and from 0.001 to 100 mg/adult/day for parenteral administration. This may be administered to the patients all at a time, or may be divided into a few portions for administration in 2 to 4 times.

As the solid composition for oral administration of the compounds of the invention, employed are tablets, powders, granules, etc. The solid composition of those types comprises one or more active substances along with at least one inert diluent, such as lactose, mannitol, glucose, hydroxypropyl cellulose, microcrystalline cellulose, starch, polyvinyl pyrrolidone, magnesium metasilicate aluminate. In an ordinary manner, the composition may contain any other additives except the inert diluents noted above, for example, a lubricant such as magnesium stearate, a disintegrator such as calcium cellulose glycolate, a stabilizer such as lactose, and a dissolution promoter such as glutamic acid or aspartic acid. If desired, the tablets and pills may be coated with a film of sugar or gastric or enteric substances such as sucrose, gelatin, hydroxypropyl cellulose, hydroxypropylmethyl cellulose phthalate.

The liquid composition for oral administration includes, for example, pharmaceutically acceptable emulsions, solutions, suspensions, syrups, elixirs and the like, which contain ordinary inactive diluents such as pure water or ethanol. In addition to the inert diluents, those compositions may further contain pharmaceutical aids such as wetting promoters, suspension promoters, and also sweeteners, flavorings, aromas and preservatives.

Injection is one typical example of parenteral administration. It includes, for example, germ-free, aqueous or non-aqueous solutions, suspensions and emulsions. The diluent for the aqueous solutions and suspensions includes, for example, distilled water and physiological saline for injections. The diluent for the non-aqueous solutions and suspensions includes, for example, propylene glycol, polyethylene glycol, vegetable oils such as olive oil, alcohols such as ethanol, Polysolvate 80 (trade name). Those compositions may further contain additives such as preservatives, wetting promoters, emulsifiers, dispersants, stabilizers (e.g., lactose), dissolution promoters (e.g., glutamic acid, aspartic acid). These are sterilized by filtering them through bacteria-trapping filters, or by adding microbicides thereto, or by exposing them to radiations. Germ-free, solid compositions may be produced previously, and they may be dissolved in germ-free water or in germ-free solvents for injection, before using them.

### EXAMPLES

The invention is described in more detail with reference to the following Examples, which, however, are not intended to restrict the scope of the invention. Methods of producing the starting compounds used in the Examples are shown as Reference Examples.

### Reference Example 1:

### Ethyl (4-hydroxytetrahydro-2H-pyran-4-yl)(4-methylphenyl)acetate

550 ml of a solution of 1 M lithium bis(trimethylsilyl)amide in tetrahydrofuran (THF) was cooled to -70°C in argon, and 50 ml of a THF solution of 92.5 g of ethyl (4-methylphenyl)acetate was dropwise added thereto over a period of 30 minutes, and then stirred at the temperature for 30 minutes. 50 ml of a THF solution of 52.3 g of tetrahydro-2*H*-pyran-4-one was dropwise added to the reaction liquid over a period of 30 minutes, still with cooling at -70°C, and then stirred at the temperature for 1 hour. 1000 ml of an aqueous 10 % citric acid solution was added to the reaction liquid, and extracted with 500 ml of ethyl acetate. The organic layer was washed with 500 ml of an aqueous 10 % citric acid solution and 500 ml of saturated saline in that order. This was dried with anhydrous magnesium sulfate, and the solvent was evaporated away under reduced pressure to obtain a pale yellow crystal. The crystal was washed with 400 ml of n-hexane to obtain 145.7 g of a colorless crystal of the entitled compound.
FAB-MS m/z:279(M⁺+1)

### Reference Example 2:

### Ethyl 3,6-dihydro-2H-pyran-4-yl(4-methylphenyl)acetate

300 ml of concentrated sulfuric acid was gradually dropwise added to 300 ml of ethanol, and cooled to 10°C in an ice bath. To the solution, added was 145 g of ethyl (4-hydroxytetrahydro-2*H*-pyran-4-yl)(4-methylphenyl)acetate produced in Reference Example 1, and then stirred at 60°C for 3 hours. 500 ml of ethyl acetate and 1500 ml of water were added to the reaction liquid, and the aqueous phase was extracted with 500 ml of ethyl acetate. The two ethyl acetate layers were combined, washed with 500 ml of saturated saline, and dried with anhydrous magnesium sulfate. The solvent was evaporated away under reduced pressure, and the residue was subjected to silica gel column chromatography for elution with ethyl acetate/n-hexane (1/9, v/v) to obtain 92.0 g of a pale yellow oil of the entitled compound.
FAB-MS m/z:261 (M⁺+1)

### Reference Example 3:

### Ethyl (4-methylphenyl)(tetrahydro-2H-pyran-4-yl)acetate

92.0 g of ethyl 3,6-dihydro-2*H*-pyran-4-yl(4-methylphenyl)acetate produced in Reference Example 2 was dissolved in 800 ml of ethanol, to which was added 10.2 g of 10 % Pd-C in an Ar atmosphere. The reactor was purged with hydrogen gas, and then the matter therein was vigorously stirred at room temperature for 4 hours. The reactor was again purged with Ar gas, and then the insoluble matter was taken away through filtration. The solvent was evaporated away under reduced pressure, and 89.9 g of a pale yellow oil of the entitled compound was obtained.
FAB-MS m/z:263(M⁺+1)

### Reference Example 4:

### (4-Methylphenyl)(tetrahydro-2H-pyran-4-yl)acetic acid

89.7 g of ethyl (4-methylphenyl)(tetrahydro-2*H*-pyran-4-yl)acetate produced in Reference Example 3 was dissolved in 300 ml of ethanol, to which was added 170 ml of an aqueous 8 M sodium hydroxide solution, and heated under reflux for 4.5 hours. Ethanol was evaporated away under reduced pressure, and 120 ml of concentrated hydrochloric acid was added to the residue. This was then extracted with 500 ml of ethyl acetate. The organic layer was washed with 200 ml of saturated saline, and dried with anhydrous magnesium sulfate, and the solvent was evaporated away under reduced pressure to obtain a colorless oil. This was dried under reduced pressure, and crystallized to obtain 74.5 g of a colorless oil of the entitled compound.
FAB-MS m/z:233 (M⁺-1)

### Reference Example 5:

### (2S)-(4-methylphenyl)(tetrahydro-2H-pyran-4-yl)acetic acid

(4-Methylphenyl)(tetrahydro-2*H*-pyran-4-yl)acetic acid produced in Reference Example 4 was optically resolved in the manner mentioned below. 74.1 g of (±)-2-(4-methylphenyl)-2-(tetrahydro-2*H*-pyran-4-yl)acetic acid was dissolved in 700 ml of acetonitrile at 80°C. 16.0 g of triethylamine was added thereto, and 19.2 g of (*S*)-(-)-1-phenylethylamine was gradually dropwise added thereto. This gave a colorless crystal. The crystal was cooled to room temperature, and then stirred at the temperature for 2 hours. The crystal thus formed was taken out through filtration, washed with 100 ml of acetonitrile, and dried to obtain 55.0 g of a colorless crystal. This was dissolved in 2600 ml of THF under heat for reflux. About 300 ml of THF was evaporated away under heat, and then the residue was cooled to room temperature to give a colorless crystal. This was stirred overnight at the temperature. The crystal thus formed was taken out through filtration, washed with 500 ml of THF and dried to obtain 43.6 g of a colorless crystal, (*S*)-(-)-1-phenylethylamine salt of the entitled compound. To this were added 700 ml of ethyl acetate and 500 ml of 1 M hydrochloric acid, and vigorously stirred at room temperature for 1 hour. The ethyl acetate layer was washed with saturated saline, and dried with anhydrous magnesium sulfate, and the solvent was evaporated away under reduced pressure to obtain 29.0 g of a colorless crystal of the entitled compound.
[α]25,D=48.3 (c=1.00, CHCl3)

### Reference Example 6:

### tert-Butyl (2S)-(4-methylphenyl)(tetrahydro-2H-pyran-4-yl)acetate

64.5 g of (*2S*)-(4-methylphenyl)(tetrahydro-2H-pyran-4-yl)acetic acid produced in Reference Example 5 was dissolved in 900 ml of methylene chloride, to which was added 2.5 ml of concentrated sulfuric acid. Isobutene gas was introduced into the reaction liquid with cooling to -70°C, to liquefy it. About 500 ml of the resulting liquid was collected, and this was stirred overnight at room temperature. (If desired, 500 ml of isobutene gas was again added thereto with cooling, and further stirred overnight at room temperature.) The solvent was evaporated away under reduced pressure, and 1000 ml of chloroform was added to the residue. This was washed with 1000 ml of an aqueous saturated sodium hydrogencarbonate solution and 1000 ml of saturated saline in that order. The organic layer was dried with anhydrous magnesium sulfate, and the solvent was evaporated away under reduced pressure to obtain 70.5 g of a colorless solid of the entitled compound.
FAB-MS m/z:291(m⁺+1); [α]25,D=17.7 (c=1.00, CHCl3)

### Reference Example 7:

### tert-Butyl (2S)-(4-bromomethylphenyl)(tetrahydro-2H-pyran-4-yl)acetate

46.5 g of *tert*-butyl (25)-(4-methylphenyl)(tetrahydro-2*H*-pyran-4-yl)acetate produced in Reference Example 6 was dissolved in 600 ml of carbon tetrachloride, to which were added 1.3 g of 2,2'-azobisisobutyronitrile (AIBN) and 34.2 g of N-bromosuccinimide (NBS) in that order at 50°C. Then, the reaction liquid was heated under reflux for 1 hour. This was cooled with ice, and the insoluble matter was taken away through filtration. The solvent was evaporated away under reduced pressure. The residue was subjected to silica gel column chromatography for elution with ethyl acetate/n-hexane (1/20 to 1/10, v/v) to obtain 34.5 g of a colorless crystal of the entitled compound.
¹H NMR (CDCl3) δ: 1.08-1.18 (2H,m), 1.38-1.41 (10H,m), 2.08-2.25 (1H,m), 3.14 (1H,d), 3.22-3.33 (1H,m), 3.42 (1H,dt), 3.80-3.90 (1H,m), 3.93-4.03 (1H,m), 4.48 (2H,s), 7.29 (2H,d), 7.34 (2H,d)
[α]25, D=8.9 (c=1.00, CHCl3)

### Reference Example 8:

### N-benzyl-2-(4-methylphenyl)-2-(tetrahydro-2H-pyran-4-yl)acetamide

8.6 ml of benzylamine was dissolved in 150 ml of DMF, and 15.4 g of (4-methylphenyl)(tetrahydro-*2H*-pyran-4-yl)acetic acid produced in Reference Example 4, 15.1 g of 1-ethyl-3-(3-dimethylaminopropyl)carbodiimide hydrochloride (WSC.HC1) and 10.7 g of 1-hydroxybenzotriazole (HOBt) were added thereto in that order at room temperature and stirred overnight at the temperature. The reaction liquid was evaporated under reduced pressure, and ethyl acetate and an aqueous saturated sodium hydrogencarbonate solution were added to the residue, and then extracted with ethyl acetate. The organic layer was washed with water, 1.0 M hydrochloric acid and saturated saline, and dried with anhydrous magnesium sulfate, and the solvent was evaporated away under reduced pressure. The residue was recrystallized from a mixed solvent of diethyl ether and n-hexane to obtain 17.6 g of a colorless crystal of the entitled compound.
FAB-MS m/z:424(M⁺+1)

### Reference Example 9:

### tert-Butyl (±)-N-benzyl[(4-methylphenyl)(tetrahydro-2H-pyran-4-yl)]ethanoylcarbamate

15.6 g of *N*-benzyl-2-(4-methylphenyl)-2-(tetrahydro-*2H*-pyran-4-yl)acetamide produced in Reference Example 8 was dissolved in 160 ml of acetonitrile, and 589 mg of 4-dimethylaminopyridine (DMAP) and 12.6 g of di-*tert*-butyl dicarbonate (Boc20) were added thereto in that order at room temperature, and stirred overnight at the temperature. Ethyl acetate and distilled water were added to the reaction liquid. The organic layer was washed with saturated saline, and then dried with anhydrous magnesium sulfate, and the solvent was evaporated away under reduced pressure. The residue was subjected to silica gel column chromatography for elution with a mixed solvent of *n*-hexane/ethyl acetate (4/1, v/v) to obtain 16.8 g of a colorless oil of the entitled compound.
FAB-MS m/z:424(M⁺+1)

### Reference Example 10:

### tert-Butyl N-benzyl-[(4-bromomethylphenyl)(tetrahydro-2H-pyran-4-yl)]ethanoylcarbamate

16.8 g of *tert*-butyl *N*-benzyl[(4-methylphenyl)(tetrahydro-*2H*-pyran-4-yl)]ethanoylcarbamate produced in Reference Example 9 was dissolved in 170 ml of carbon tetrachloride, to which were added 326 mg of 2,2'-azobisisobutyronitrile (AIBN) and 7.42 g of N-bromosuciinimide (NBS) in that order at 60°C. Then, the reaction liquid was heated under reflux for 1 hour. This was evaporated under reduced pressure, and the residue was subjected to silica gel column chromatography for elution with ethyl acetate/*n*-hexane (1/15 to 1/9, v/v) to obtain 13.8 g of a colorless foam substance of the entitled compound.
FAB-MS m/z: 402 (M⁺-Boc), ¹H NMR (DMSO) δ: 1.31 (9H,s)

### Reference Example 11:

### 4-Chloro-3-nitrobenzenesulfonyl chloride

26.2 g of sodium 4-chloro-3-nitrobenzenesulfonate was dissolved in 250 ml of 1,2-dichloroethane, to which were added 26.2 g of thionyl chloride and 4.0 g of DMF. Then, this was heated under reflux for 6 hours. The solvent was evaporated away under reduced pressure, 400 ml of ethyl acetate was added to the residue, and this was washed with an aqueous saturated sodium hydrogencarbonate solution and saturated saline in that order, and dried with anhydrous magnesium sulfate. The solvent was evaporated away under reduced pressure to obtain 12.3 g of a colorless oil of the entitled compound.
¹H NMR (CDCl3) δ: 7.88 (1H,d), 8.18 (1H,dd), 8.54 (1H,d)

### Reference Example 12:

### 4-Chloro-N,N-dimethyl-3-nitrobenzenesulfonamide

7.7 g of 4-chloro-3-nitrobenzenesulfonyl chloride produced in Reference Example 11 was dissolved in 80 ml of 1,2-dichloroethane, to which were added 2.7 g of dimethylamine hydrochloride and 7.4 g of triethylamine with cooling with ice, and stirred at room temperature for 2 hours. The reaction liquid was washed with 1 M hydrochloric acid, saturated sodium hydrogencarbonate and saturate saline in that order, and then dried with anhydrous magnesium sulfate. The solvent was evaporated away under reduced pressure, and the residue was subjected to silica gel column chromatography for elution with chloroform to obtain 4.12 g of a pale yellow solid of the entitled compound.
FAB-MS m/z:264 (M⁺+1)

### Reference Example 13:

### Ethyl cyano-[4-(N,N-dimethylaminosulfonyl)-2-nitrophenyl]acetate

3.5 g of potassium *tert*-butoxide was dissolved in 50 ml of pyridine, and 3.5 g of ethyl cyanoacetate and a pyridine solution (15 ml) of 4.1 g of 4-chloro-*N,N*-dimethyl-3-nitrobenzenesulfonamide that had been produced in Reference Example 12 were added thereto in that order, and stirred at 80°C for 30 minutes. The solvent was evaporated away under reduced pressure, and 150 ml of toluene and 200 ml of water were added thereto. The aqueous layer was acidified with 1 M hydrochloric acid, and extracted with 200 ml of ethyl acetate. The organic layer was washed with saturated saline. This was dried with anhydrous magnesium sulfate, and the solvent was evaporated away under reduced pressure to obtain 5.76 g of a red oil of the entitled compound.
FAB-MS m/z:342(M⁺+1)

### Reference Example 14:

### Ethyl 2-amino-6-(N,N-dimethylaminosulfonyl)-1H-indole-3-carboxylate

9.2 g of ethyl cyano-[4-(*N,N*-dimethylaminosulfonyl)-2-nitrophenyl]acetate produced in Reference Example 13 was dissolved in 100 ml of acetic acid, and 4.5 g of reduced iron was added thereto at 100°C. After reacted vigorously, this was stirred at 100°C for 1 hour. The reaction liquid was cooled to room temperature, and the insoluble matter was filtered away. 200 ml of ethyl acetate and 100 ml of 1 M hydrochloric acid were added to the residue, and the organic layer was washed with 1 M hydrochloric acid and saturated saline in that order, and then dried with anhydrous magnesium sulfate. The solvent was evaporated away under reduced pressure. 60 ml of diisopropyl ether was added to the residue, and heated under reflux for 20 minutes. This was cooled to room temperature, and the insoluble matter was taken out through filtration and dried to obtain 4.83 g of a bluish violet solid of the entitled compound.
FAB-MS m/z:312(M⁺+1)

### Reference Example 15:

### tert-Butyl 2-amino-6-cyano-1H-indole-3-carboxylate

18.4 g of potassium tert-butoxide was dissolved in 150 ml of pyridine, to which were added 17 ml of *tert*-cyanoacetate and 15.0 g of 1-chloro-5-cyano-2-nitrobenzene at room temperature, and stirred at 80°C for 4 hours. The solvent was evaporated away under reduced pressure, 200 ml of water was added to the residue, and this was washed with 300 ml of toluene. 120 ml of concentrated hydrochloric acid was added to the aqueous layer, and then extracted with 300 ml of ethyl acetate. The organic layer was washed with saturated saline, and then dried with anhydrous magnesium sulfate, and the solvent was evaporated away under reduced pressure to obtain 22.9 g of a dark red oil of crude tert-butyl (±)-cyano-(4-cyano-2-nitrophenyl)acetate. Not further purified, this was used in the next reaction.

22.6 g of the crude tert-butyl (±)-cyano-(4-cyano-2-nitrophenyl)acetate was dissolved in 200 ml of acetic acid, and heated up to 100°C. 13.2 g of reduced iron was added to it, and stirred at 100°C for 1.5 hours. This was cooled to room temperature, the insoluble matter was filtered away, and the solvent was evaporated away under reduced pressure. 300 ml of ethyl acetate and 300 ml of 1 M hydrochloric acid were added to the residue, and the reaction liquid was filtered through Celite. The filtrate was extracted with ethyl acetate, and the organic layer was washed with 1 M hydrochloric acid and saturated saline, and dried with anhydrous magnesium sulfate. The solvent was evaporated away under reduced pressure, and the residue was subjected to silica gel column chromatography for elution with ethyl acetate/*n*-hexane (3/7, v/v) to obtain 7.94 g of a dark brown foam substance of the entitled compound.
FAB-MS m/z:258 (M⁺+1)

### Reference Example 16:

### 6-(N,N-dimethylaminosulfonyl)-2-imino-2,3-dihydro-1H-indol monohydrochloride

20 ml of concentrated hydrochloric acid was added to 4.83 g of ethyl 2-amino-6-(*N,N*-dimethylaminosulfonyl)-*1H*-indole-3-carboxylate that had been produced in Reference Example 14, and this was heated under reflux for 1 hour. The solvent was evaporated away under reduced pressure, 20 ml of ethanol was added to the residue to dissolve it at 80°C, and this was restored to room temperature. 20 ml of diethyl ether was added to it, and the solid thus precipitated was taken out through filtration and dried to obtain 3.14 g of a pale brown solid of the entitled compound.
FAB-MS m/z:240(M⁺+1)

### Reference Example 17:

### 6-Cyano-2-imino-2,3-dihydro-1H-indole monohydrochloride

3.00 g of *tert*-butyl 2-amino-6-cyano-*1H*-indole-3-carboxylate produced in Reference Example 15 was dissolved in 30 ml of 1,2-dichloroethane, to which was added 15 ml of trifluoroacetic acid at room temperature and stirred overnight. The solvent was evaporated away under reduced pressure, the residue was dissolved in 30 ml of ethyl acetate, and 4 ml of a solution of 4 M hydrochloride in ethyl acetate was added thereto. The resulting solid was taken out through filtration and dried to obtain 1.84 g of a gray solid of the entitled compound.
FAB-MS m/z:158(M⁺+1)

### Reference Example 18:

### Ethyl 3-(2,4-dimethyl-9H-pyrido[2,3-b]indol-3-yl)propanoate

80 ml of isopropyl alcohol was added to 8.0 g of 2-imino-2,3-dihydro-*1H*-indole monohydrochloride, to which were added 9.5 g of ethyl 4-acetyl-5-oxohexanoate and 14.4 g of triethylamine in that order, and stirred overnight at 80°C. The reaction liquid was cooled to room temperature, and the crystal formed was taken out through filtration. 90 ml of methanol was added to it, the resulting suspension was heated under reflux as it was, and then cooled to room temperature. The crystal formed was taken out through filtration and dried to obtain 7.75 g of a gray crystal of the entitled compound.

In the same manner as in Reference Example 18, the compounds shown in Table 1 below were produced. The meanings of the abbreviations in the Table are mentioned below.
Rex: Reference Example
DATA: physicochemical properties
NMR: proton nuclear magnetic resonance spectrum (TMS internal standard) δ, DMSO-d₆ unless otherwise specifically indicated.

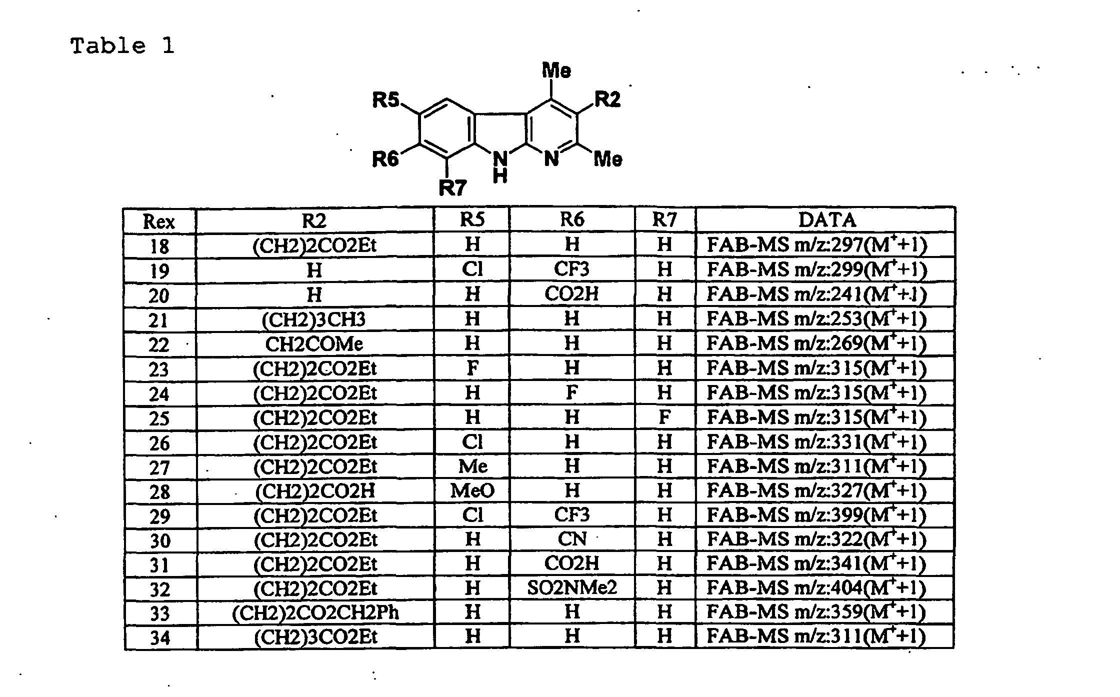

### Reference Example 35:

### Methyl 3-(2,4-dimethyl-9H-pyrido[2,3-b]indol-3-yl)-7-carboxylate

1.19 g of 3-(2,4-dimethyl-*9H*-pyrido[2,3-b]indol-3-yl)-7-carboxylic acid produced in Reference Example 20 was dissolved in 50 ml of methanol, to which was added 0.5 ml of concentrated sulfuric acid, and overnight heated under reflux. The solvent was evaporated away under reduced pressure, 100 ml of chloroform was added to the residue, and this was then washed with saturated sodium hydrogencarbonate and saturated saline in that order. The organic layer was dried with anhydrous magnesium sulfate, and the solvent was evaporated away under reduced pressure to obtain 930 mg of a brown solid of the entitled compound. FAB-MS m/z:255(M⁺+1)

### Reference Example 36:

### Methyl 3-(2,4-dimethyl-7-methoxycarbonyl-9H-pyrido[2,3-b]indol-3-yl)propanoate

1.0 g of ethyl 3-(7-carboxy-2,4-dimethyl-*9H*-pyrido[2,3-b]indol-3-yl)propanoate produced in Reference Example 31 was dissolved in 20 ml of methanol, to which was added 0.5 ml of concentrated sulfuric acid and stirred overnight. The solvent was evaporated away under reduced pressure, 100 ml of chloroform was added to the residue, and this was washed with saturated sodium hydrogencarbonate and saturated saline in that order. The organic layer was dried with anhydrous magnesium sulfate, and the solvent was evaporated away under reduced pressure to obtain 840 mg of a colorless solid of the entitled compound.
FAB-MS m/z:341(M⁺+1)

### Reference Example 37:

### (2,4-Dimethyl-9H-pyrido[2,3-b]indol-3-yl)acetic acid

870 mg of methyl (2,4-dimethyl-*9H*-pyrido[2,3-b]indol-3-yl)acetate produced in Reference Example 22 was dissolved in 16 ml of methanol, to which was added 7 ml of aqueous 1 M sodium hydroxide solution and heated under reflux for 19 hours. Aqueous citric acid was added to it, and the solid thus formed was taken out through filtration to obtain 760 mg of a colorless solid of the entitled compound.
FAB-MS m/z:255(M⁺+1)

### Reference Example 38:

### (2,4-Dimethyl-9H-pyrido[2,3-b]indol-3-yl)-N,N-dimethylacetamide

400 mg of (2,4-dimethyl-*9H*-pyrido[2,3-b]indol-3-yl)acetic acid produced in Reference Example 37 was dissolved in 8 ml of DMF, to which were added 256 mg of dimethylamine hydrochloride, 0.66 ml of triethylamine, 361 mg of 1-ethyl-3-(3-dimethylaminopropyl)carbodiimide hydrochloride (WSC·HCl) and 255 mg of 1-hydroxybenzotriazole (HOBt) in that order at room temperature, and this was stirred overnight at the temperature. Aqueous sodium hydrogencarbonate was added to the reaction liquid, and the solid formed was taken out through filtration to obtain 320 mg of a colorless solid of the entitled compound.
FAB-MS m/z:282(M⁺+1)

### Reference Example 39:

### 2-(2,4-Dimethyl-9H-pyrido[2,3-b]indol-3-yl)-1-(morpholin-4-yl)ethanone

360 mg of (2,4-dimethyl-*9H*-pyrido[2,3-b]-indol-3-yl)acetic acid produced in Reference Example 37 was dissolved in 8 ml of DMF, to which were added 0.25 g of morpholine, 361 mg of 1-ethyl-3-(3-dimethylaminopropyl)carbodiimide hydrochloride (WSC·HCl) and 255 mg of 1-hydroxybenzotriazole (HOBt) in that order at room temperature, and this was stirred overnight at the temperature. Aqueous sodium hydrogencarbonate was added to the reaction liquid, and the solid formed was taken out through filtration to obtain 350 mg of a colorless solid of the entitled compound.
FAB-MS m/z:324 (M⁺+1)

### Reference Example 40:

### 2,4-Dimethyl-3-[2-(N,N-dimethylamino)ethyl]-9H-pyrido[2,3-b]indole

320 mg of (2,4-dimethyl-*9H*-pyrido[2,3-b]indol-3-yl)-*N,N*-dimethylacetamide produced in Reference Example 38 was added to a THF solution of 75 mg of lithiumaluminium hydride, and heated under reflux for 3.5 hours. 120 mg of lithiumaluminium hydride was added to the reaction liquid, and heated under reflux for 4 hours. Distilled water, aqueous 1 M sodium hydroxide solution and ethyl acetate were added in that order to the reaction liquid, and filtered through Celite. The reaction liquid was extracted with ethyl acetate, and washed with saturated saline, and the organic layer was dried with anhydrous magnesium sulfate. The solvent was evaporated away under reduced pressure to obtain 288 mg of a pale yellow solid of the entitled compound.
FAB-MS m/z:324(M⁺+1)

### Reference Example 41:

### 2,4-Dimethyl-3-[2-(morpholin-4-yl)ethyl]-9H-pyrido[2,3-b]indole

350 mg of 2-(2,4-dimethyl-*9H*-pyrido[2,3-b]indol-3-yl)-1-(morpholin-4-yl)ethanone produced in Reference Example 39 was added to a THF solution of 82 mg of lithiumaluminium hydride, and heated under reflux for 9 hours. Distilled water, aqueous 1 M sodium hydroxide solution and ethyl acetate were added in that order to the reaction liquid, and filtered through Celite. The reaction liquid was extracted with ethyl acetate, and washed with saturated saline, and the organic layer was dried with anhydrous magnesium sulfate. The solvent was evaporated away under reduced pressure to obtain 338 mg of a pale yellow solid of the entitled compound.
FAB-MS m/z:310(M⁺+1)

### Reference Example 42:

### 3-(2,4-Dimethyl-9H-pyrido[2,3-b]indol-3-yl)propanoic acid

2.40 g of ethyl 3-(2,4-dimethyl-*9H*-pyrido[2,3-b]indol-3-yl)propanoate produced in Reference Example 18 was dissolved in 30 ml of ethanol, to which was added 16 ml of aqueous 1 M sodium hydroxide solution and overnight heated under reflux. Aqueous citric acid was added to it, and the solid formed was taken out through filtration to obtain 2.17 g of a colorless solid of the entitled compound. FAB-MS m/z:269(M⁺+1)

### Reference Example 43:

### 3-(2,4-Dimethyl-9H-pyrido[2,3-b]indol-3-yl)propanamide

268 mg of 3-(2,4-dimethyl-*9H*-pyrido[2,3-b]indol-3-yl)propanoic acid amide produced in Reference Example 42 was dissolved in 4 ml of DMF, to which were added 288 mg of 1-ethyl-3-(3-dimethylaminopropyl)carbodiimide hydrochloride (WSC·HCl) and 203 mg of 1-hydroxybenzotriazole (HOBt) in that order at room temperature, and this was stirred overnight at the temperature. 160 mg of ammonium chloride and 0.70 ml of diisopropylethylamine were added to the reaction liquid, and stirred overnight. Aqueous sodium hydrogencarbonate was added to the reaction liquid, and the solid formed was taken out through filtration to obtain 230 mg of a colorless solid of the entitled compound.
FAB-MS m/z:268 (M⁺+1)

### Reference Example 44:

### 3-(2,4-Dimethyl-9H-pyrido[2,3-b]indol-3-yl]propionitrile

267 mg of 3-(2,4-dimethyl-*9H*-pyrido[2,3-b]indol-3-yl)propanamide produced in Reference Example 43 was dissolved in 2.7 ml of DMF, to which was added 0.11 ml of phosphorus oxychloride with cooling with ice, and stirred for 1.5 hours. Ethyl acetate and aqueous sodium hydrogencarbonate were added to the reaction liquid in that order, and the solid formed was taken out through filtration and dried to obtain 92 mg of a pale red solid of the entitled compound. The filtrate was extracted with ethyl acetate, and washed with saturated saline, and the organic layer was dried with anhydrous magnesium sulfate. The solvent was evaporated away under reduced pressure to obtain 142 mg of a colorless solid of the entitled compound.
FAB-MS m/z:250(M⁺+1)

### Reference Example 45:

### Ethyl (+)-3-(9-{4-[(S)-2-(tert-butoxy)-2-oxo-1-(tetrahydro-2H-pyran-4-yl)ethyl]benzyl}-2,4-dimethyl-9H-pyrido[2,3-b]indol-3-yl)propanoate

7.7 g of ethyl 3-(2,4-dimethyl-*9H*-pyrido[2,3-b]indol-3-yl)propanoate produced in Reference Example 18 was dissolved in 150 ml of *N,N*-dimethylformamide, and 3.2 g of potassium tert-butoxide was added thereto with cooling to 0°C, and then stirred for 30 minutes at room temperature. The reaction liquid was again cooled to 0°C, and 30 ml of a DMF solution of 10.0 g of tert-butyl (*2S*) (4-bromomethylphenyl)(tetrahydro-*2H*-pyran-4-yl)acetate produced in Reference Example 7 was gradually dropwise added thereto. The reaction liquid was restored to room temperature and stirred for 8 hours at the temperature. The solvent was evaporated away under reduced pressure, and 300 ml of chloroform was added to the residue, and washed with 300 ml of saturated saline. The organic layer was dried with anhydrous magnesium sulfate. The solvent was evaporated away under reduced pressure, and the residue was subjected to silica gel column chromatography for elution with ethyl acetate/*n*-hexane (1/5 to 1/2, v/v) to obtain 8.1 g of a pale yellow solid of the entitled compound.

In the same manner as in Reference Example 45, the compounds shown in Table 2 below were produced.

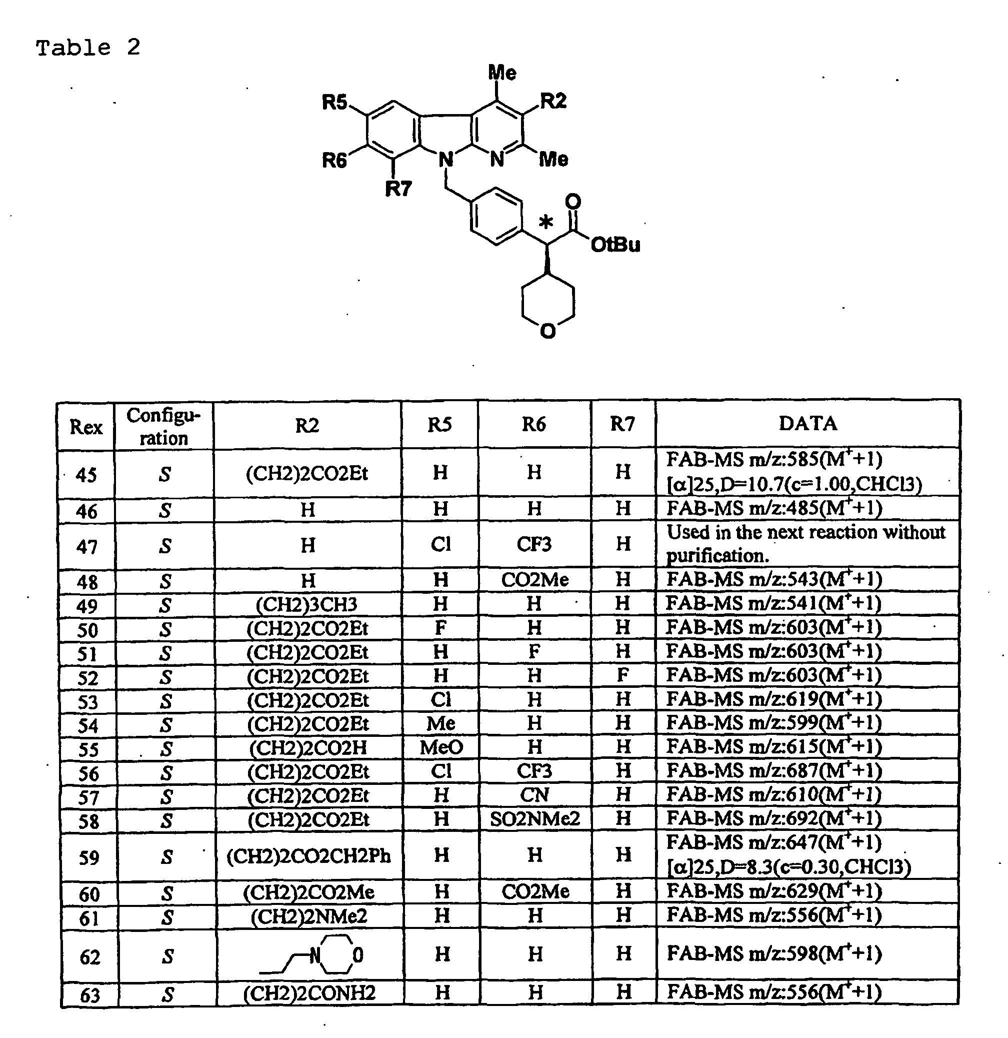

### Reference Example 64:

### (S)-(+)-(4-{[3-(3-ethoxy-3-oxopropyl)-2,4-dimethyl-9H-pyrido[2,3-b]indol-9-yl]methyl}phenyl)(tetrahydro-2H-pyran-4-yl)acetic acid

20.4 g of ethyl (+)-3-(9-{4-[(*S*)-2-(*tert*-butoxy)-2-oxo-1- (tetrahydro-*2H*-pyran-4-yl)ethyl]benzyl}-2,4-dimethyl-*9H-*pyrido[2,3-b]indol-3-yl)propanoate produced in Reference Example 45 was dissolved in 100 ml of 1,2-dichloroethane, and 100 ml of trifluoroacetic acid was added thereto and stirred for 1 hour. The reaction liquid was evaporated under reduced pressure, and 200 ml of ethyl acetate and aqueous saturated sodium hydrogencarbonate were added to the residue. The reaction liquid was neutralized with aqueous citric acid, and the solid formed was taken out through filtration to obtain 5.45 g of a colorless solid of the entitled compound. The filtrate was extracted with ethyl acetate, and washed with saturated saline, and the organic layer was dried with anhydrous magnesium sulfate. The solvent was evaporated away under reduced pressure, and the residue was recrystallized from ethyl acetate to obtain 7.47 g of a colorless crystal of the entitled compound.

In the same manner as in Reference Example 64, the compounds shown in Table 3 below were produced.

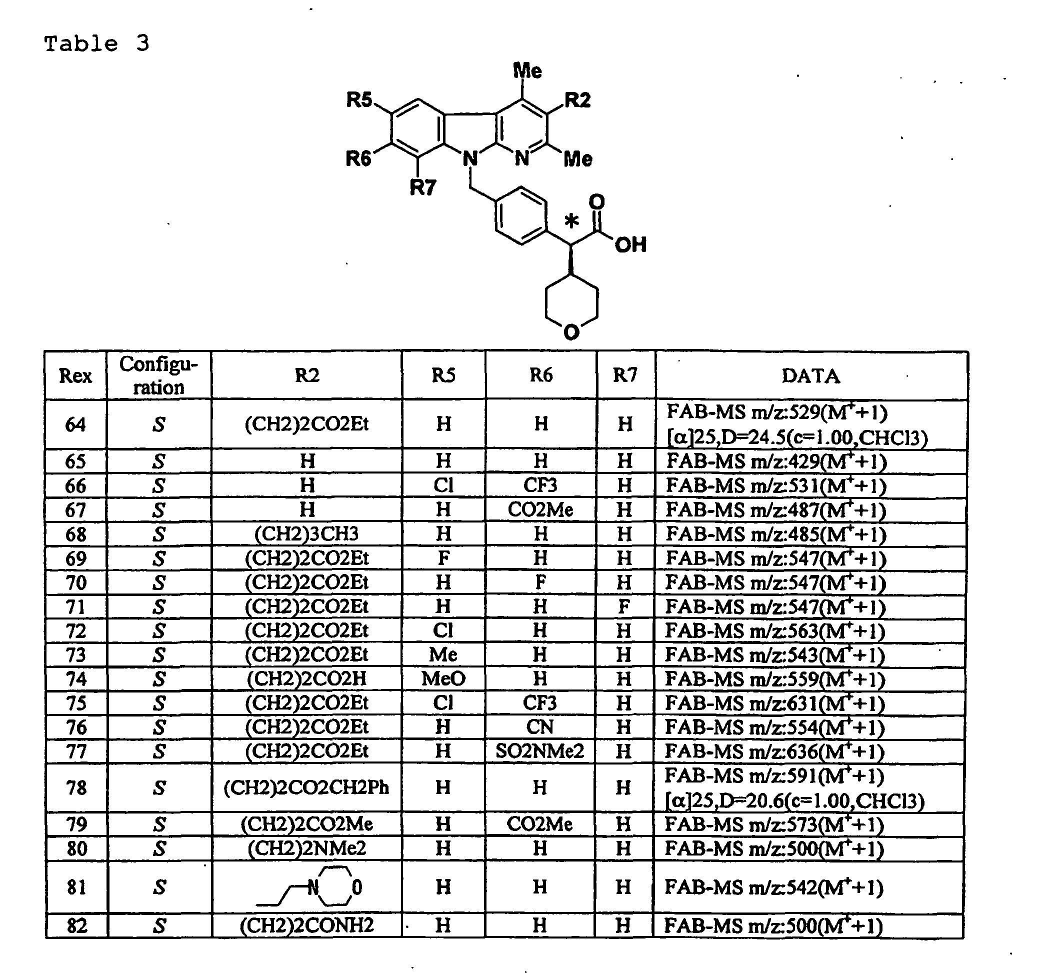

### Reference Example 83:

### Methyl (9-{4-[2-(benzyl-tert-butoxycarbonylamino)-2-oxo-1-(tetrahydro-2H-pyran-4-yl)ethyl]benzyl}-2,4-dimethyl-9H-pyrido[2,3-b]indol-3-yl)acetate

320 mg of methyl 3-(2,4-dimethyl-*9H*-pyrido[2,3-b]indol-3-yl)acetate produced in Reference Example 22 was dissolved in 3 ml of *N,N*-dimethylformamide (DMF), and 134 mg of potassium tert-butoxide was added thereto with cooling to 0°C, and then stirred for 15 minutes at room temperature. The reaction liquid was again cooled to 0°C, and 2 mg of a DMF solution of 600 mg of *tert*-butyl *N*-benzyl-[(4-bromomethylphenyl)(tetrahydro-*2H*-pyran-4-yl)]ethanoylcarbamate produced in Reference Example 10 was gradually dropwise added thereto. The reaction liquid was restored to room temperature, and then overnight stirred at the temperature. Ethyl acetate and distilled water were added to the reaction liquid, and extracted with ethyl acetate. The organic layer was washed with distilled water and saturated saline, and dried with anhydrous magnesium sulfate, and the solvent was evaporated away under reduced pressure. The residue was subjected to silica gel column chromatography for elution with a mixed solvent of *n*-hexane/ethyl acetate (9/1 to 4/1, v/v) to obtain 503 mg of a colorless oil of the entitled compound.
FAB-MS m/z:690(M⁺+1)
In the same manner as in Reference 83, the compounds shown in Table 4 below were produced.

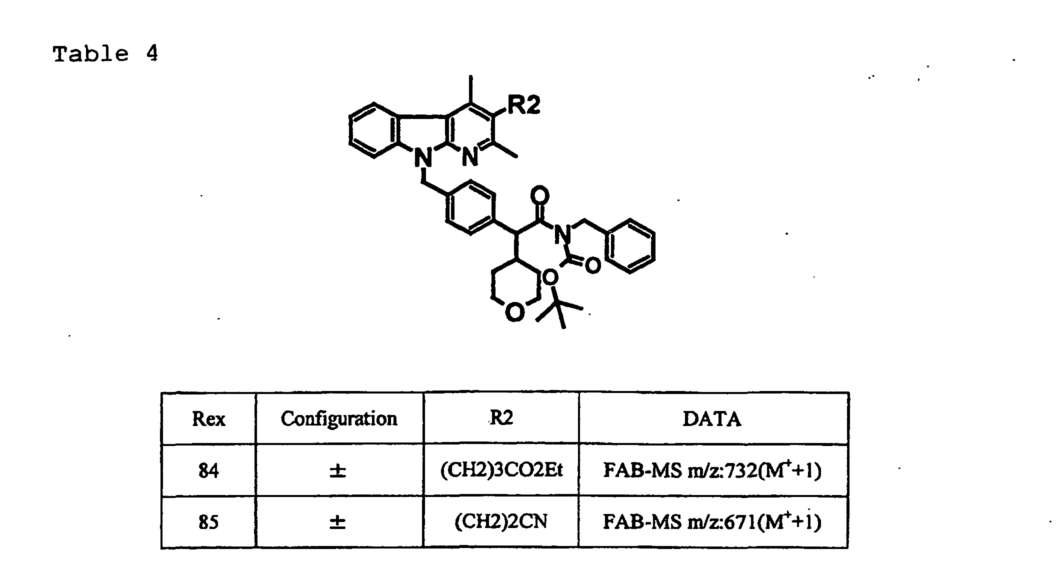

### Reference Example 86:

### (4-Methylphenyl)(tetrahydro-2H-pyran-4-yl)acetic acid

(1) To tetrahydropyran-4-ol (5 g) were added pyridine (7.6 times moles), TsC1 (2.2 times moels), and water (1.1 times moles), followed by stirring at 24°C overnight. Water (60 mL) was added to the reaction mixture, followed by extraction with toluene (60 mL). The extract was successively washed with 2N hydrochloric acid (60 mL) and water (40 mL). The organic layer was concentrated to dryness to obtain tetrahydro-*2H*-pyran-4-yl 4-methylbenzensulfonate (8.98 g).
¹H NMR (DMSO) δ: 7.83-7.81 (d,2H), 7.49-7.47 (d,2H), 4.75-4.65 (m,1H), 3.74-3.36 (m,4H), 2.42 (s,3H), 1.78-1.52 (m,4H)

(2) 1.58M n-BuLi n-hexane solution (17.7 mL) and diisopropylamine (3.1 g) were added to THF (10 mL) at 0°C or lower (LDA preparation). Then, p-tolylacetic acid (2 g) dissolved in THF (8 mL) was added, followed by stirring for 1 hour. Then, tetrahydro-*2H*-pyran-4-yl 4-methylbenzensulfonate (3.41 g) was added at 0°C or lower, followed by stirring at 24°C for 6 hours. Water (10 mL) was added to the reaction mixture and the mixture was washed twice with ethyl acetate (12 mL X 2). Then, concentrated hydrochloric acid was added to adjust pH to 4 and the mixture was extracted twice with ethyl acetate (12 mL X 2). The extract was concentrated and ethanol (7 mL) was added to dissolve the residue. Then, water (14 mL) was added to effect crystallization to obtain (4-methylphenyl)(tetrahydro-*2H*-pyran-4-yl)acetic acid (2.65 g).
¹H NMR (DMSO)δ: 12.32 (s,1H), 7.20-7.12 (q,4H) 3.86-3.14(m,5H), 2.27 (s,3H), 2.08 (m,1H), 1.69-1.00 (m,4H)

### Reference Example 87:

### 2-Imino-2,3-dihydro-1H-indole monohydrochloride

(1) According to a literature (Grob, C.A.; Helv Chim Acta 1961, **44**, 1748.), (2-nitrophenyl)acetonitrile was synthesized from 1-chloro-2-nitrobenzene via (±)-ethyl cyano(2-nitrophenyl)acetate.

Then, (2-nitrophenyl)acetonitrile (3.37 g) was subjected to catalytic hydrogenation in methanol (17 mL) under a normal pressure using 0.15 g of 10% Pd-C (54% wet). The catalyst was removed by filtration and the solvent was evaporated. Diisopropyl ether (20 mL) was added to the resulting concentrated residue and crystals were recovered by filtration to obtain (2-aminophenyl)acetonitrile (2.05 g).
FAB (pos.): m/z 133

(2) (2-Aminophenyl)acetonitrile (1.34 g) was dissolved in THF (10 mL), and concentrated hydrochloric acid was added, followed by heating under reflux for 2 hours. The solvent was evaporated, and ethanol (5 mL) and diisopropyl ether (15 mL) were added to suspend the concentrated residue. The crystals were recovered by filtration to obtain 2-imino-2,3-dihydro-*1H*-indole monohydrochloride (1.42 g).
¹H NMR(DMSO) δ: 12.46 (s,1H), 10.25 (s,1H), 10.00 (s,1H), 7.42-7.11 (m,4H), 4.18 (s,2H)

### Example 1:

### Ethyl (-)-3-(2,4-dimethyl-9-{4-[(S)-2-oxo-2-(4-pyridin-2-ylpiperazin-1-yl)-1-(tetrahydro-2H-pyran-4-yl)ethyl]-benzyl}-9H-pyrido[2,3-b]indol-3-yl)propanoate

2.0 g of (S)-(+)-(4-{[3-(3-ethoxy-3-oxopropyl)-2,4-dimethyl-*9H*-pyrido[2,3-b]indol-9-yl]methyl}phenyl)(tetrahydro-*2H*-pyran-4-yl)acetic acid produced in Reference Example 64 was dissolved in 30 ml of DMF, to which were added 560 mg of 1-hydroxybenzotriazole (HOBt), 780 mg of 1-ethyl-3-(3-dimethylaminopropyl)carbodiimide hydrochloride (WSC·HCl), 680 mg of 1-(2-pyridyl)piperazine and 580 mg of triethylamine in that order at room temperature, and stirred overnight at the temperature. The solvent was evaporated away under reduced pressure, and 100 ml of ethyl acetate was added to the residue, and washed with water, aqueous saturated sodium hydrogencarbonate and saturated saline. The organic layer was dried with anhydrous magnesium sulfate, and the solvent was evaporated away under reduced pressure. The residue was subjected to silica gel column chromatography for elution with a mixed solvent of chloroform/methanol (100/1, v/v) to obtain 2.42 g of a pale yellow foam substance of the entitled compound. This was recrystallized from ethanol to obtain 1.85 g of a colorless crystal of the entitled compound.

### Example 2:

### Benzyl (+)-3-(2,4-dimethyl-9-{4-[(S)-2-oxo-2-(4-propylpiperazin-1-yl)-1-(tetrahydro-2H-pyran-4-yl)ethyl]benzyl}-9H-pyrido[2,3-b]indol-3-y)propanoate

1.8 g of (*S*)-(+)-(4-{[3-(3-benzyloxy-3-oxopropyl)-2,4-dimethyl-*9H*-pyrido[2,3-b]indol-9-yl]methyl}phenyl)(tetrahydro-*2H*-pyran-4-yl)acetic acid produced in Reference Example 78 was dissolved in 40 ml of DMF, to which were added 460 mg of HOBt, 650 mg of WSC·HCl, 980 mg of 1-(*n*-propyl)piperazine dihydrobromide and 930 mg of triethylamine in that order at room temperature, and stirred for 10 hours at the temperature. The solvent was evaporated away under reduced pressure, and 150 ml of ethyl acetate was added to the residue, and washed with water, aqueous saturated sodium hydrogencarbonate and saturated saline. The organic layer was dried with anhydrous magnesium sulfate, and the solvent was evaporated away under reduced pressure. The residue was subjected to silica gel column chromatography for elution with a mixed solvent of chloroform/methanol (20/1, v/v) to obtain 1.85 g of a colorless foam substance of the entitled compound. 300 mg of this was recrystallized from ethanol to obtain 148 mg of a colorless crystal of the entitled compound.

In the same manner as in Example 1, the compounds shown in Table 5 were produced.

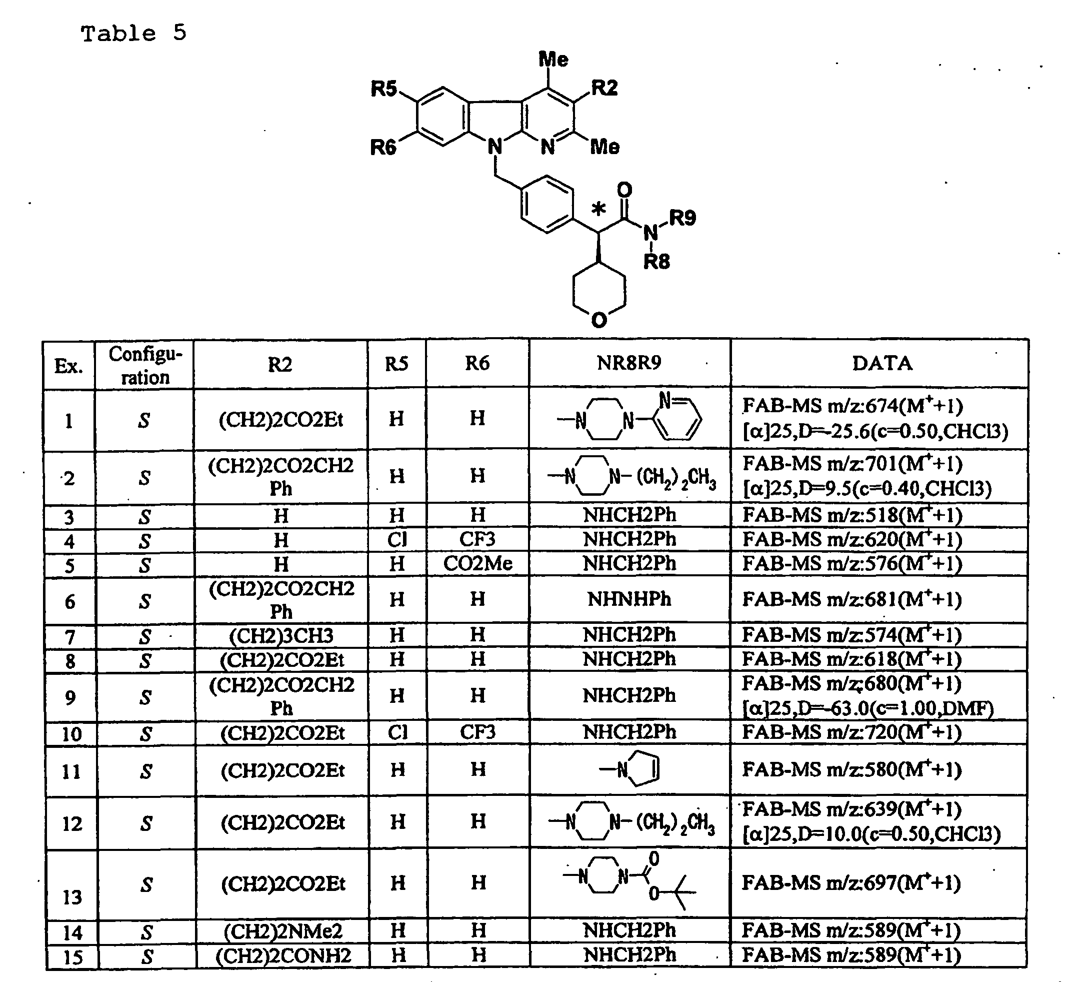

### Example 16:

### Ethyl (+)-3-(2,4-dimethyl-9-{4-[(S)-2-oxo-2-(piperazin-1-yl)-1-(tetrahydro-2H-pyran-4-yl)ethyl]benzyl}-9H-pyrido[2,3-b]indol-3-yl)propanoate dihydrochloride

2.11 g of 4-(*tert*-butoxycarbonyl)piperazine was dissolved in 40 ml of DMF, to which were added 4.0 g of (*S*)-(+)-(4-{[3-(3-ethoxy-3-oxopropyl)-2,4-dimethyl-*9H*-pyrido[2,3-b]indol-9-yl]methyl}phenyl)(tetrahydro-*2H*-pyran-4-yl)acetic acid produced in Reference Example 64, 1.74 g of 1-ethyl-3-(3-dimethylaminopropyl)carbodiimide hydrochloride (WSC·HCl) and 1.23 g of 1-hydroxybenzotriazole (HOBt) in that order at room temperature, and stirred overnight at the temperature. Ethyl acetate and aqueous sodium hydrogencarbonate were added to the reaction liquid, and extracted with ethyl acetate. The organic layer was washed with water, 1.0 M hydrochloric acid and saturated saline, and then dried with anhydrous magnesium sulfate. The solvent was evaporated away under reduced pressure to obtain a colorless foam substance. The resulting foam substance was dissolved in 20 ml of ethyl acetate, to which was added 20 ml of an ethyl acetate solution of 4 M hydrogen chloride at room temperature, and stirred for 1 hour. The reaction liquid was evaporated under reduced pressure, and washed with ethyl acetate to obtain 5.07 g of a colorless solid of the entitled compound.
FAB-MS m/z:597 (M⁺+1)

### Example 17:

### (-)-3-(2,4-Dimethyl-9-{4-[(S)-2-oxo-2-(4-pyridin-2-ylpiperazin-1-yl)-1-(tetrahydro-2H-pyran-4-yl)ethyl]benzyl}-9H-pyrido[2,3-b]indol-3-yl)propanoic acid

100 ml of methanol and 15 ml of water were added to 1.84 g of ethyl (-)-3-(2,4-dimethyl-9-{4-[(*S*)-2-oxo-2-(4-pyridin-2-ylpiperazin-1-yl)-1-(tetrahydro-*2H*-pyran-4-yl)ethyl]benzyl}-*9H*-pyrido[2,3-b]indol-3-yl)propanoate produced in Example 1, to which was added 830 mg of anhydrous potassium carbonate and stirred at 70°C for 3 hours. After this was cooled with ice, 5.95 ml of 1 M hydrochloric acid was added thereto, and the solvent was evaporated away under reduced pressure. Chloroform was added to the residue, and washed with saturated saline. The organic layer was dried with anhydrous magnesium sulfate, and the solvent was evaporated away under reduced pressure. The residue was subjected to silica gel column chromatography for elution with a mixed solvent of chloroform/methanol (20/1, v/v) to obtain 1.8 g of a colorless foam substance of the entitled compound. This was crystallized from ethanol and then from acetone to obtain 710 mg of a colorless crystal of the entitled compound.

### Example 18:

### (+)-3-(2,4-Dimethyl-9-{4-[(S)-2-oxo-2-(4-propylpiperazin-1-yl)-1-(tetrahydro-2H-pyran-4-yl)ethyl]benzyl}9H-pyrido[2,3-b]indol-3-yl)propanoic acid

1.96 g of benzyl (+)-3-(2,4-dimethyl-9-{4-[(*S*)-2-oxo-2-(4-propylpiperazin-1-yl)-1-(tetrahydro-*2H*-pyran-4-yl)ethyl]benzyl}*9H*-pyrido[2,3-b]indol-3-yl)propanoate produced in Example 2 was dissolved in 50 ml of tetrahydrofuran, and 300 mg of 10 % palladium-carbon was added thereto in argon. Hydrogen gas was introduced into the reaction system, and stirred overnight at room temperature. The reaction system was purged with argon gas, the insoluble matter was taken out through filtration through Celite, and the filtrate was evaporated under reduced pressure. The residue was subjected to silica gel column chromatography for elution with a mixed solvent of chloroform/methanol (20/1, v/v) to obtain 1.75 g of a colorless foam substance of the entitled compound. This was crystallized from ethyl acetate and then from ethanol to obtain 765 mg of a colorless crystal of the entitled compound.

### Example 19:

### (-)-3-(9-{4-[(S)-(benzylcarbamoyl) (tetrahydro-2H-pyran-4-yl)methyl]benzyl}-2,4-dimethyl-9H-pyrido[2,3-b]indol-3-yl)propanoic acid

3.2 g of benzyl (-)-3-(9-{4-[(*S*)-(benzylcarbamoyl) (tetrahydro-*2H*-pyran-4-yl)methyl]benzyl}-2,4-dimethyl-*9H*-pyrido[2,3-b]indol-3-yl)propanoate produced in Example 9 was dissolved in 120 ml of tetrahydrofuran, and 500 mg of 10 % palladium-carbon was added thereto in argon. Hydrogen gas was introduced into the reaction system, and stirred for 2 days at room temperature. The reaction system was purged with argon gas, the insoluble matter was taken out through filtration through Celite, and the filtrate was evaporated under reduced pressure. The residue was subjected to silica gel column chromatography for elution with a mixed solvent of chloroform/methanol (20/1, v/v) to obtain 3.31 g of a colorless foam substance of the entitled compound. This was crystallized from ethanol to obtain 2.58 g of a colorless crystal of the entitled compound.

### Example 20:

### (+)-3-(9-{4-[(S)-2-(4-cyclopropylmethylpiperazin-1-yl)-2-oxo-1-(tetrahydro-2H-pyran-4-yl)ethyl]benzyl-2,4-dimethyl-9H-pyrido[2,3-b]indol-3-yl}propanoic acid

94 mg of cyclopropylaldehyde was dissolved in 6 ml of 1,2-dichloroethane, and 300 mg of ethyl (+)-3-(2,4-dimethyl-(9-{4-[(*S*)-2-oxo-2-(piperazin-1-yl)-1-(tetrahydro-*2H*-pyran-4-yl)ethyl]benzyl}-9H-pyrido[2,3-b]indol-3-yl)propanoate dihydrochloride produced in Example 16 was added thereto and stirred for 1 hour. 285 mg of sodium triacetoxyborohydride was added thereto at the temperature and stirred for 1 hour. Chloroform and aqueous saturated sodium hydrogencarbonate were added to the reaction liquid, and then extracted with chloroform. The organic layer was washed with saturated saline, and dried with anhydrous magnesium sulfate, and the solvent was evaporated away under reduced pressure. The residue was subjected to silica gel column chromatography for elution with a mixed solvent of chloroform/methanol (99/1, v/v) to obtain 261 mg of a colorless foam substance. The resulting foam substance was dissolved in 12 ml of methanol and 6 ml of distilled water, to which was added 196 mg of potassium carbonate at room temperature. The reaction liquid was stirred overnight at 80°C, and then evaporated under reduced pressure. 2.8 ml of 1 M hydrochloric acid was added to the residue, and extracted with chloroform. The organic layer was dried with anhydrous magnesium sulfate, and the solvent was evaporated away under reduced pressure. The residue was recrystallized from a mixed solvent of ethyl acetate and n-hexane to obtain 194 mg of a colorless crystal of the entitled compound.

In the same manner as in Examples 17, 18, 19 or 20, the compounds shown in Table 6 below were produced.

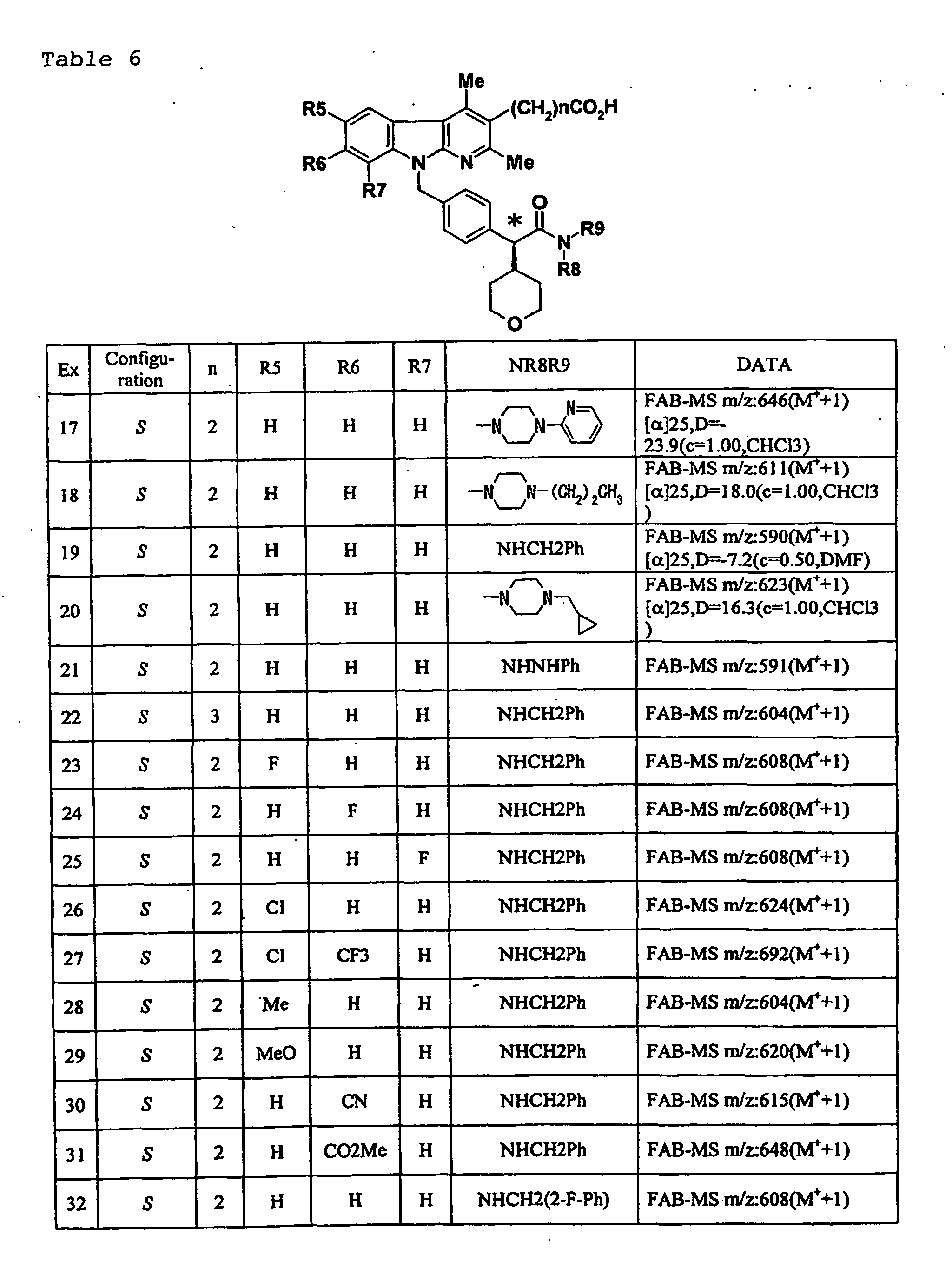

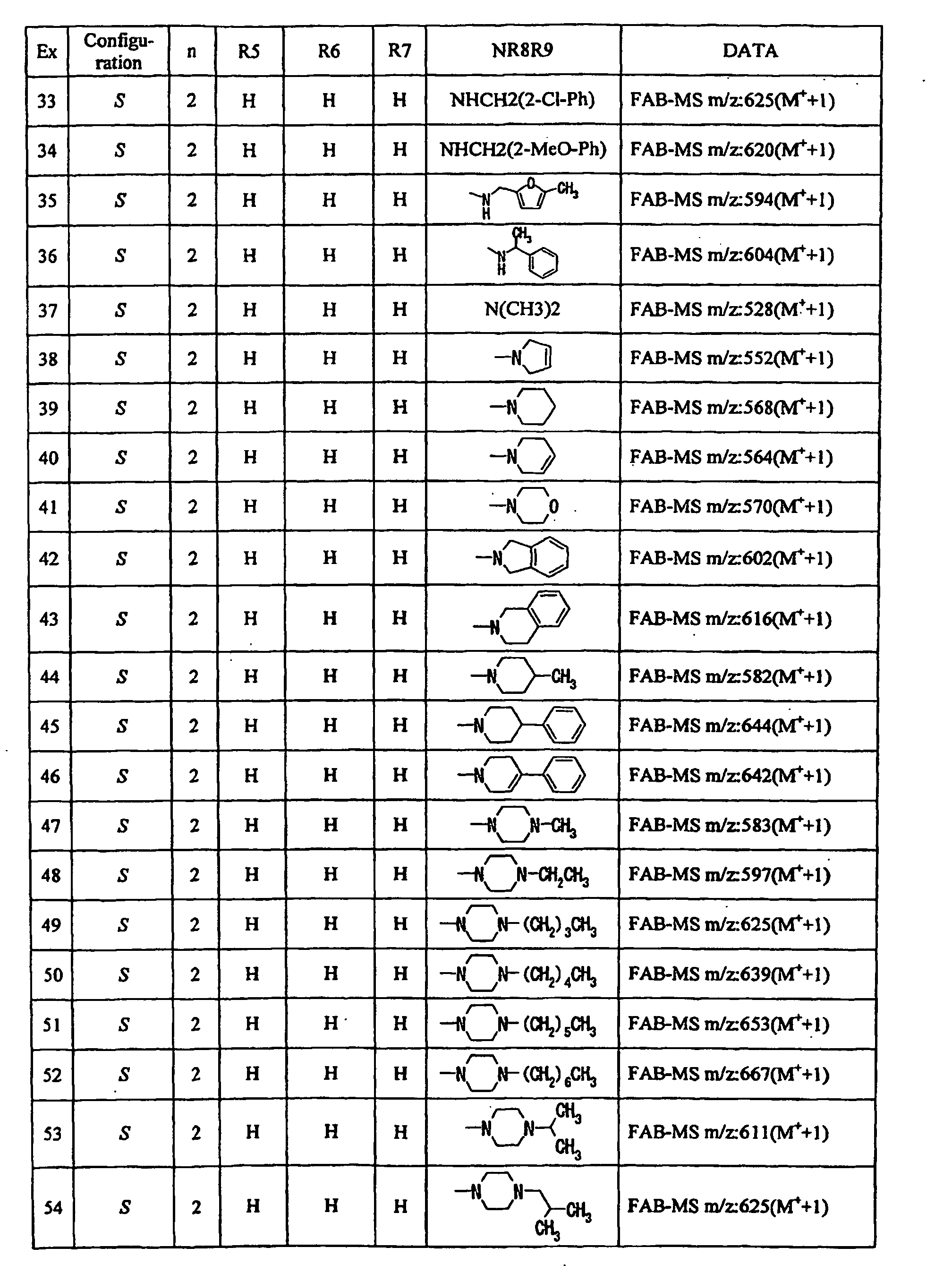

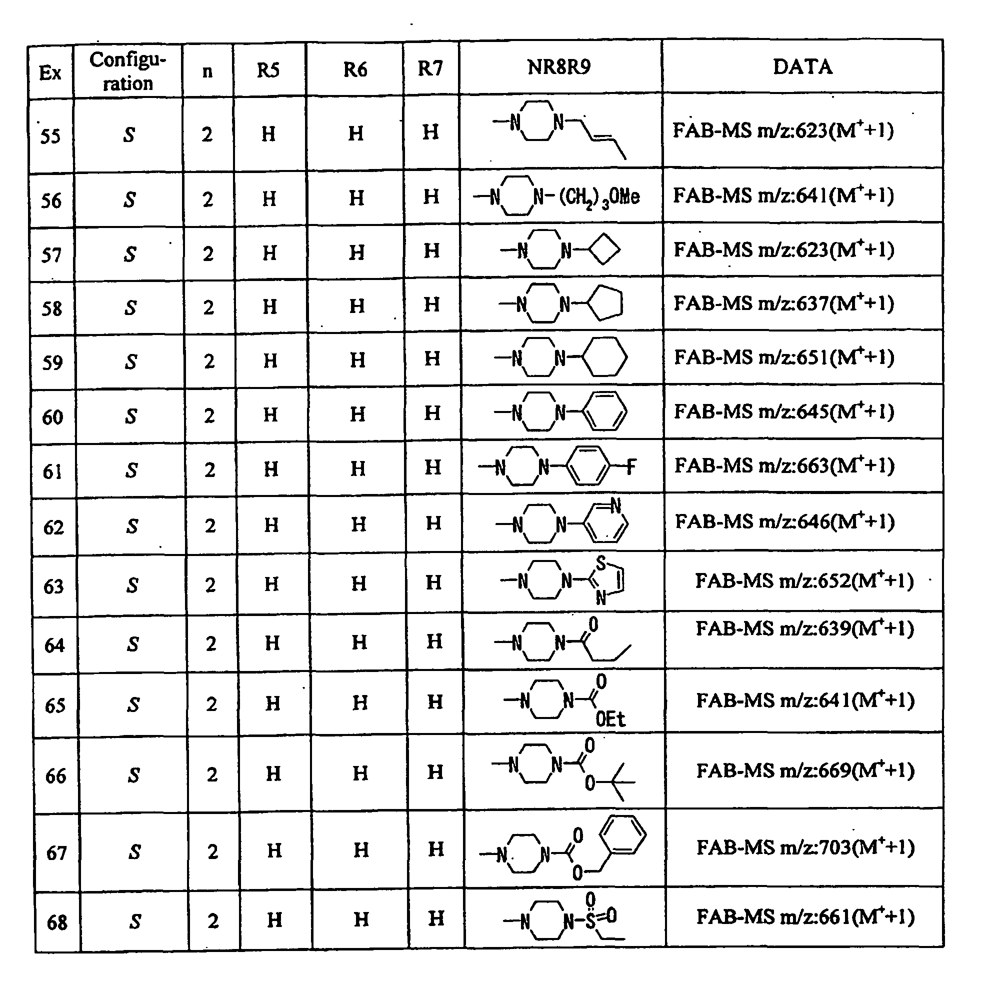

### Example 69:

### (S)-(-)-N-benzyl-2-(4-{ [2,4-dimethyl-3-(3-hydroxypropyl)-9H-pyrido[2,3-b]indol-9-yl]methyl}phenyl)-2-(tetrahydro-2H-pyran-4-yl)acetamide

295 mg of (-)-3-(9-{4-[(*S*)-(benzylcarbamoyl)(tetrahydro-*2H*-pyran-4-yl)methyl]benzyl}-2,4-dimethyl-*9H*-pyrido[2,3-b]indol-3-yl)propanoic acid produced in Example 19 was dissolved in 10 ml of tetrahydrofuran, to which was added 3.0 ml of a tetrahydrofuran solution of 1.0 M borane-tetrahydrofuran complex at 0°C in argon. This was stirred at room temperature for 3.5 hours, and 15 ml of water and 6.0 g of potassium carbonate were added thereto and stirred at room temperature for 1 hour. The reaction liquid was extracted with ethyl acetate, and the organic layer was washed with saturated saline, and dried with anhydrous magnesium sulfate. The solvent was evaporated away under reduced pressure, and the residue was subjected to silica gel column chromatography for elution with chloroform/methanol (50/1, v/v) to obtain 296 mg of a colorless solid of the entitled compound. This was crystallized from ethanol to obtain 217 mg of a crystal of the entitled compound.
FAB-MS m/z:576(M⁺+1), [α]25.0,D=-72.0(c=1.00, DMF)

### Example 70:

### (±)-N-benzyl-2-(4-{[3-(2-cyanoethyl)-2,4-dimethyl-9H-pyrido[2,3-b]indol-9-yl]methyl}phenyl)-2-(tetrahydro-2H-pyran-4-yl)acetamide

1.1 g of (±)-N-benzyl-[2-(4-{[3-(2-cyanoethyl)-2,4-dimethyl-*9H*-pyrido[2,3-b]indol-9-yl]methyl}phenyl)-2-(tetrahydro-*2H*-pyran-4-yl)ethanoyl]carbamate produced in Reference Example 85 was dissolved in 10 ml of 1,2-dichloroethane, to which was added 5 ml of trifluoroacetic acid at room temperature and stirred for 3 hours. The reaction liquid was evaporated under reduced pressure, and ethyl acetate and aqueous sodium hydrogencarbonate were added to the residue and extracted with ethyl acetate. The organic layer was washed with distilled water and saturated saline, and then dried with anhydrous magnesium sulfate. The solvent was evaporated away under reduced pressure. The residue was recrystallized from ethyl acetate to obtain 728 mg of a colorless crystal of the entitled compound.
FAB-MS m/z:571(M⁺+1)

### Example 71:

### Sodium (±)-(9-{4-[benzylcarbamoyl-(tetrahydro-2H-pyran-4-yl)methyl]benzyl}-2,4-dimethyl-9H-pyrido[2,3-b]indol-3-yl)acetate

500 mg of methyl (±)-(9-{4-[2-(benzyl-*tert*-butoxycarbonylamino)-2-oxo-1-(tetrahydro-*2H*-pyran-4-yl)ethyl]benzyl}-2,4-dimethyl-*9H*-pyrido[2,3-b]indol-3-yl)acetate produced in Reference Example 83 was dissolved in 5 ml of ethyl acetate, to which was added 5 ml of an ethyl acetate solution of 4 M hydrogen chloride at room temperature, and stirred for 2 hours. The reaction liquid was evaporated under reduced pressure, and the residue was dissolved in 5 ml of methanol. 3.6 ml of aqueous 1 M sodium hydroxide was added thereto at room temperature, and stirred overnight at room temperature. The reaction liquid was evaporated under reduced pressure, and ethyl acetate and aqueous citric acid were added to the residue. The colorless solid formed was taken out through filtration. The filtrate was extracted with ethyl acetate, and the organic layer was washed with distilled water and aqueous saline, and dried with anhydrous magnesium sulfate. The solvent was evaporated away under reduced pressure. The residue was mixed with the colorless solid collected previously, and washed with ethyl acetate to obtain 240 mg of a colorless solid. 240 mg of the resulting colorless solid was dissolved in 5 ml of ethanol, to which was added 0.41 ml of an aqueous 1 M sodium hydroxide at room temperature. The solid formed was taken out through filtration and dried to obtain 185 mg of a colorless solid of the entitled compound.
FAB-MS m/z:571(M⁺+1)

In the same manner as in Example 71, the compound of the following Example 72 was produced.

### Example 72:

### Sodium (±)-(9-{4-[benzylcarbamoyl-(tetrahydro-2H-pyran-4-yl)methyl]benzyl}-2,4-dimethyl-9H-pyrido[2,3-b]indol-3-yl)butanoate

FAB-MS m/z:626(M⁺+1)

### Example 73:

### (±)-N-benzyl-2-[4-({2,4-dimethyl-3-[2-(1H-tetrazol-5-yl)ethyl]-9H-pyrido[2,3-b]indol-9-yl}methyl)phenyl]-2-(tetrahydro-2H-pyran-4-yl)acetamide

1.45 g of tributyltin azide was dissolved in 5 ml of toluene, and 500 mg of (±)-N-benzyl-2-(4-{ [3- (2-cyanoethyl)-2,4-dimethyl-*9H*-pyrido[2,3-b]indol-9-yl]methyl}phenyl)-2-(tetrahydro-*2H*-pyran-4-yl)acetamide produced in Example 70 was added thereto at room temperature, and stirred overnight under heat for reflux. 10 ml of 1 M hydrochloric acid and 20 ml of ethyl acetate were added to the reaction liquid. Aqueous saturated sodium hydrogencarbonate and aqueous citric acid were added thereto, and the solid formed was taken out through filtration. The filtrate was extracted with ethyl acetate, the organic layer was washed with saturated saline and dried with anhydrous magnesium sulfate, and the solvent was evaporated away under reduced pressure. The residue was mixed with the solid collected previously, and washed with hot hexane. The resulting colorless solid was recrystallized from ethanol to obtain 272 mg of a colorless crystal of the entitled compound.
FAB-MS m/z:598(M⁺+1)

The compounds shown in Tables 7 and 8 below were produced in the same manner as in the above-mentioned Reference Examples and Examples, optionally further combined with any other operation generally employed by those skilled in the art.

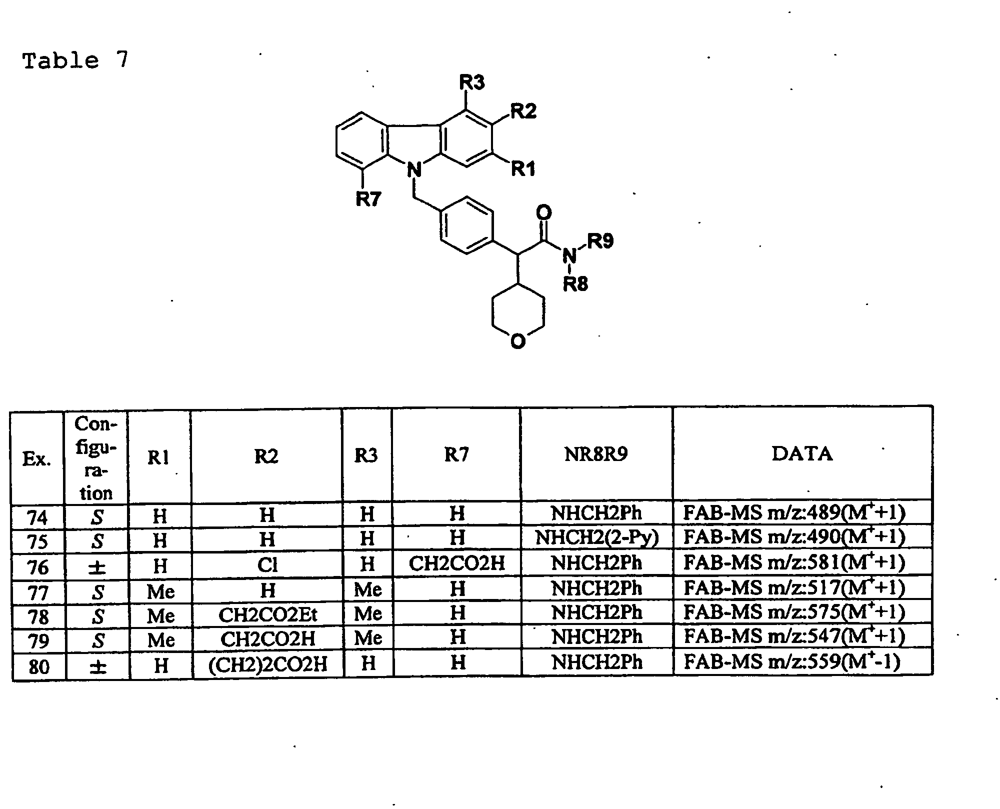

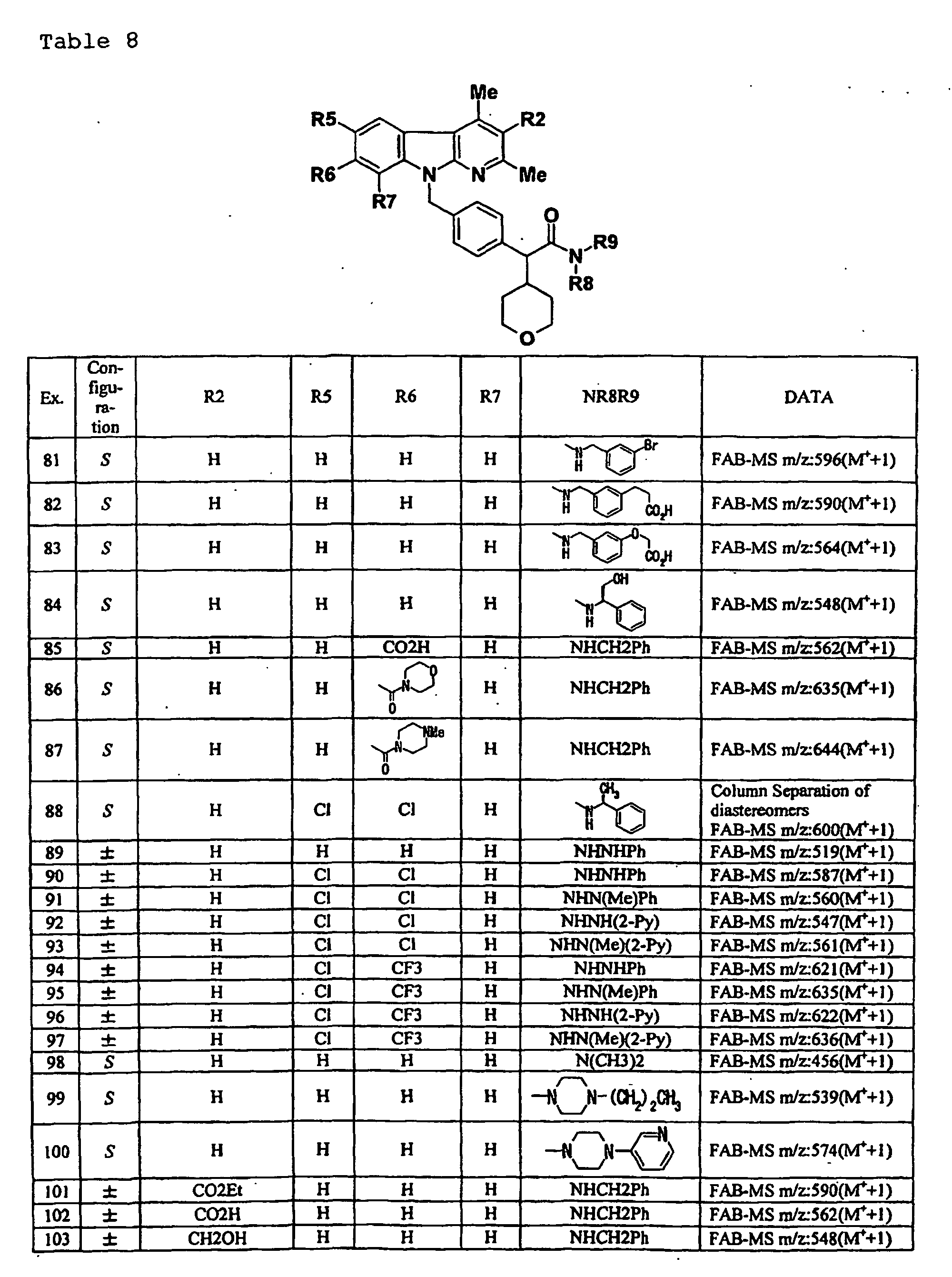

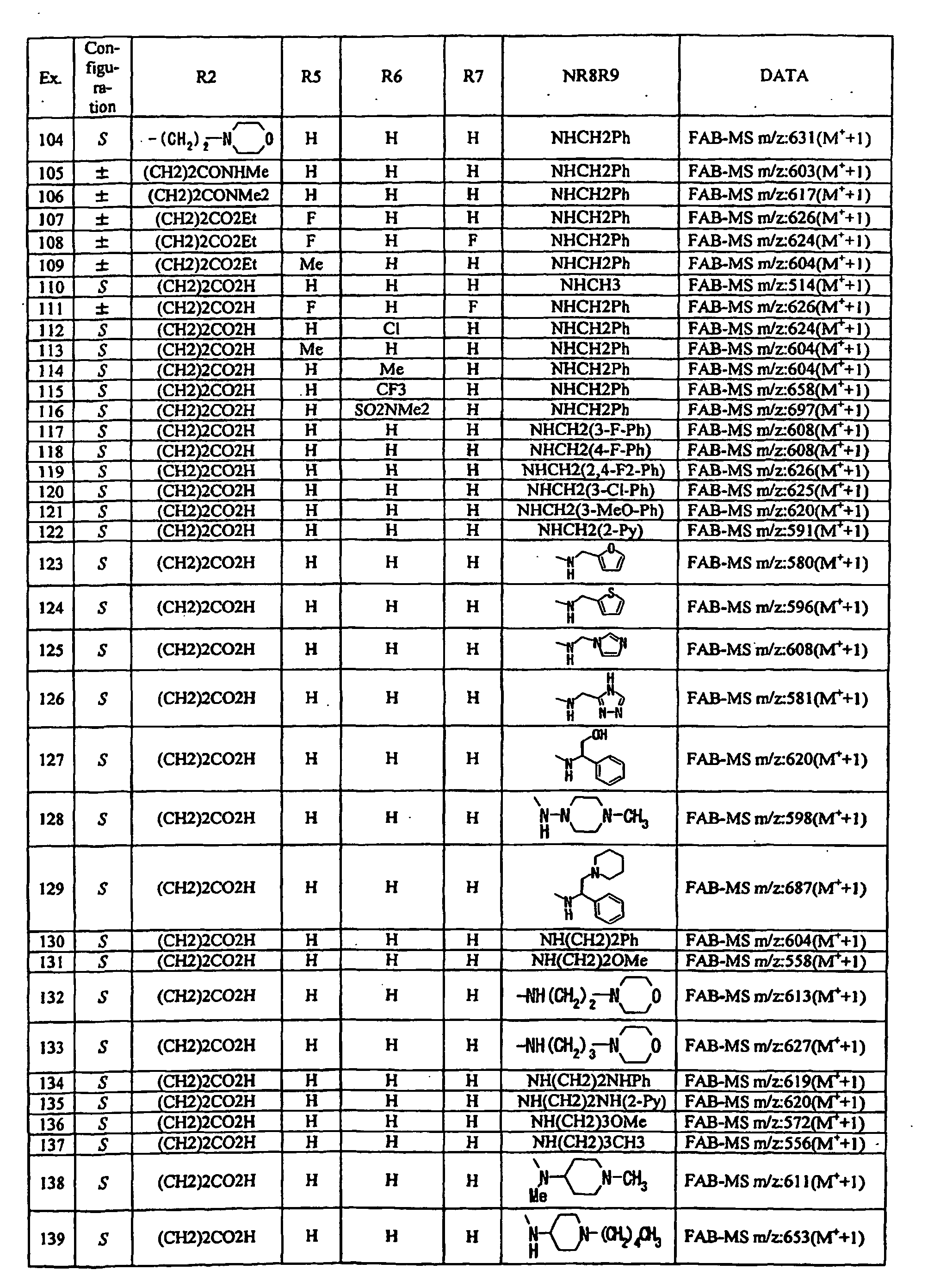

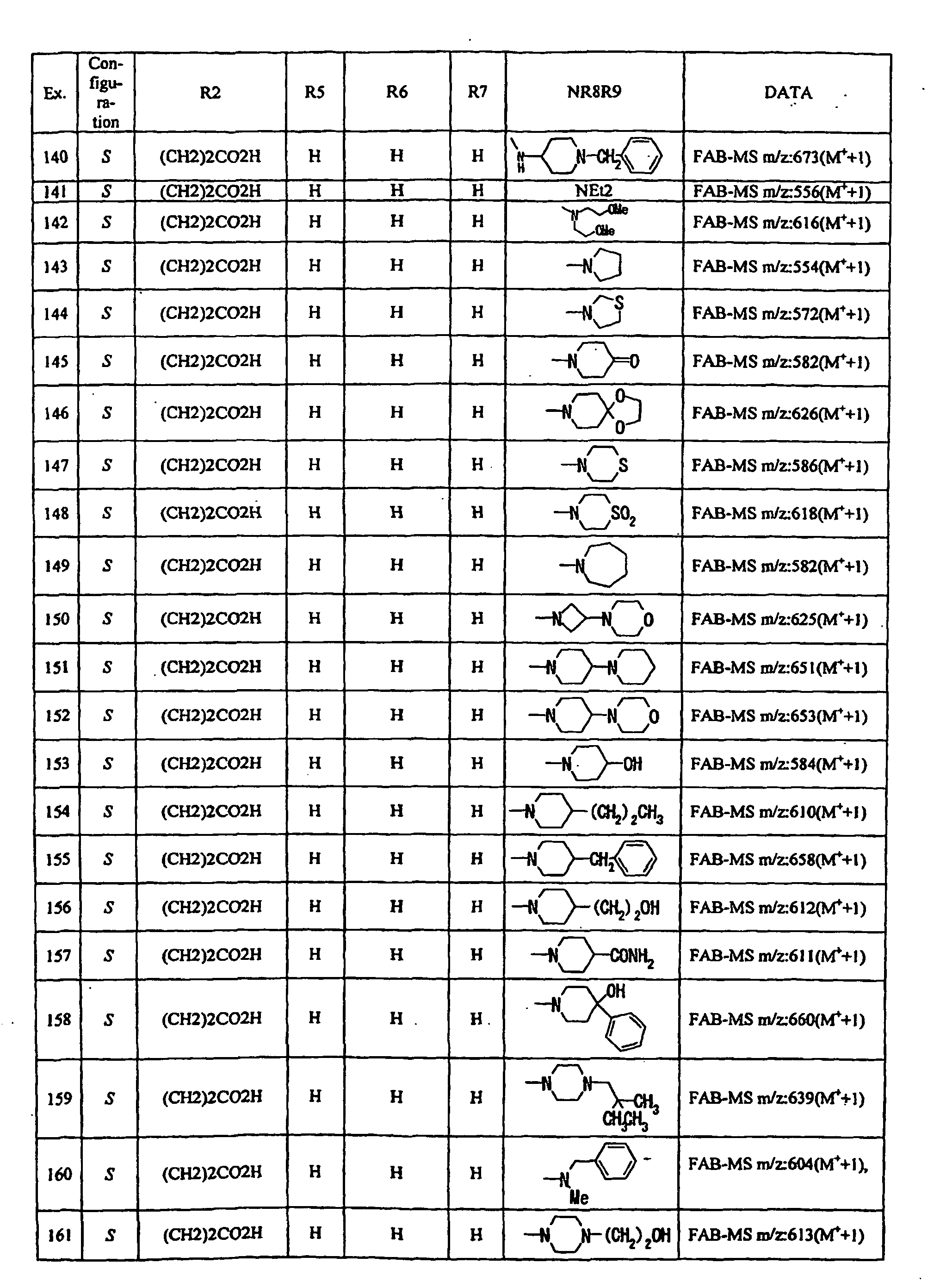

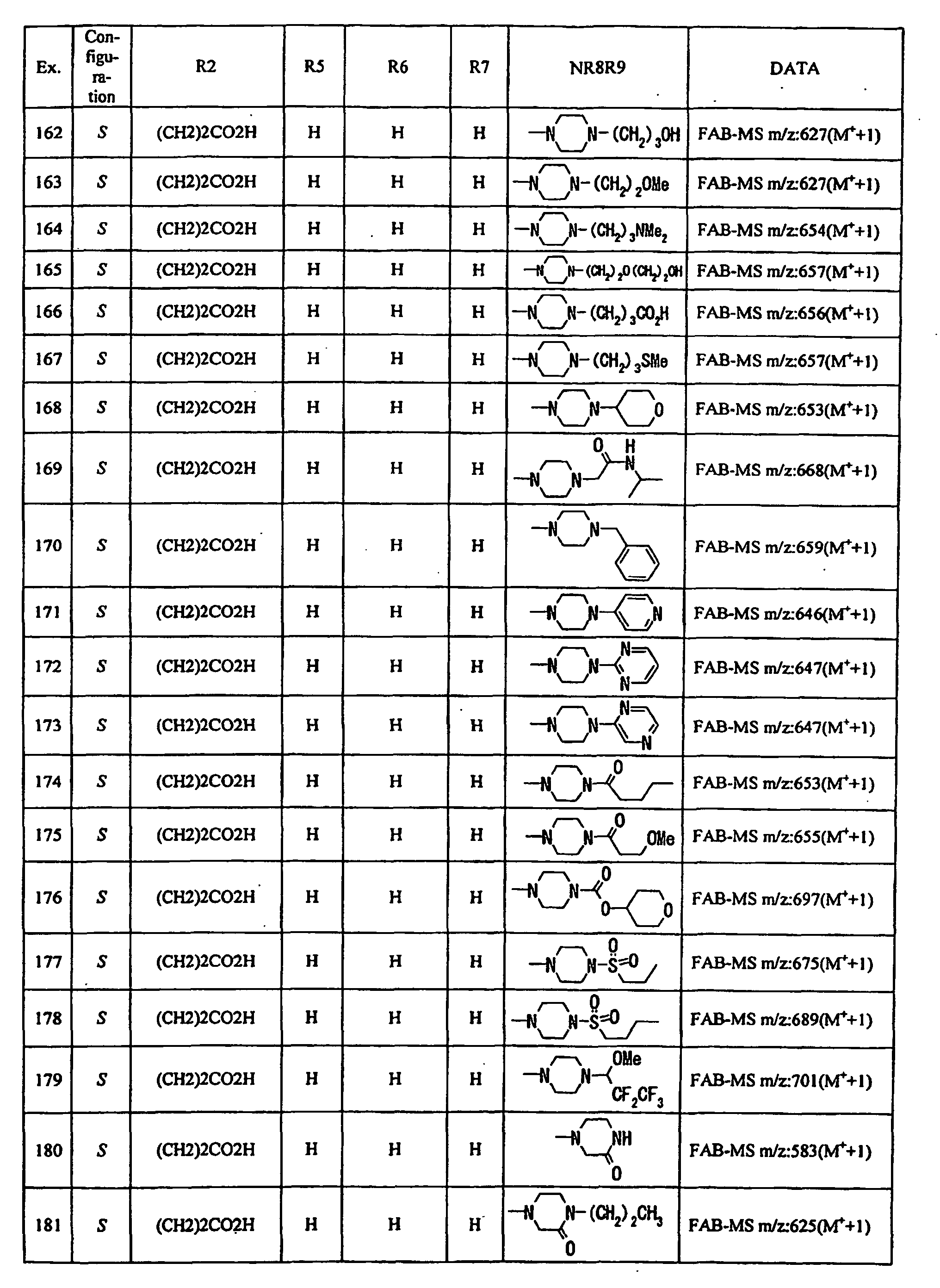

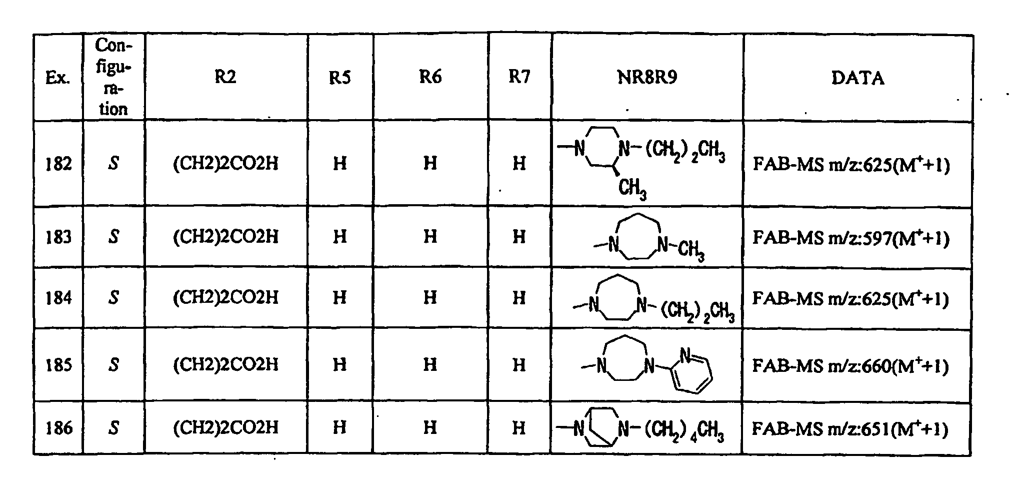

Of the compounds of the Examples mentioned above, those not specifically indicated were produced as free bases or free acids. The compounds of the following Examples were produced as hydrochlorides or fumarate. Hydrochlorides:

Examples 3, 4, 7, 8, 10, 22, 27, 32, 35, 75, 84, 88, 89, 90, 91, 92, 93, 94, 95, 96, 97, 101, 102, 103, 107, 108, 109, 110, 111, 113, 119, 120, 123, 124, 132, 133, 134, 136, 137, 138, 185.

### Fumarate:

### Example 99.

The following compounds are also produced in the same manner as in these Examples or in an ordinary manner.

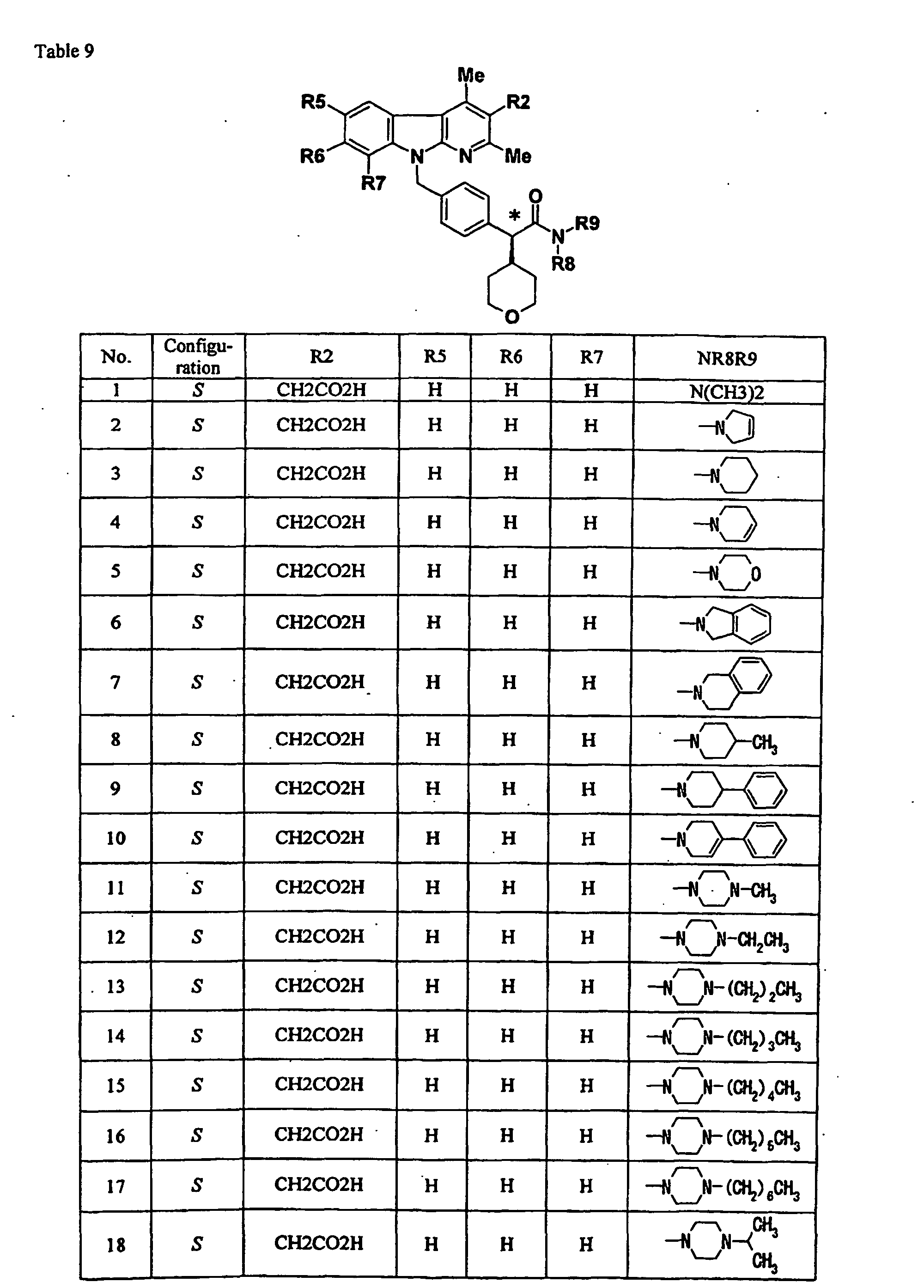

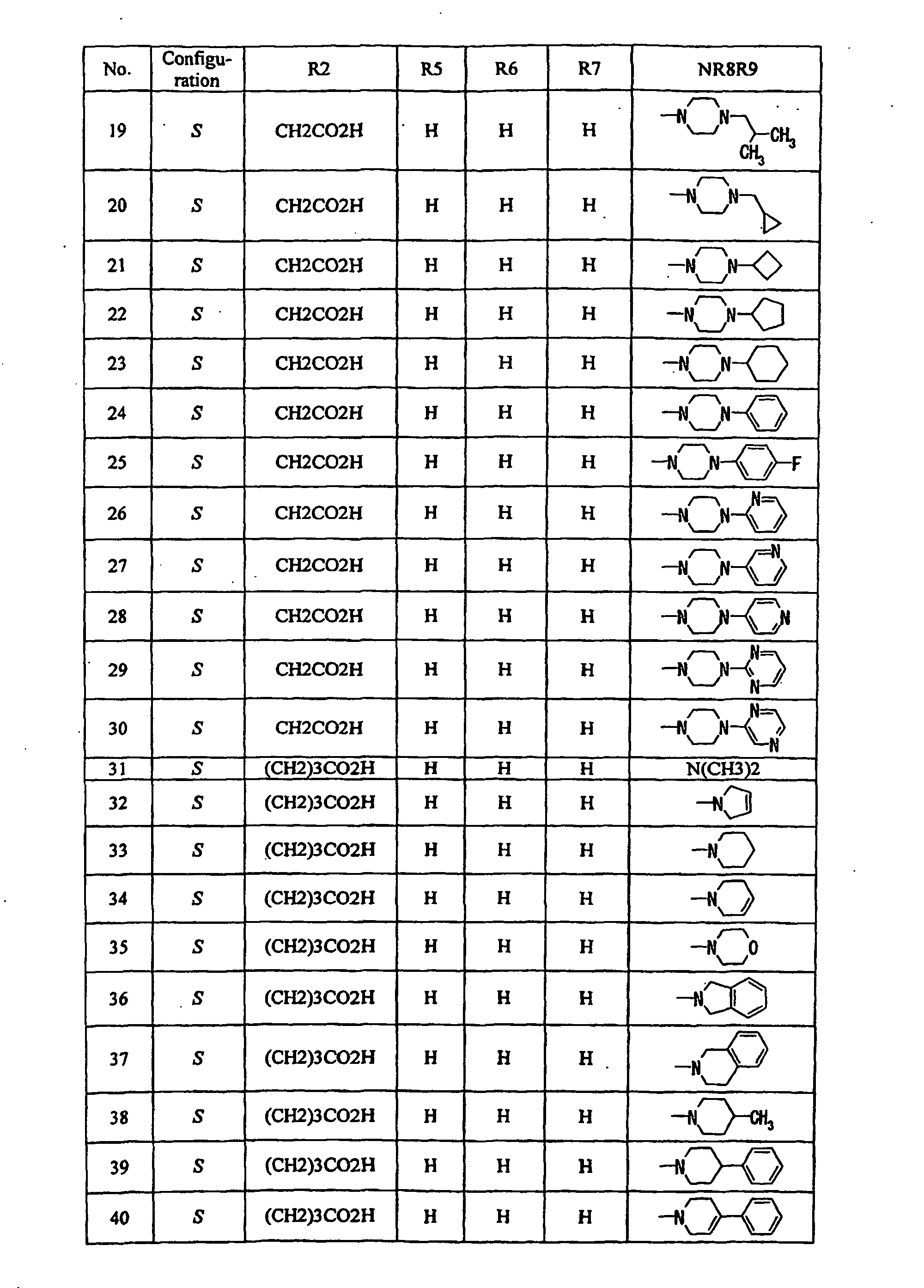

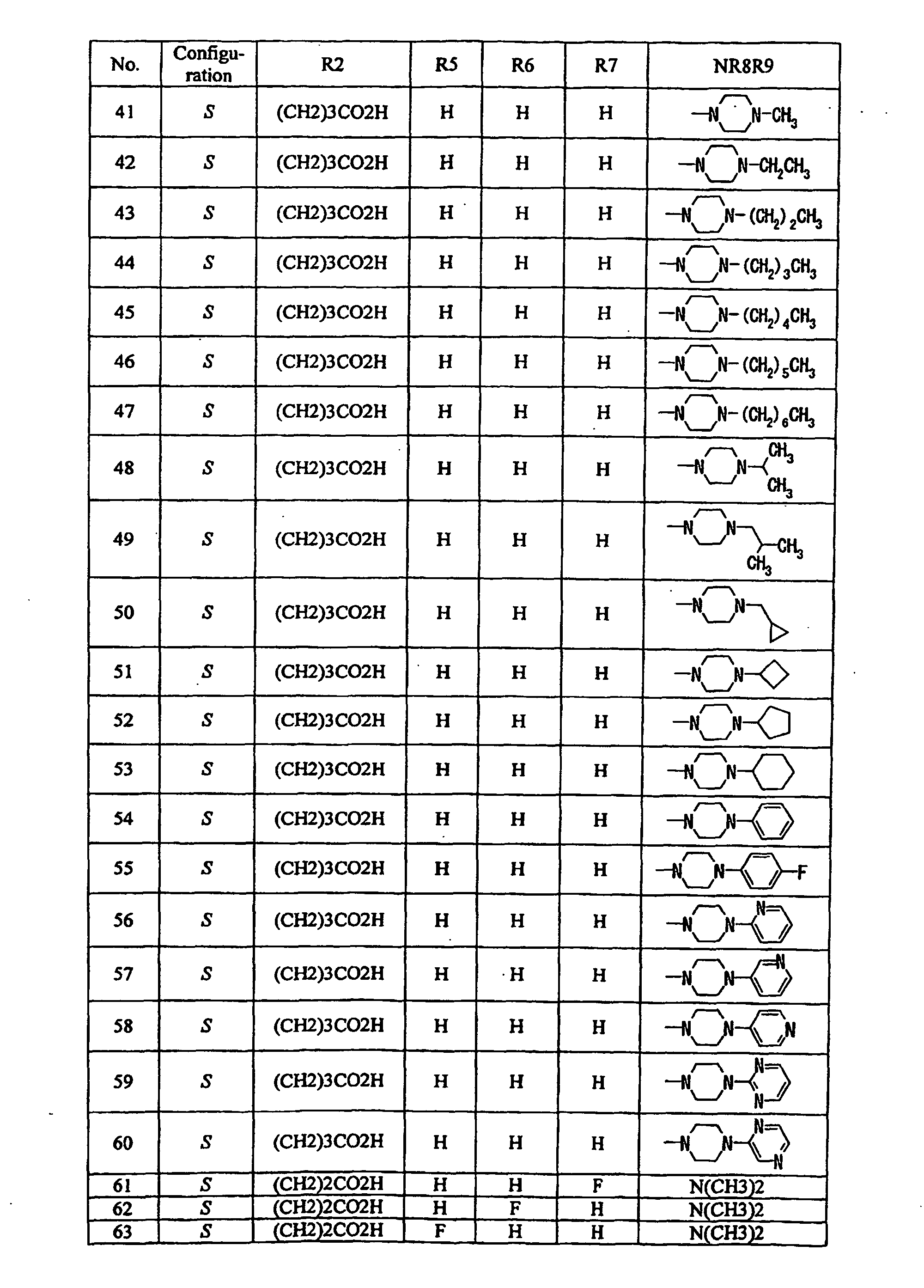

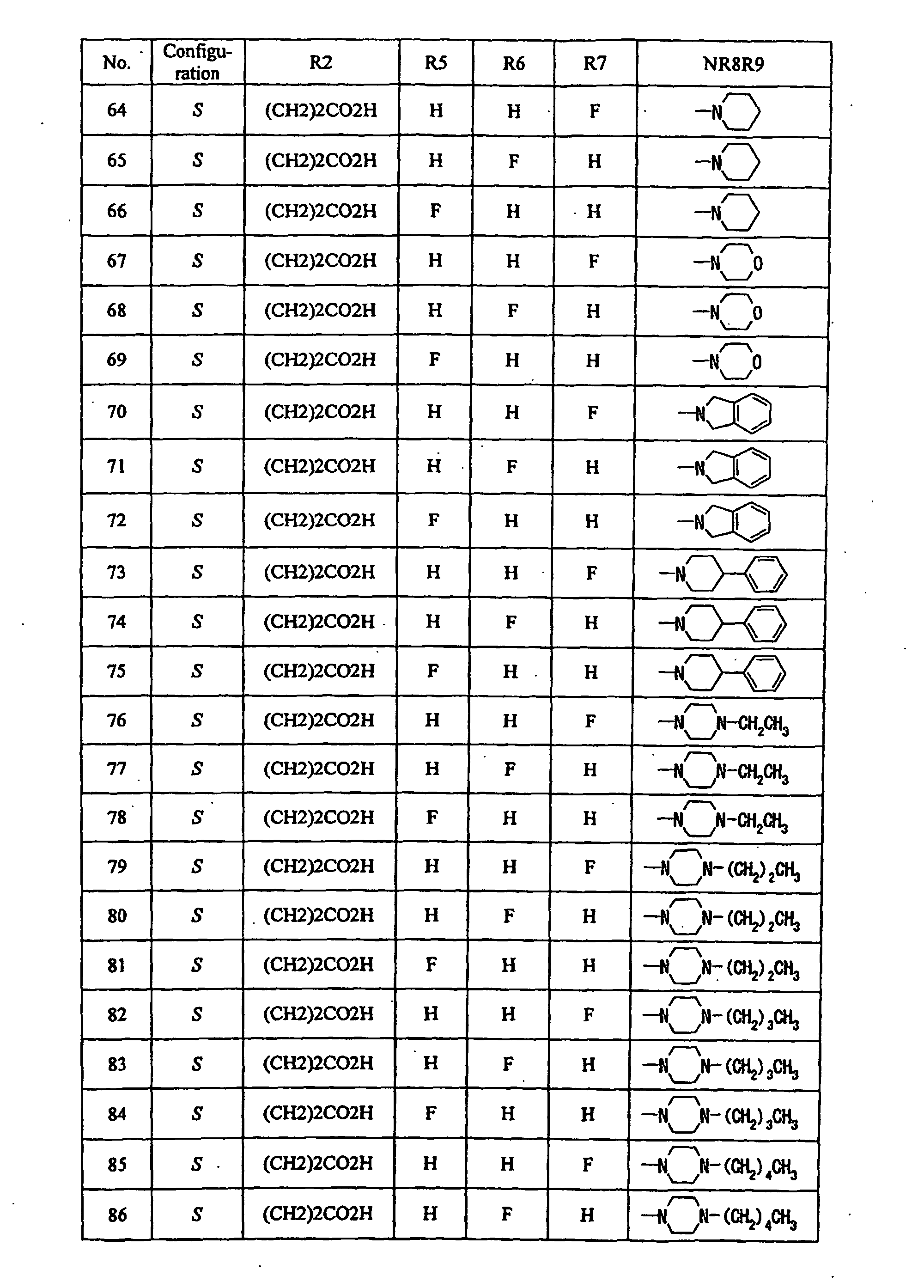

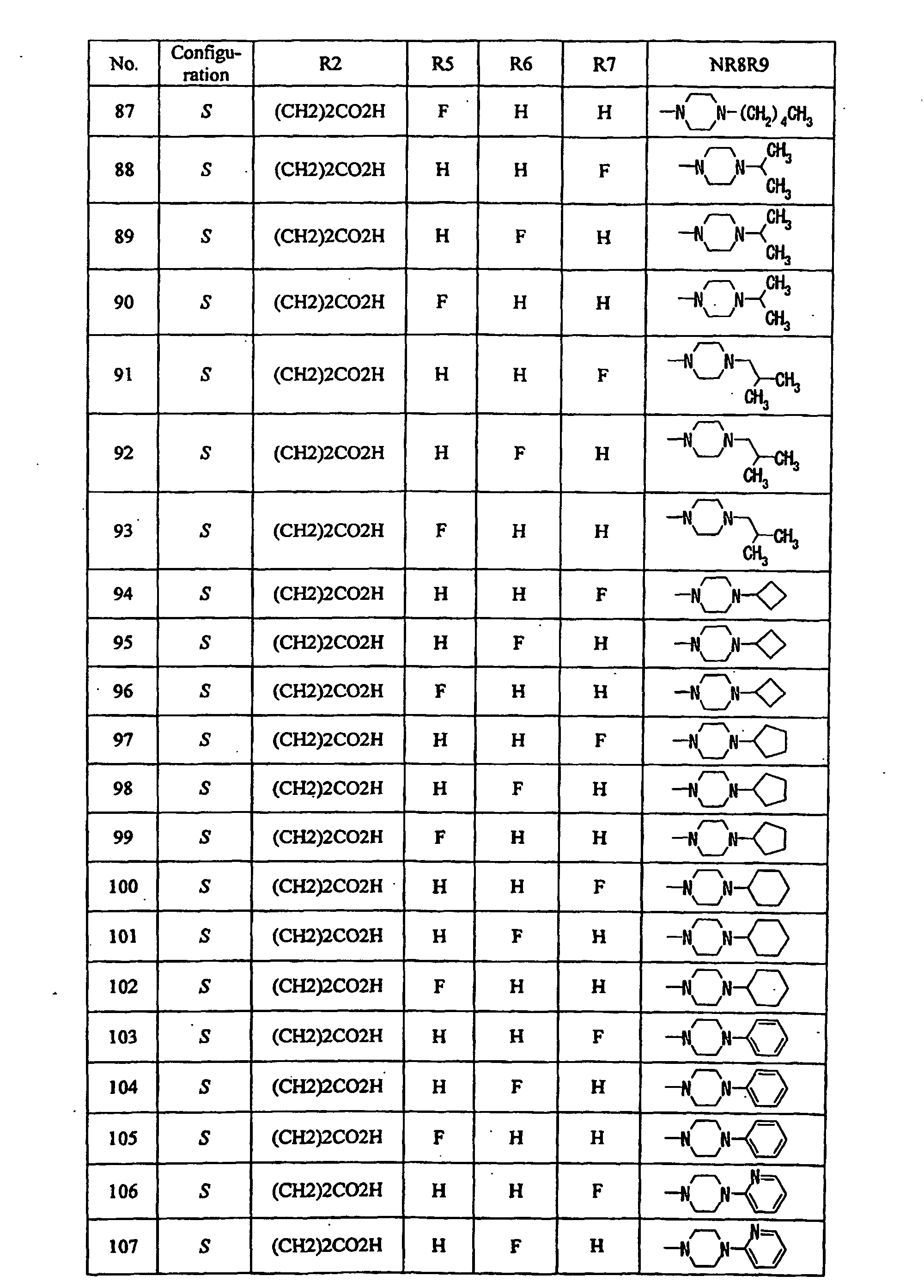

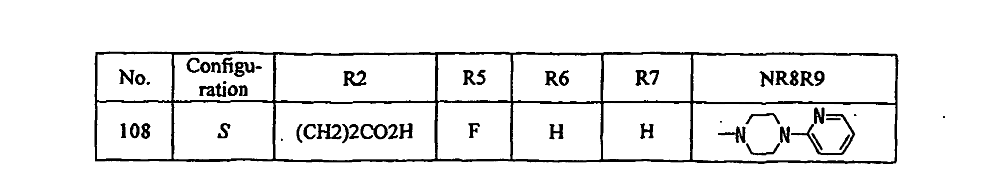

### Test Methods

The compounds of the invention were tested for their apo B-related lipoprotein secretion-inhibiting activity and for their blood cholesterol and triglyceride-lowering effect in rats, according to the methods mentioned below.

### 1. Measurement of apo B-related lipoprotein secretion in liver cells:

Hep G2 cells were incubated in 96-well plate with a 10 % fetal bovine serum-containing Dulbecco-modified Eagle medium (hereinafter referred to as "10 % FBS-containing DMEM). The medium was exchanged with a 10 % FBS-containing DMEM that contained a DMSO solution of the test compound (DMSO final concentration 0.1 %), and the cells were further incubated therein for 18 hours. The culture supernatant was collected. On the other hand, a 10 % FBS-containing DMEM that contained DMSO alone(final concentration 0.1 %) was used, and the culture supernatant was collected. This is control. The apo B-related lipoprotein produced in the culture supernatant was determined through enzyme immunoassay in the manner mentioned below.

A solution of anti-human apolipoprotein B monoclonal antibody (4 µg/ml) was put into every well (100 µl/well) of a 96-well plate and kept at 4°C for 18 hours. Each well was washed three times with a phosphate buffer to which had been added 0.1 % Tween 20 (hereinafter referred to as "PBS-T"), and 300 µl of a 4-fold diluted immune blocker, Block Ace was added thereto and kept at 37°C for 1 hour. The above-mentioned culture supernatant was added to it, and kept at 37°C for 3 hours. This was washed three times with PBS-T, and then 100 µl of a solution of caprine anti-human apolipoprotein B polyclonal antibody (2000-fold dilution) was added to it and kept at 37°C for 1 hour. This was washed three times with PBS-T, and 100 µl of a solution of alkali phosphatase-labeled anti-caprine IgG antibody (2000*-fold dilution) was added thereto and kept at 37°C for 1 hour. This was washed four times with PBS-T, once with PBS and then with a carbonate buffer (pH 9.5), and 100 µl of a solution of alkali phosphatase substrate was added to it and kept at room temperature for 5 minutes. 50 µl of 0.2 M sodium hydroxide was added to it to stop the reaction, and the absorbance at 405 nm of the reaction system was measured. From the calibration curve based on a standard, human low-density lipoprotein, the absolute amount of apo B-related lipoprotein in the reaction system was obtained.

The result is given in Table 10 below.

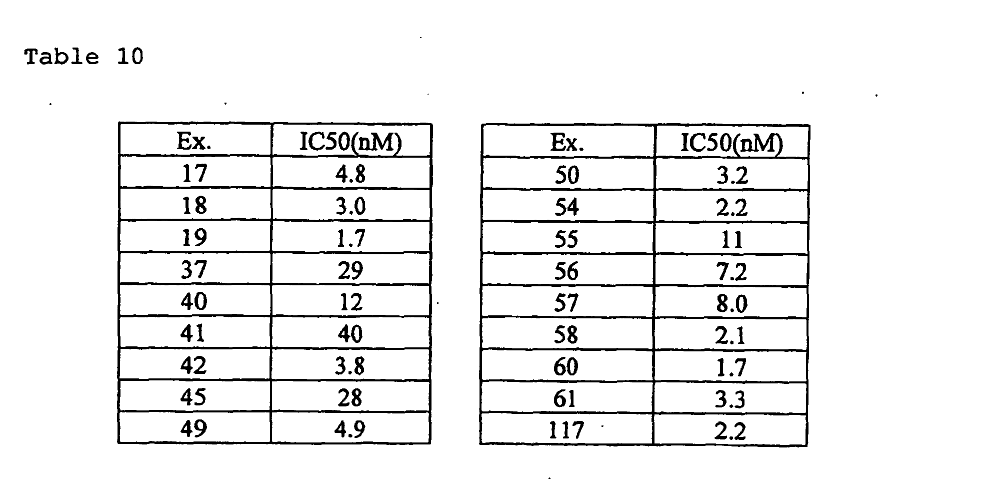

The data indicate the apo B-related lipoprotein secretion-inhibiting activity of the compounds of the invention.

### 2. Measurement of blood cholesterol and triglyceride in rats:

The influence of test compounds on VLDL secretion *in vivo* in rats was investigated. A 0.5 % methyl cellulose suspension containing a test compound was orally administered to male SD rats. After 1 hour, a physiological saline solution of Triton WR-1339 (corresponding to 400 mg/kg) was injected to the rats through their tail vein. After 4 hours, their blood was collected under anesthetization with diethyl ether. The total cholesterol and triglyceride in blood were measured by the use of "Cholesterol C Test Wako" and "Triglyceride G Test Wako" (both from Wako Pure Chemical), respectively. The rats orally administered with the 0.5 % methyl cellulose solution and then intravenously injected with physiological saline are in a normal group; and those orally administered with the 0.5 % methyl cellulose solution and then intravenously injected with physiological saline of Triton WR-1339 are in a control group. The difference in the total cholesterol (TC) or triglyceride (TG) in the serum between the control group and the normal group is the amount of secretion per hour, and the lowering effect in the amount of secretion of the test compound is represented by ED₅₀.

The result is given in Table 11 below.

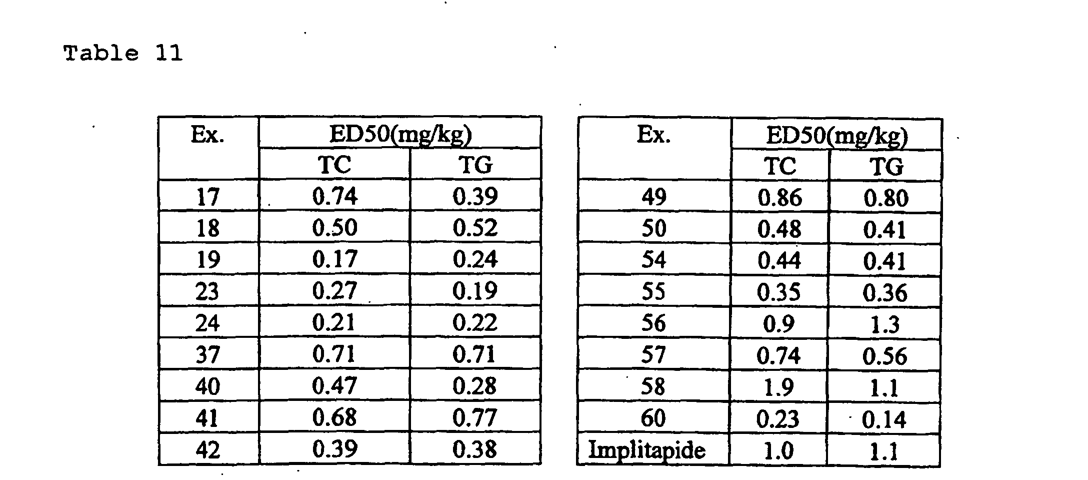

Implitapide: Compound of Example 5 in JP-A 8-225526

The cholesterol and triglyceride-lowering effect of the compounds of the invention is comparable to or higher than that of Implitapide having the cholesterol and triglyceride lowering effect.

### 3. Measurement of blood cholesterol in high-cholesterol feed-loaded rats:

The influence of test compounds on plasma lipid *in vivo* was investigated with high-cholesterol feed-loaded rats. A 0.5 % methyl cellulose suspension containing a test compound was forcedly orally administered everyday to male SD rats loaded with high-cholesterol feed (1.5 % cholesterol, 10 % coconut oil, 0.5 % cholic acid), once a day for continuous 7 days. After the final administration, the rats were fed with nothing, and after 6 hours, their blood was collected under anesthetization with diethyl ether. Using an automatic analyzer, Hitachi's Model 7250, the total cholesterol, HDL cholesterol and triglyceride in plasma were measured. Non-HDL cholesterol was obtained by subtracting HDL cholesterol from total cholesterol. The non-HDL cholesterol lowering effect of the test compound, relative to the control group, is represented by ED₅₀.

The result is given in Table 12 below.

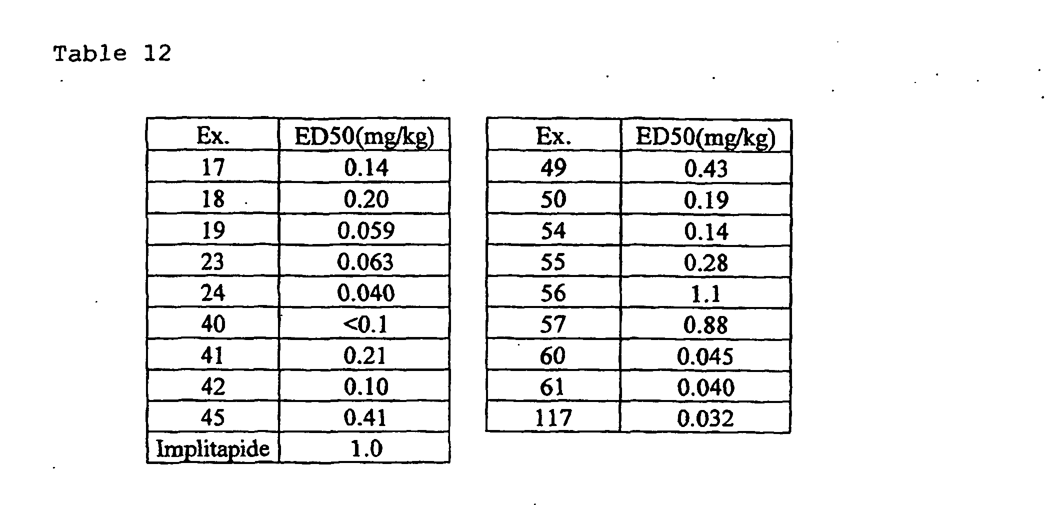

The data confirm that the compounds of the invention have an excellent cholesterol-lowering effect in dietary hyperchloesterolemia and their activity is comparable to or at least 30 times higher than that of Implitapide, and this supports the usefulness of the compounds as a cholesterol lowering agent.

### 4. Measurement of plasma lipid in KK-Ay mice:

The influence of test compounds on plasma lipid *in vivo* was investigated with KK-Ay mice. A 0.5 % methyl cellulose suspension containing a test compound was forcedly orally administered everyday to male KK-Ay mice once a day for continuous 7 days. After the final administration, the mice were fed with nothing, and after 6 hours, their blood was collected under anesthetization with diethyl ether. Using the automatic analyzer, Hitachi's Model 7250, the total cholesterol, HDL cholesterol and triglyceride in plasma were measured. Non-HDL cholesterol was obtained by subtracting HDL cholesterol from total cholesterol. The plasma lipid lowering effect of the test compound is expressed in percentage relative to the control group.

The result is given in Table 13 below.

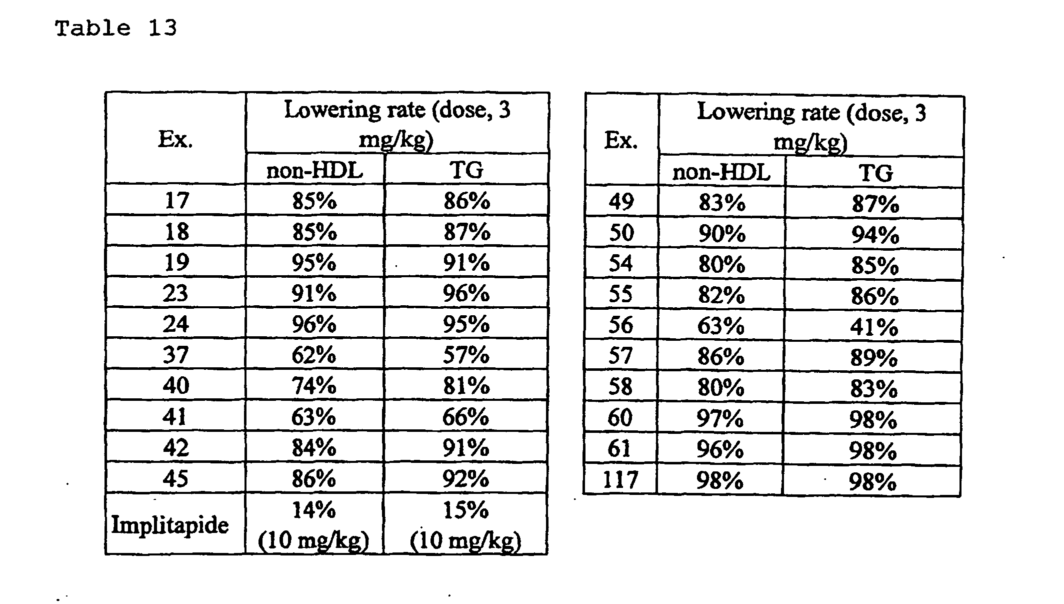

The data confirm that the compounds of the invention have an excellent cholesterol and triglyceride-lowering effect in disease models having high cholesterol and high triglyceride and their activity is higher than that of Implitapide, and this suggests the usefulness of the compound as a medicament for hyperlipemia.

### INDUSTRIAL APPLICABILITY

The compounds of the invention have apo B-related lipoprotein secretion-inhibiting activity and have excellent blood cholesterol and triglyceride-lowering effect, and they are useful for remedies for hyperlipemia, arteriosclerosis, obesity and pancreatitis.

## Claims

1. A tetrahydropyran derivative of the following general formula (I) or a salt thereof: 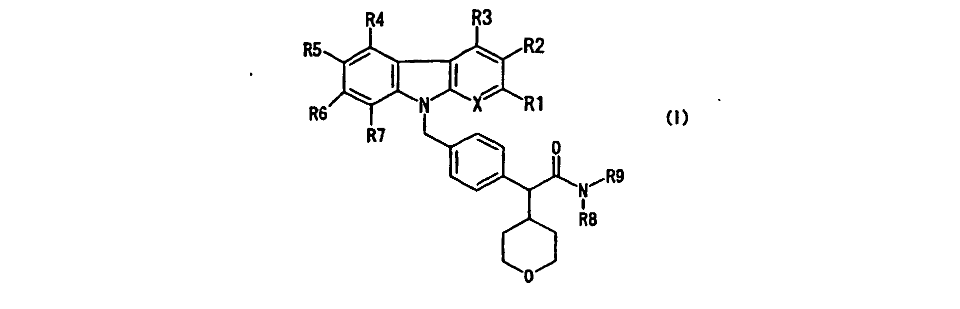 wherein R¹ and R³ are the same or different and each represents H or C₁-C₆ alkyl;
R² represents H, halogen, R^{a}- C₁-C₆ alkyl-, or R²⁰O-CO-;
R^{a} represents H, R²¹O-CO-, R²²R²³N-, R²⁴R²⁵N-CO-, R²⁶O-, cyano, or optionally-substituted hetero ring-;
R⁴, R⁵, R⁶ and R⁷ are the same or different and each represents H, halogen, haloalkyl, cyano, C₁-C₆ alkyl, C₁-C₆ alkyl-O-, R²¹O-CO- C₁-C₆ alkyl-, R²⁷-CO-, or R²⁸R²⁹N-S(O)₂-;
R⁸ and R⁹ are the same or different and each represents H, C₁-C₆ alkyl, R³⁰- C₁-C₆ alkyl-, R³¹R³²N-, optionally-substituted hetero ring, or R³³R³⁴R³⁵C-;
R⁸ and R⁹ may together form optionally-substituted hetero ring-;
X represents N or CR³⁶;
R²⁰, R²² to R²⁶, R²⁸, R²⁹, R³², R³⁵ and R³⁶ are the same or different and each represents H or C₁-C₆ alkyl;
R²³ represents H, C₁-C₆ alkyl, or aryl- C₁-C₆ alkyl-;
R²⁷ represents HO-, C₁-C₆ alkyl-O-, or optionally-substituted hetero ring-;
R³⁰ represents optionally-substituted aryl, optionally-substituted hetero ring-, or C₁-C₆ alkyl-O-;
R³¹ represents optionally-substituted aryl, or optionally-substituted hetero ring-;
R³³ represents HO- C₁-C₆ alkyl-, or optionally-substituted hetero ring- C₁-C₆ alkyl;
R³⁴ represents optionally-substituted aryl-;
wherein aryl is a mono- to tricyclic aromatic hydrocarbon group that is 6- to 14-membered; and hetero ring represents a saturated hetero ring or an unsaturated hetero ring which are optionally-condensed having from 1 to 3 hetero atoms selected from nitrogen, oxygen and sulfur atoms; unsaturated hetero ring means an optionally-condensed, 5- or 6-membered unsaturated heterocyclic group having from 1 to 3 hetero atoms selected from nitrogen, oxygen and sulfur atoms; and saturated hetero ring means an optionally-condensed, 3- to 10-membered saturated hetero ring having from 1 to 3 hetero atoms selected from nitrogen, oxygen and sulfur atoms; and
wherein the optionally-substituted arylgroups and the optionally-substituted hetero ring groups may have from 1 to 3 substituents selected from halogen, oxo, C₁-C₆ alkyl optionally substituted with OH, haloalkyl optionally substituted with C₁-C₆ alkyl-O-, C₂-C₆ alkenyl, C₂-C₆ alkynyl, C₃-C₈ cycloalkyl, C₃-C₈ cycloalkyl- C₁-C₆ alkyl-, aryl optionally substituted with halogen, aryl- C₁-C₆ alkyl-, hetero ring-, hetero ring- C₁-C₆ alkyl-, HO-, C₁-C₆ alkyl-O-, C₁-C₆ alkyl-O- C₁-C₆ alkyl- optionally substituted with OH, C₁-C₆ alkyl-O- C₁-C₆ alkyl-CO-, C₂-C₆ alkenyl-O-, C₂-C₆ alkynyl-O-, C₃-C₈ cycloalkyl-O-, aryl-O-, aryl-C₁-C₆ alkyl-O-, hetero ring-O-, hetero ring- C₁-C₆ alkyl-O-, C₁-C₆ alkyl-S(O)ₘ-, C₁-C₆ alkyl-S(O)ₘ- C₁-C₆ alkyl-, C₂-C₆ alkenyl-S(O)ₘ-, C₂-C₆ alkynyl-S(O)ₘ-, C₃-C₈ . cycTvalkyl-S (0) ₘ-, aryl-S (O) ₘ-, aryl- C₁-C₆ alkyl-S(O)ₘ-, hetero ring-S(O)ₘ-, hetero ring- C₁-C₆ alkyl-S(O)ₘ-, C₁-C₆ alkyl-O-CO-, C₂-C₆ alkenyl-O-CO-, C₂-C₆ alkynyl-O-CO-, C₃-C₈ cycloalkyl-O-CO-, aryl-O-CO, aryl- C₁-C₆ alkyl-O-CO-, hetero ring-O-CO-, hetero ring- C₁-C₆ alkyl-O-CO-, HO-CO-, HO-CO- C₁-C₆ alkyl-, HO-CO- C₁-C₆ alkyl- O-, Ci-C₆ alkyl-CONR^{x}-, C₁-C₆ alkyl-CONR^{x}- C₁-C₆ alkyl-, R^{x}R^{y}N-, R^{x}R^{y}N-C₁-C₆ alkyl-, R^{x}R^{y}N-CO-, R^{x}R^{y}N-CO- C₁-C₆ alkyl, HCO-, C₁-C₆ alkyl-CO-, C₂-C₆ alkenyl-CO-, C₂-C₆ alkynyl-CO-, C₃-C₈ cycloalkyl-CO-, aryl-CO-, aryle- C₁-C₆ alkyl-CO-, hetero ring-CO-, hetero ring- C₁-C₆ alkyl-CO-, HCS-, C₁-C₆ alkyl-CS-, C₂-C₆ alkenyl-CS-, C₂-C₆ alkynyl-CS-, C₃-C₈ cycloalkyl-CS-, aryl-CS-, aryl-C₁-C₆ alkyl-CS-, hetero ring-CS-, hetero ring- C₁-C₆ alkyl-CS-, C₁-C₆ alkyl-o-CO-CO-, HCO-O-, C₁-C₆ alkyl-CO-O-, C₂-C₆ alkenyl-CO-O-, C₂-C₆ alkynyl-CO-O-, C₃-C₆ cycloalkyl-CO-O-, aryl-CO-O, aryl- C₁-C₆ alkyl-CO-O-, hetero ring-CO-O-, hetero ring-C₁-C₆ alkyl-CO-O-, aryl- C₁-C₆ alkyl-CO-O-, hetero ring-O-CO-; wherein m = 0, 1 or .2; and R^{x} and R^{y} are the same or different and each represents H or C₁-C₆ alkyl.

2. A tetrahydropyran derivative of the formula (I) or a salt thereof according to claim 1, wherein the optionally-substituted aryl and the optionally-substituted hetero ring are optionally substituted with 1 to 3 substituents selected from: halogen, oxo, C₁-C₆ alkyl optionally substituted with OH, haloalkyl optionally substituted with C₁-C₆ alkyl-O-, C₂-C₆ alkenyl, C₃-C₈ cycloalkyl- , C₃-C₈ cycloalkyl- C₁-C₆ alkyl-, aryl optionally substituted with halogen, aryl- C₁-C₆ alkyl-, hetero ring-, HO-, C₁-C₆ alkyl-O-, C₁-C₆ alkyl-O- C₁-C₆ alkyl optionally substituted with OH, C₁-C₆ alkyl-O- C₁-C₆ alkyl-CO-, C₁-C₆ alkyl-S(O)ₘ, C₁-C₆ alkyl-S(O)ₘ-C₁-C₆ alkyl-, C₁-C₆ alkyl-O-CO-, HO-CO- C₁-C₆ alkyl-, HO-CO- C₁-C₆ alkyl-O-, R^{x}R^{y}N-CO-, R^{x}R^{y}N-CO- C₁-C₆ alkyl-, R^{x}R^{y}N- C₁-C₆ alkyl-, C₁-C₆ alkyl-CO-, aryl- C₁-C₆ alkyl-O-CO-, and hetero ring-O-CO,
wherein R^{x} and R^{y} are the same or different and each represents H or C₁-C₆ alkyl, and m indicates O, 1 or 2.

3. A tetrahydropyran derivative of the formula (I) or a salt thereof as claimed in claim 1, wherein R2 is R^{a}-C₁-C₆ alkyl; R⁴, R⁵, R⁶ and R⁷ are the same or different, each representing H or halogen; R⁸ and R⁹ are the same or different, each representing H, C₁-C₆ alkyl-, R³⁰-C₁-C₆ alkyl-, or R⁸ and R⁹ may together form a hetero ring; and X is N.

4. A compound selected from the group consisting of
3-(2,4-dimethyl-9-{4-[(S)-2-oxo-2-(4-pyridin-2-ylpiperazin-1-yl)(tetrahydro-2H-pyran-4-yl)ethyl]benzyl}-9H-pyrido[2,3-b]indol-3-yl)propanoic acid;
3-(2,4-dimethyl-9-{4-[(S)-2-oxo-2-(4-propylpiperazin-1-yl)-1-(tetrahydro-2H-pyran-4-yl)ethyl]benzyl}-9H-pyrido[2,3-b]indol-3-yl)propanoic acid;
3-(9-{4-[(S)-(benzylcarbamoyl) (tetrahydro-2H-pyran-4-yl)methyl]benzyl}-2,4-dimethyl-9H-pyrido[2,3-b]indol-3-yl)propanoic acid;
3-(2,4-dimethyl-9-{4-[(S)-(dimethylcarbamoyl)(tetrahydro-2H-pyran-4-yl)methyl]benzyl}-9H-pyrido[2,3-b]indol-3-yl)propanoic acid;
3-(2,4-dimethyl-9-(4-[(S)-2-morpholin-4-yl-2-oxo-1-(tetrahydro-2H-pyran-4-yl)ethyljbenzyl)-9H-pyrido[2,3-b]indol-3-yl)propanoic acid;
3-(2,4-dimethyl-9-{4-[(S)-2-(4-isobutylpiperazin-1-yl)-2-oxo-1-(tetrahydro-2H-pyran-4-yl)ethyl]benzyl}-9H-pyrido[2,3-b]indol-3-yl)propanoic acid;
3-(9-{4-[(S)-2-(4-cyclobutylpiperazin-1-yl)-2-oxo-1-(tetrahydro-2H-pyran-4-yl)ethyl]benzyl}-2,4- dimethyl-9H-pyrido[2,3-b]indol-3-yl)propanoic acid;
3-(9-{4-[(S)-2-(4-cyclopentylpiperazin-1-yl)-2-oxo-1-(tetrahydro-2H-pyran-4-yl)ethyl]benzyl}-2,4-dimethyl-9H-pyrido[2,3-b]indol-3-yl)propanoic acid;
and their salts.

5. A pharmaceutical composition which comprises, as an active ingredient, a tetrahydropyran derivative or a pharmaceutically acceptable salt thereof as claimed in any one of claims 1 to 4 and a pharmaceutically acceptable carrier.

6. Use of a tetrahydropyran derivative or a pharmaceutically acceptable salt thereof as claimed in any one of claims 1 to 4 for the production of a medicament for treating hyperlipemia.

## Patentansprüche

1. Tetrahydropyranderivat der folgenden allgemeinen Formel (I) oder ein Salz davon: 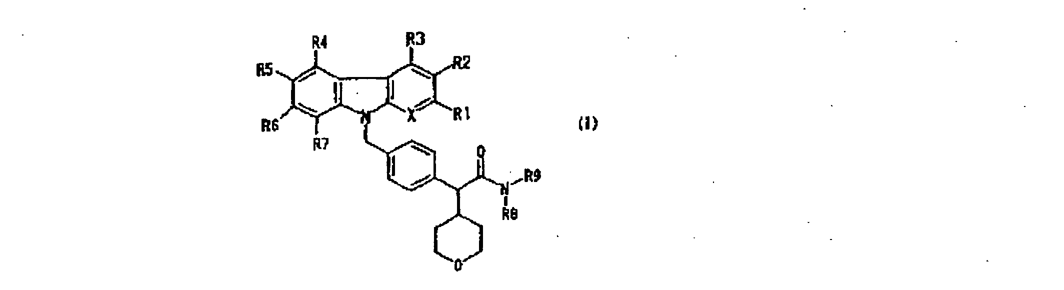 wobei R¹ und R³ gleich oder unterschiedlich sind und jeweils H oder C₁-C₆ Alkyl repräsentieren;
R² H, Halogen, R^{a}- C₁-C₆ Alkyl- oder R²⁰O-CO-repräsentiert;
R^{a} H, R²¹O-CO-, R²²R²³N-, R²⁰R²⁵N-CO-, R²⁶O-, Cyano oder einen optional substituierten Heteroring- repräsentiert;
R⁴, R⁵, R⁶ und R⁷ gleich oder unterschiedlich sind und jeweils H, Halogen, Haloalkyl, Cyano, C₁-C₆ Alkyl, C₁-C₆ Alkyl-O-, R²¹O-CO- C₁-C₆ Alkyl-, R²⁷-CO- oder R²⁸R²⁹N-S(O)₂-repräsentieren;
R⁸ und R⁹ gleich oder unterschiedlich sind und jeweils H, C₁-C₆ Alkyl, R³⁰- C₁-C₆ Alkyl-, R³¹R³²N-, einen optional substituierten Heteroring oder R³³R³⁴R³⁵C- repräsentieren;
R⁸ und R⁹ zusammen einen optional substituierten Heteroring- bilden können;
X N oder CR³⁶ repräsentiert;
R²⁰, R²² bis R²⁶, R²⁸, R²⁹, R³², R³⁵ und R³⁶ gleich oder unterschiedlich sind und jeweils H oder C₁-C₆ Alkyl repräsentieren;
R²¹ H, C₁-C₆ Alkyl oder Aryl- C₁-C₆ Alkylrepräsentiert;
R²⁷ HO-, C₁-C₆ Alkyl-O- oder einen optional substituierten Heteroring- repräsentiert;
R³⁰ optional substituiertes Aryl, einen optional substituierten Heteroring- oder C₁-C₆ Alkyl-O-repräsentiert;
_{R}³¹ optional substituiertes Aryl oder einen optional substituierten Heteroring- repräsentiert;
R³³ HO- C₁-C₆ Alkyl- oder ein optional substituiertes Heteroring- C₁-C₆ Alkyl repräsentiert;
R³⁴ optional substituiertes Aryl repräsentiert;
wobei Aryl eine mono- bis trizyklische, 6- bis 14-gliedrige aromatische Kohlenwasserstoffgruppe ist; und der Heteroring einen gesättigten Heteroring oder einen ungesättigten Heteroring repräsentiert, der optional kondensiert ist und 1 bis 3 Heteroatome hat, die ausgewählt sind aus Stickstoff-, Sauerstoff- und Schwefelatomen; wobei der ungesättigte Heteroring eine optional kondensierte, 5-oder 6-gliedrige, ungesättigte heterozyklische Gruppe mit 1 bis 3 Heteroatomen ist, die ausgewählt sind aus Stickstoff-, Sauerstoff- und Schwefelatomen; und der gesättigte Heteroring ein optional kondensierter, 3- bis 10-gliedriger, gesättigter Heteroring mit 1 bis 3 Heteroatomen ist, die ausgewählt sind aus Stickstoff-, Sauerstoff- und Schwefelatomen; und wobei die optional substituierten Arylgruppen und die optional substituierten Heteroringgruppen 1 bis 3 Substituenten haben können, die ausgewählt sind aus Halogen, Oxo, C₁-C₆ Alkyl, optional substituiert durch OH, Haloalkyl, optional substituiert durch C₁-C₆ Alkyl-0-, C₂-C₆ Alkenyl, C₂-C₆ Alkynyl, C₃-C₈ Cycloalkyl, C₃-C₈ Cycloalkyl- C₁-C₆ Alkyl-, Aryl, optional substituiert durch Halogen, Aryl- C₁-C₆ Alkyl-, Heteroring-, Heteroring- C₁-C₆ Alkyl-, HO-, C₁-C₆ Alkyl-0-, C₁-C₆ Alkyl-0-C₁-C₆ Alkyl-, optional substituiert durch OH, C₁-C₆ Alkyl-O-C₁-C₆ Alkyl-CO-, C₂-C₆ Alkenyl-O-, C₂-C₆ Alkynyl-O-, C₃-C₈ Cycloalkyl-O-,Aryl-O-, Aryl-C₁-C₆ Alkyl-O-, Heteroring-O-, Heteroring- C₁-C₆ Alkyl-O-, C₁-C₆ Alkyl-S(O)ₘ-, C₁-C₆ Alkyl-S(O)ₘ- C₁-C₆ Alkyl-, C₂-C₆ Alkenyl-S(O)ₘ-, C₂-C₆ Alkynyl-S (O)ₘ-, C₃-C₈ Cycloalkyl-S(O)ₘ-, Aryl-S(O)ₘ-, Aryl- C₁-C₆ Alkyl-S(O)ₘ-, Heteroring-S(O)ₘ-, Heteroring- C₁-C₆ Alkyl-S(O)ₘ-, C₁-C₆ Alkyl-O-CO-, C₂-C₆ Alkenyl-O-CO-, C₂-C₆ Alkynyl-O-CO-, C₃-C₈ Cycloalkyl-O-CO-, Aryl-O-CO, Aryl- C₁-C₆ Alkyl-O-CO-, Heteröring-O-CO-, Heteroring- C₁-C₆ Alkyl-O-CO-, HO-CO-, HO-CO- C₁-C₆ Alkyl-, HO-CO- C₁-C₆ Alkyl- O-, C₁-C₆ Alkyl-CONR^{x}-, C₁-C₆ Alkyl-CONR^{x}- C₁-C₆ Alkyl-, R^{x}R^{y}N-, R^{x}R^{y}N-C₁-C₆ Alkyl-, R^{x}R^{y}N-CO-, R^{x}R^{y}N-CO- C₁-C₆ Alkyl, HCO-, C₁-C₆ Alkyl-CO-, C₂-C₆ Alkenyl-CO-, C₂-C₆ Alkynyl-CO-, C₃-C₈-Cycloalkyl-CO-, Aryl-CO-, Aryl- C₁-C₆ Alkyl-CO-, Heteroring-CO-, Heteroring- C₁-C₆ Alkyl-CO-, HCS-, C₁-C₆ Alkyl-CS-, C₂-C₆ Alkenyl-CS-, C₂-C₆ Alkynyl-CS-, C₃-C₈ Cycloalkyl-CS-, Aryl-CS-, Aryl-C₁-C₆ Alkyl-CS-, Heteroring-CS-, Heteroring-C₁-C₆ Alkyl-CS-, C₁-C₆ Alkyl-O-CO-CO-, HCO-O-, C₁-C₆ Alkyl-CO-O-, C₂-C₆ Alkenyl-CO-O-, C₂-C₆ Alkynyl-CO-O-, C₃-C₈ Cycloalkyl-CO-O-, Aryl-CO-O, Aryl- C₁-C₆ Alkyl-CO-O-, Heteroring-CO-O-, Heteroring-C₁-C₆ Alkyl-CO-O-, Aryl- C₁-C₆ Alkyl-CO-O-, Heteroring-O-CO; wobei m = 0, 1 oder 2 ist; und R^{x} und R^{y} gleich oder unterschiedlich sind und jeweils H oder C₁-C₆ Alkyl repräsentieren.

2. Tetrahydropyranderivat der Formel (I) oder ein Salz davon nach Anspruch 1, wobei das optional substituierte Aryl und der optional substituierte Heteroring optional substituiert sind durch 1 bis 3 Substituenten, ausgewählt aus: Halogen, Oxo, C₁-C₆ Alkyl, optional substituiert durch OH, Haloalkyl, optional substituiert durch C₁-C₆ Alkyl-O-, C₂-C₆ Alkenyl, C₃-C₈ Cycloalkyl-, C₃-C₈ Cycloalkyl- C₁-C₆ Alkyl-, Aryl, optional substituiert durch Halogen, Aryl- C₁-C₆ Alkyl-, Heteroring-, HO-, C₁-C₆ Alkyl-O-, C₁-C₆ Alkyl-O-C₁-C₆ Alkyl, optional substituiert durch OH, C₁-C₆ Alkyl-O-C₁-C₆ Alkyl-CO-, C₁-C₆ Alkyl-S(O)ₘ, C₁-C₆ Alkyl-S(O)ₘ-C₁-C₆ Alkyl-, C₁-C₆ Alkyl-O-CO-, HO-CO- C₁-C₆ Alkyl-, HO-CO- C₁-C₆ Alkyl-O-, R^{x}R^{y}N-CO-, R^{x}R^{y}N-CO- C₁-C₆ Alkyl-, R^{x}R^{y}N- C₁-C₆ Alkyl-, C₁-C₆ Alkyl-CO-, Aryl- C₁-C₆ Alkyl-O-CO- und Heteroring-O-CO, wobei R^{x} und R^{y} gleich oder unterschiedlich sind und jeweils H oder C₁-C₆ Alkyl repräsentieren und m 0, 1 oder 2 ist.

3. Tetrahydropyranderivat der Formel (I) oder ein Salz davon nach Anspruch 1, wobei R2 Rⁿ-C₁-C₆ Alkyl ist; R⁴, R⁵, _{R}⁶ und R⁷ gleich oder unterschiedlich sind und jeweils H oder Halogen repräsentieren; R⁸ und R⁹ gleich oder unterschiedlich sind und jeweils H, C₁-C₆ Alkyl-, R³⁰-C¹-C⁶ Alkyl- repräsentieren oder R⁸ und R⁹ zusammen einen Heteroring bilden können; und X N ist.

4. Verbindung, ausgewählt aus der Gruppe bestehend aus
3-(2,4-Dimethyl-9-(4-[(S)-2-oxo-2-(4-pyridin-2-ylpiperazin-1-yl)(tetrahydro-2H-pyran-4-yl)ethyl]benzyl)-9H-pyrido[2,3-b]indol-3-yl)propionsäure;
3-(2,4-Dimethyl-9-(4-[(S)-2-oxo-2-(4-propylpiperazin-1-yl)-1-(tetrahydro-2H-pyran-4-yl)ethyl]benzyl)-9H-pyrido[2,3-b]indol-3-yl)propionsäure;
3- (9- (4- [ (S) - (Benzylcarbamoyl) (tetrahydro-2H-pyran-4-yl)methyl]benzyl)-2,4-dimethyl-9H-pyrido[2,3-b]indol-3-yl)propionsäure;
3-(2,4-Dimethyl-9-(4-[(S)-(dimethylcarbamoyl) (tetrahydro-2H-pyran-4-yl)methyl]benzyl)-9H-pyrido[2,3-b]indol-3-yl)propionsäure;
3-(2,4-Dimethyl-9-(4-[(S)-2-morpholin-4-yl-2-oxo-1-(tetrahydro-2H-pyran-4-yl)ethyl]benzyl)-9H-pyrido[2,3-b]indol-3-yl)propionsäure;
3-(2,4-Dimethyl-9-(4-[(S)-2-(4-isobutylpiperazin-1-yl)-2-oxo-1-(tetrahydro-2H-pyran-4-yl)ethyl]benzyl)-9H-pyrido[2,3-b]indol-3-yl)propionsäure;
3- (9- (4- [(S)-2- (4-Cyclobutylpiperazin-1-yl) -2-oxo-1-(tetrahydro-2H-pyran-4-yl)ethyl]benzyl)-2,4-dimethyl-9H-pyrido[2,3-b]indol-3-yl)propionsäure;
3-(9-(4-[(S)-2-(4-Cyclopentylpiperazin-1-yl)-2-oxo-1-(tetrahydro-2H-pyran-4-yl)ethyl]benzyl)-2,4-dimethyl-9H-pyrido[2,3-b]indol-3-yl)propionsäure;
und ihre Salze.

5. Pharmazeutische Zusammensetzung, die als Wirkstoff ein Tetrahydropyranderivat oder ein pharmazeutisch akzeptables Salz davon nach einem der Ansprüche 1 bis 4 und einen pharmazeutisch akzeptablen Träger umfasst.

6. Verwendung eines Tetrahydropyranderivats oder eines pharmazeutisch akzeptablen Salzes davon nach einem der Ansprüche 1 bis 4 zur Herstellung eines Medikamentes zur Behandlung von Hyperlipämie.

## Revendications

1. Dérivé de tétrahydropyrane de la formule générale suivante (I) ou un sel de celui-ci: 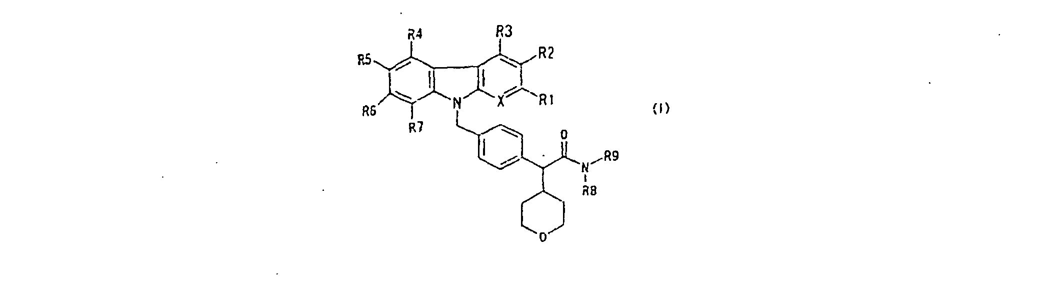 où R¹ et R³ sont les mêmes ou différents et chacun représente H ou un alkyle C₁-C₆;
R² représente H, un halogène, R^{a}- alkyle C₁-C₆-, ou R²⁰ O-CO-;
R^{a} représente H, R²¹O-CO-, R²²R²³N-, R²⁴R²⁵N-CO-, R²⁶O-, un cyano ou un hétérocycle-optionnellement substitué;
R⁴, R⁵, R⁶ et R⁷ sont les mêmes ou différents et chacun représente H, un halogène, un halo-alkyle, un cyano, un alkyle C₁-C₆, un alkyle C₁-C₆-O-, R²¹O-CO-alkyle C₁-C₆-, R²⁷-CO-, ou R²⁸R²⁹N-S(O)₂-;
R⁸ et R⁹ sont les mêmes ou différents et chacun représente H, alkyle C₁-C₆, R³⁰- alkyle C₁-C₆-, R³¹R³²N-, un hétérocycle optionnellement substitué, ou R³³R³⁴R³⁵C-;
R⁸ et R⁹ peuvent former ensemble un hétérocycle- optionnellement substitué;
X représente N ou CR³⁶;
R²⁰, R²² à R²⁶, R²⁸, R²⁹, R³², R³⁵ et R³⁶ sont les mêmes ou différents et chacun représente H ou un alkyle C₁-C₆;
R²¹ représente H, un alkyle C₁-C₆, ou un aryle-alkyle C₁-C₆-;
R²⁷ représente HO-, alkyle C₁-C₆-O-, ou un hétérocycle- optionnellement substitué;
R³⁰ représente un aryle optionnellement substitué, un hétérocycle- optionnellement substitué, ou un alkyle C₁-C₆-O-;
R³¹ représente un aryle optionnellement substitué, ou un hétérocycle- optionnellement substitué;
R³³ représente HO-alkyle C₁-C₆-, ou un hétérocycle-alkyle C₁-C₆ optionnellement substitué;
R³⁴ représente un aryle- optionnellement substitué;
où un aryle est un groupe d'hydrocarbure aromatique de mono à tricyclique qui a de 6 à 14 chaînons; et un hétérocycle représente un hétérocycle saturé ou un hétérocycle insaturé qui sont optionnellement condensés ayant de 1 à 3 hétéro-atomes sélectionnés parmi les atomes d'azote, d'oxygène et de soufre; un hétérocycle insaturé signifie un groupe hétérocyclique insaturé à 5 ou 6 chaînons, optionnellement condensé, ayant de 1 à 3 hétéro-atomes sélectionnés parmi les atomes d'azote, d'oxygène et de soufre; et un hétérocycle saturé signifie un hétérocycle saturé de 3 à 10 chaînons, optionnellement condensé, ayant de 1 à 3 hétéro-atomes sélectionnés parmi les atomes d'azote, d'oxygène et de soufre; et où les groupes aryle optionnellement substitués et les groupes hétérocycle optionnellement substitués peuvent avoir de 1 à 3 substituants sélectionnés parmi l'halogène, oxo, alkyle C₁-C₆ optionnellement substitué par OH, haloalkyle optionnellement substitué par alkyle C₁-C₆-O-, alcényle C₂-C₆, alcynyle C₂-C₆, cycloalkyle C₃-C₈, cycloalkyle C₃-C₈-alkyle C₁-C₆-, aryle optionnellement substitué par un halogène, aryle-alkyle C₁-C₆-, hétérocycle-, hétérocycle-alkyle C₁-C₆-, HO-, alkyle C₁-C₆-O-, alkyle C₁-C₆-O-alkyle C₁-C₆- optionnellement substitué par OH, alkyle C₁-C₆-O-alkyle C₁-C₆-CO-, alcényle C₂-C₆-O-, alcynyle C₂-C₆-O-, cycloalkyle C₃-C₈-O-, aryle-O-, aryle-alkyle C₁-C₆-O-, hétérocycle-O-, hétérocycle-alkyle C₁-C₆-O-, alkyle C₁-C₆-S (O)ₘ-alkyle C₁-C₆-, alcényle C₂-C₆-S (O)ₘ-, alcynyle C₂-C₆-S (O)ₘ-, cycloalkyle C₃-C₈-S (O)ₘ-, aryle-S (O)ₘ-, aryle-alkyle C₁-C₆-S (O)ₘ-, hétérocycle-S (O)ₘ-, hétérocycle-alkyle C₁-C₆-S (O)ₘ-, alkyle C₁-C₆-O-CO-, alcényle C₂-C₆-O-CO-, alcynyle C₂-C₆-O-CO-, cycloalkyle C₃-C₈-O-CO-, aryle-O-CO, aryle-alkyle C₁-C₆-O-CO-, hétérocycle-O-CO-, hétérocycle-alkyle C₁-C₆-O-CO-, HO-CO, HO-CO-alkyle C₁-C₆-, HO-CO-alkyle C₁-C₆-O-, alkyle C₁-C₆-CONR^{x}-, alkyle C₁-C₆-CONR^{x}-alkyle C₁-C₆, R^{x}R^{y}N-, R^{x}R^{y}N-alkyle C₁-C₆-, R^{x}R^{y}N-CO-, R^{x}R^{y}N-CO-alkyle C₁-C₆, HCO-, alkyle C₁-C₆-CO-, alcényle C₂-C₆-CO-, alcynyle C₂-C₆-CO-, cycloalkyle C₃-C₈-CO-, aryle-CO-, aryle-alkyle C₁-C₆-CO-, hétérocycle-CO-, hétérocycle-alkyle C₁-C₆-CO-, HCS-, alkyle C₁-C₆-CS-, alcényle C₂-C₆-CS-, alcynyle C₂-C₆-CS-, cycloalkyle C₃-C₈-CS-, aryle-CS-, aryle-alkyle C₁-C₆-CS-, hétérocycle-CS-, hétérocycle-alkyle C₁-C₆-CS-, alkyle C₁-C₆-O-CO-CO-, HCO-O-, alkyle C₁-C₆-CO-O-, alcényle C₂-C₆-CO-O-, alcynyle C₂-C₆-CO-O-, cycloalkyle C₃-C₈-CO-O-, aryle-CO-O, aryle-alkyle C₁-C₆-CO-
O-, hétérocycle-CO-O-, hétérocycle-alkyle C₁-C₆-CO-O-, aryle-alkyle C₁-C₆-CO-O-, hétérocycle-O-CO-; où m = O, 1 ou 2; et R^{x} et R^{y} sont les mêmes ou différents et chacun représente H ou un alkyle C₁-C₆.

2. Dérivé de tétrahydropyrane de la formule (I) ou un sel de celui-ci selon la revendication 1, dans lequel l'aryle optionnellement substitué et l'hétérocycle optionnellement substitué, sont optionnellement substitués par de 1 à 3 substituants sélectionnés parmi : halogène, oxo, alkyle C₁-C₆ optionnellement substitué par OH, haloalkyle optionnellement substitué par alkyle C₁-C₆-O-, alcényle C₂-C₆, cycloalkyle C₃-C₈, cycloalkyle C₃-C₈-alkyle C₁-C₆-, aryle optionnellement substitué par halogène, aryle-alkyle C₁-C₆-, hétérocycle-, HO-, alkyle C₁-C₆-O-, alkyle C₁-C₆-O-alkyle C₁-C₆ optionnellement substitué par OH, alkyle C₁-C₆-O-alkyle C₁-C₆-CO-, alkyle C₁-C₆-S (O)ₘ, alkyle C₁-C₆-S (O)m-alkyle C₁-C₆-, alkyle C₁-C₆-O-CO-, HO-CO-alkyle C₁-C₆-, HO-CO-alkyle C₁-C₆-O-, R^{x}R^{y}N-CO-, R^{x}R^{y}N-CO-alkyle C₁-C₆-, R^{x}R^{y}N -alkyle C₁-C₆-, alkyle C₁-C₆-CO-, aryle-alkyle C₁-C₆-O-CO-, et hétérocycle-O-CO, où R^{x} et R^{y} sont les mêmes ou différents et chacun représente H ou alkyle C₁-C₆, et m indique 0, 1 ou 2.

3. Dérivé de tétrahydropyrane de la formule (I) ou un sel de celui-ci tel que revendiqué dans la revendication 1, où R2 est un alkyle R^{a}-C₁-₆; R⁴, R⁵, R⁶ et R⁷ sont les mêmes ou différents, chacun représentant H ou un halogène; R⁸ et R⁹ sont les mêmes ou différents, chacun représentant H, alkyle C₁-C₆-, R³⁰-alkyle C₁-C₆-, ou R⁸ et R⁹ peuvent former ensemble un hétérocycle; et X est N.

4. Composé sélectionné parmi le groupe consistant en
acide 3-(2,4-diméthyl-9-{4-[(S)-2-oxo-2-(4-pyridin-2-ylpipérazin-1-yl) (tétrahydro-2H-pyran-4-yl) éthyl] benzyl}-9H-pyrido [2, 3-b] indol-3-yl) propanoïque;
acide 3-(2,4-diméthyl-9-{4-[(S)-2-oxo-2-(4-propylpipérazin-1-yl)-1-(tétrahydro-2H-pyran-4-yl) éthyl] benzyl}-9H-pyrido [2, 3-b] indol-3-yl) propanoïque;
acide 3-(9- {4-[(S)-(benzylcarbamoyl) (tétrahydro-2H-pyran-4-yl) méthyl] benzyl}-2, 4-diméthyl-9H-pyrido [2, 3-b] indol-3-yl) propanoïque;
acide 3-(2,4-diméthyl-9-{4-[(S)-(diméthylcarbamoyl) (tétrahydro-2H-pyran-4-yl) méthyl] benzyl}-9H-pyrido [2, 3-b] indol-3-yl) propanoïque;
acide 3-(2,4-diméthyl-9-{4-[(S)-2-morpholin-4-yl-2-oxo-1-(tétrahydro-2H-pyran-4-yl) éthyl] benzyl}-9H-pyrido [2, 3-b] indol-3-yl) propanoïque;
acide 3-(2,4-diméthyl-9-{4-[(S)-2-(4-isobultylpipérazin-1-yl)-2-oxo-1-(tétrahydro-2H-pyran-4-yl) éthyl] benzyl}-9H-pyrido [2, 3-b] indol-3-yl) propanoïque;
acide 3-(9-{4- [(S)-2-4(4-cyclobutylpipérazin-1-yl)-2-oxo-1- (tétrahydro-2H-pyran-4-yl) éthyl] benzyl}-2, 4-diméthyl-9H-pyrido [2, 3-b] indol-3-yl) propanoïque;
acide 3-(9-{4- [(S) -2-4(4-cyclopentylpipérazin-1-yl)-2-oxo-1-(tétrahydro-2H-pyran-4-yl) éthyl] benzyl}-2, 4-diméthyl-9H-pyrido [2, 3-b] indol-3-yl) propanoïque;
et leurs sels.

5. Composition pharmaceutique qui comprend, en tant que principe actif, un dérivé de tétrahydropyrane ou un sel pharmaceutiquement acceptable de celui-ci tel que revendiqué dans l'une quelconque des revendications 1 à 4, et un excipient pharmaceutiquement acceptable.

6. Utilisation d'un dérivé de tétrahydropyrane ou d'un sel pharmaceutiquement acceptable de celui-ci tel que revendiqué dans l'une quelconque des revendications 1 à 4, dans la production d'un médicament pour le traitement de l'hyperlipémie.
